(19) Europäisches Patentamt
European Patent Office
Office européen des brevets

(11) EP 1 915 625 B1

(12) **EUROPEAN PATENT SPECIFICATION**

(45) Date of publication and mention of the grant of the patent:
**04.01.2012 Bulletin 2012/01**

(21) Application number: **06813452.7**

(22) Date of filing: **15.08.2006**

(51) Int Cl.:
***G01N 33/68*** *(2006.01)*

(86) International application number:
**PCT/US2006/031785**

(87) International publication number:
**WO 2007/022157 (22.02.2007 Gazette 2007/08)**

(54) **APOPTOSIS SENSITIVITY TO APO2L/TRAIL BY TESTING FOR GALNAC-T14 EXPRESSION IN CELLS/TISSUES**

APOPTOSESENSITIVITÄT GEGENÜBER APO2L/TRAIL MITTELS TESTEN AUF GALNAC-T14-EXPRESSION IN ZELLEN/GEWEBE

SENSIBILITE APOPTOTIQUE A APO2/TRAIL PAR ESSAI D'EXPRESSION DE GALNAC-T14 DANS DES CELLULES/TISSUS

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **16.08.2005 US 708677 P**
**24.05.2006 US 808076 P**

(43) Date of publication of application:
**30.04.2008 Bulletin 2008/18**

(60) Divisional application:
**10010136.9 / 2 287 615**

(73) Proprietor: **Genentech, Inc.**
**South San Francisco CA 94080-4990 (US)**

(72) Inventors:
- **WAGNER, Klaus, W.**
  **Carmel, Indiana 46032 (US)**
- **ASHKENAZI, Avi, J.**
  **San Mateo**
  **CA 94402 (US)**

(74) Representative: **Kiddle, Simon John et al**
**Mewburn Ellis LLP**
**33 Gutter Lane**
**London**
**EC2V 8AS (GB)**

(56) References cited:
**US-A1- 2003 228 309**

- **WANG HAN ET AL: "Cloning and characterization of a novel UDP-GalNAc:polypeptide N-acetylgalactosaminyltransferase, pp-GalNAc-T14." BIOCHEMICAL AND BIOPHYSICAL RESEARCH COMMUNICATIONS, vol. 300, no. 3, 17 January 2003 (2003-01-17), pages 738-744, XP002433582 ISSN: 0006-291X**
- **AZUMA Y ET AL: "Expression of cell surface Lewis X and Y antigens and FUT4 mRNA is increased in Jurkat cells undergoing apoptosis" BIOCHIMICA ET BIOPHYSICA ACTA, AMSTERDAM, NL, vol. 1672, no. 3, 11 June 2004 (2004-06-11), pages 157-163, XP002368228 ISSN: 0006-3002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 915 625 B1

**Description**

## FIELD OF THE INVENTION

The inventions described herein relate to methods and assays to detect biomarkers predictive of sensitivity of mammalian cells to Apo2L/TRAIL. More particularly, the inventions herein relate to methods and assays which detect molecules associated with the GalNac-T family of proteins which are predictive of sensitivity of mammalian cancer cells to Apo2L/TRAIL.

## BACKGROUND OF THE INVENTION

Various ligands and receptors belonging to the tumor necrosis factor (TNF) superfamily have been identified in the art. Included among such ligands are tumor necrosis factor-alpha ("TNF-alpha"), tumor necrosis factor-beta ("TNF-beta" or "lymphotoxin-alpha"), lymphotoxin-beta ("LT-beta"), CD30 ligand, CD27 ligand, CD40 ligand, OX-40 ligand, 4-1BB ligand, LIGHT, Apo-1 ligand (also referred to as Fas ligand or CD95 ligand), Apo-2 ligand (also referred to as Apo2L or TRAIL), Apo-3 ligand (also referred to as TWEAK), APRIL, OPG ligand (also referred to as RANK ligand, ODF, or TRANCE), and TALL-1 (also referred to as BlyS, BAFF or THANK) (See, e.g., Ashkenazi, Nature Review, 2:420-430 (2002); Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997) Wallach, Cytokine Reference, Academic Press,' 2000, pages 377-411; Locksley et al., Cell, 104:487-501 (2001); Gruss and Dower, Blood, 85:3378-3404 (1995); Schmid et al., Proc. Natl. Acad. Sci., 83:1881 (1986); Dealtry et al., Eur. J. Immunol., 17:689 (1987); Pitti et al., J. Biol. Chem., 271:12687-12690 (1996); Wiley et al., Immunity, 3:673-682 (1995); Browning et al., Cell, 72:847-856 (1993); Armitage et al. Nature, 357: 80-82 (1992), WO 97/01633 published January 16, 1997; WO 97/25428 published July 17, 1997; Marsters et al., Curr. Biol., 8:525-528 (1998); Chicheportiche et al., Biol. Chem., 272:32401-32410 (1997); Hahne et al., J. Exp. Med., 188: 1185-1190 (1998); WO98/28426 published July 2, 1998; WO98/46751 published October 22, 1998; WO/98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)).

Induction of various cellular responses mediated by such TNF family ligands is typically initiated by their binding to specific cell receptors. Some, but not all, TNF family ligands bind to, and induce various biological activity through, cell surface "death receptors" to activate caspases, or enzymes that carry out the cell death or apoptosis pathway (Salvesen et al., Cell, 91:443-446 (1997). Included among the members of the TNF receptor superfamily identified to date are TNFR1, TNFR2, TACI, GITR, CD27, OX-40, CD30, CD40, HVEM, Fas (also referred to as Apo-1 or CD95), DR4 (also referred to as TRAIL-R1), DR5 (also referred to as Apo-2 or TRAIL-R2), DcR1, DcR2, osteoprotegerin (OPG), RANK and Apo-3 (also referred to as DR3 or TRAMP) (see, e.g., Ashkenazi, Nature Reviews, 2:420-430 (2002); Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997) Wallach, Cytokine Reference, Academic Press, 2000, pages 377-411; Locksley et al., Cell, 104:487-501 (2001); Gruss and Dower, Blood, 85:3378-3404 (1995); Hohman et al., J. Biol. Chem., 264:14927-14934 (1989); Brockhaus et al., Proc. Natl. Acad. Sci., 87:3127-3131 (1990); EP 417,563, published March 20, 1991; Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991); Stamenkovic et al., EMBO J., 8:1403-1410 (1989); Mallett et al., EMBO J., 9:1063-1068 (1990); Anderson et al., Nature, 390:175-179 (1997); Chicheportiche et al., J. Biol. Chem., 272 :32401-32410 (1997); Pan et al., Science, 276:111-113 (1997); Pan et al., Science, 277:815-818 (1997); Sheridan et al., Science, 277:818-821 (1997); Degli-Esposti et al., J. Exp. Med., 186: 1165-1170 (1997); Marsters et al., Curr. Biol., 7:1003-1006 (1997); Tsuda et al., BBRC, 234:137-142 (1997); Nocentini et al., Proc. Natl. Acad. Sci., 94:6216-6221 (1997); vonBulow et al., Science, 278:138-141 (1997)).

Most of these TNF receptor family members share the typical structure of cell surface receptors including extracellular, transmembrane and intracellular regions, while others are found naturally as soluble proteins lacking a transmembrane and intracellular domain. The extracellular portion of typical TNFRs contains a repetitive amino acid sequence pattern of multiple cysteine-rich domains (CRDs), starting from the $NH_2$-terminus.

The ligand referred to as Apo-2L or TRAIL was identified several years ago as a member of the TNF family of cytokines. (see, e.g., Wiley et al., Immunity, 3:673-682 (1995); Pitti et al., J. Biol. Chem., 271:12697-12690 (1996); WO 97/01633; WO 97/25428; US Patent 5,763,223 issued June 9, 1998; US Patent 6,284,236 issued September 4, 2001). The full-length native sequence human Apo2L/TRAIL polypeptide is a 281 amino acid long, Type II transmembrane protein. Some cells can produce a natural soluble form of the polypeptide, through enzymatic cleavage of the polypeptide's extracellular region (Mariani et al., J. Cell. Biol., 137:221-229 (1997)). Crystallographic studies of soluble forms of Apo2L/TRAIL reveal a homotrimeric structure similar to the structures of TNF and other related proteins (Hymowitz et al., Molec. Cell, 4:563-571 (1999); Cha et al., Immunity, 11:253-261 (1999); Mongkolsapaya et al., Nature Structural Biology, 6: 1048 (1999); Hymowitz et al., Biochemistry, 39:633-644 (2000)). Apo2L/TRAIL, unlike other TNF family members however, was found to have a unique structural feature in that three cysteine residues (at position 230 of each subunit in the homotrimer) together coordinate a zinc atom, and that the zinc binding is important for trimer stability and biological activity. (Hymowitz et al., supra; Bodmer et al., J. Biol. Chem., 275:20632-20637 (2000)).

It has been reported in the literature that Apo2L/TRAIL may play a role in immune system modulation, including autoimmune diseases such as rheumatoid arthritis [see, e.g., Thomas et al., J. Immunol., 161:2195-2200 (1998); Johnsen et al., Cytokine, 11:664-672 (1999); Griffith et al., J. Exp. Med., 189:1343-1353 (1999); Song et al., J. Exp. Med., 191: 1095-1103 (2000)].

Soluble forms of Apo2L/TRAIL have also been reported to induce apoptosis in a variety of cancer cells, including colon, lung, breast, prostate, bladder, kidney, ovarian and brain tumors, as well as melanoma, leukemia, and multiple myeloma (see, e.g., Wiley et al., supra; Pitti et al., supra; US Patent 6,030,945 issued February 29, 2000; US Patent 6,746,668 issued June 8, 2004; Rieger et al., FEBS Letters, 427:124-128 (1998); Ashkenazi et al., J. Clin. Invest., 104: 155-162 (1999); Walczak et al., Nature Med., 5:157-163 (1999); Keane et al., Cancer Research, 59:734-741 (1999); Mizutani et al., Clin. Cancer Res., 5:2605-2612 (1999); Gazitt, Leukemia, 13:1817-1824 (1999); Yu et al., Cancer Res., 60:2384-2389 (2000); Chinnaiyan et al., Proc. Natl. Acad. Sci., 97:1754-1759 (2000)). *In vivo* studies in murine tumor models further suggest that Apo2L/TRAIL, alone or in combination with chemotherapy or radiation therapy, can exert substantial anti-tumor effects (see, e.g., Ashkenazi et al., supra; Walzcak et al., supra; Gliniak et al., Cancer Res., 59: 6153-6158 (1999); Chinnaiyan et al., supra; Roth et al., Biochem. Biophys. Res. Comm., 265:1999 (1999); PCT Application US/00/15512; PCT Application US/01/23691). In contrast to many types of cancer cells, most normal human cell types appear to be resistant to apoptosis induction by certain recombinant forms of Apo2L/TRAIL (Ashkenazi et al., supra; Walzcak et al., supra). Jo et al. has reported that a polyhistidine-tagged soluble form of Apo2L/TRAIL induced apoptosis *in vitro* in normal isolated human, but not non-human, hepatocytes (Jo et al., Nature Med., 6:564-567 (2000); see also, Nagata, Nature Med., 6:502-503 (2000)). It is believed that certain recombinant Apo2t/TRAIL preparations may vary in terms of biochemical properties and biological activities on diseased versus normal cells, depending, for example, on the presence or absence of a tag molecule, zinc content, and % trimer content (See, Lawrence et al., Nature Med., Letter to the Editor, 7:383-385 (2001); Qin et al., Nature Med., Letter to the Editor, 7:385-386 (2001)).

Apo2L/TRAIL has been found to bind at least five different receptors. At least two of the receptors which bind Apo2L/TRAIL contain a functional, cytoplasmic death domain. One such receptor has been referred to as "DR4" (and alternatively as TR4 or TRAIL-R1) (Pan et al., Science, 276:111-113 (1997); see also WO98/32856 published July 30, 1998; WO99/37684 published July 29, 1999; WO 00/73349 published December 7, 2000; US 2003/0036168 published February 20, 2003; US 6,433,147 issued August 13, 2002; US 6,461,823 issued October 8, 2002, and US 6,342,383 issued January 29, 2002).

Another such receptor for Apo2L/TRAIL has been referred to as DR5 (it has also been alternatively referred to as Apo-2; TRAIL-R or TRAIL-R2, TR6, Tango-63, hAPO8, TRICK2 or KILLER) (see, e.g., Sheridan et al., Science, 277:818-821 (1997), Pan et al., Science, 277:815-818 (1997), WO98/51793 published November 19, 1998; WO98/41629 published September 24, 1998; Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/1179 published March 11, 1999; WO 03/042367 published May 22, 2003; WO 02/097033 published December 5, 2002; WO 03/038043 published May 8, 2003;; US 2002/0072091 published August 13, 2002; US 2002/0098550 published December 7, 2001; US 6,313,269 issued December 6, 2001; US 2001/0010924 published August 2, 2001; US 2003/01255540 published July 3, 2003; US 2002/0160446 published October 31, 2002, US 2002/0048785 published April 25, 2002; US 2004/0141952 published July 22, 2004; US 2005/0129699 published June 16, 2005; US 2005/0129616 published June 16, 2005; US 6,342,369 issued February, 2002; US 6,569,642 issued May 27, 2003, US 6,072,047 issued June 6, 2000, US 6,642,358 issued November 4, 2003; US 6,743,625 issued June 1, 2004). Like DR4, DR5 is reported to contain a cytoplasmic death domain and be capable of signaling apoptosis upon ligand binding (or upon binding a molecule, such as an agonist antibody, which mimics the activity of the ligand). The crystal structure of the complex formed between Apo-2L/TRAIL and DR5 is described in Hymowitz et al., Molecular Cell, 4:563-571 (1999).

Upon ligand binding, both DR4 and DR5 can trigger apoptosis independently by recruiting and activating the apoptosis initiator, caspase-8, through the death-domain-containing adaptor molecule referred to as FADD/Mort1 [Kischkel et al., Immunity, 12:611-620 (2000); Sprick et al., Immunity, 12:599-609 (2000); Bodmer et al., Nature Cell Biol., 2:241-243 (2000)].

Apo2L/TRAIL has been reported to also bind those receptors referred to as DcR1, DcR2 and OPG, which believed to function as inhibitors, rather than transducers of signaling (see., e.g., DCR1 (also referred to as TRID, LIT or TRAIL-R3) [Pan et al., Science, 276:111-113 (1997); Sheridan et al., Science, 277:818-821 (1997); McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998); DCR2 (also called TRUNDD or TRAIL-R4) [Marsters et al., Curr. Biol., 7:1003-1006 (1997); Pan et al., FEBS Letters, 424:41-45 (1998); Degli-Esposti et al., Immunity, 7:813-820 (1997)], and OPG [Simonet et al., supra]. In contrast to DR4 and DR5, the DcR1 and DcR2 receptors do not signal apoptosis.

Certain antibodies which bind to the DR4 and/or DR5 receptors have been reported in the literature. For example, anti-DR4 antibodies directed to the DR4 receptor and having agonistic or apoptotic activity in certain mammalian cells are described in, e.g., WO 99/37684 published July 29, 1999; WO 00/73349 published July 12, 2000; WO 03/066661 published August 14, 2003. See, also, e.g., Griffith et al., J. Immunol., 162:2597-2605 (1999); Chuntharapai et al., J. Immunol., 166:4891-4898 (2001); WO 02/097033 published December 2, 2002; WO 03/042367 published May 22, 2003; WO 03/038043 published May 8, 2003; WO 03/037913 published May 8, 2003; US 2003/0073187 published April 17, 2003; US 2003/0108516 published June 12, 2003. Certain anti-DR5 antibodies have likewise been described, see, e.g., WO 98/51793 published November 8, 1998; Griffith et al., J. Immunol., 162:2597-2605 (1999); Ichikawa et al., Nature Med., 7:954-960 (2001); Hylander et al., "An Antibody to DR5 (TRAIL-Receptor 2) Suppresses the Growth of Patient Derived Gastrointestinal Tumors Grown in SCID mice", Abstract, 2d International Congress on Monoclonal Antibodies in Cancers, Aug. 29-Sept. 1, 2002, Banff, Alberta, Canada; WO 03/038043 published May 8, 2003; WO 03/037913 published May 8, 2003; US 2003/0180296 published September 25, 2003;. In addition, certain antibodies having cross-reactivity to both DR4 and DR5 receptors have been described (see, e.g., US patent 6,252,050 issued June 26, 2001).

## SUMMARY OF THE INVENTION

The invention disclosed herein provides methods and assays examining expression of one or more biomarkers in a mammalian tissue or cell sample, wherein the expression of one or more such biomarkers is predictive of whether the tissue or cell sample will be sensitive to agents such as Apo2L/TRAIL. In various embodiments of the invention, the methods and assays examine expression of molecules in the GalNac-T family of proteins, in particular GalNAc-T14 see WANG et al, vol 300, p.738-744 which discloses the cloning of the GalNAC polypeptide.

As discussed above, most normal human cell types appear to be resistant to apoptosis induction by certain recombinant forms of Apo2L/TRAIL (Ashkenazi et al., supra; Walzcak et al., supra). It has also been observed that some populations of diseased human cell types (such as certain populations of cancer cells) are resistant to apoptosis induction by certain recombinant forms of Apo2L/TRAIL (Ashkenazi et al., J. Clin. Invest., 1999, supra; Walczak et al., Nature Med., 1999, supra). Consequently, by examining a mammalian tissue or cell sample for expression of selected biomarkers by way of an assay, one can conveniently and efficiently obtain information useful in assessing appropriate or effective therapies for treating patients. For example, information obtained from an assay to detect GalNac-T14 expression in a mammalian tissue or cell sample can provide physicians with useful data that can be used to determine an optimal therapeutic regimen (using Apo2L/TRAIL) for patients suffering from a disorder such as cancer.

The invention provides methods for predicting the sensitivity of a mammalian cancer tissue or cell sample to Apo2L/TRAIL, the method comprising examining the tissue or cell sample to detect expression of GalNac-T14, wherein expression of said GalNac-T14 is predictive that said tissue or cell sample is sensitive to apoptosis-inducing activity of Apo2L/TRAIL.

In certain embodiments, the methods comprise obtaining a mammalian tissue or cell sample and examining the tissue or cell for expression of GalNac-T14. The methods may be conducted in a variety of assay formats, including assays detecting mRNA and/or protein expression, enzymatic activity assays and others discussed herein. Determination of expression of GalNac-T14 in said tissues or cells will be predictive that such tissues or cells will be sensitive to the apoptosis-inducing activity of Apo2L/TRAIL. In optional embodiments, the tissues or cells may also be examined for expression of DR4, DR5, DcR1 or DcR2 receptors.

Further methods of the invention method for inducing apoptosis in a mammalian cancer tissue sample or cell sample which comprises:

examining the tissue sample or cell sample to detect expression of GalNac-T14, and
subsequent to detecting expression of said GalNac-T14, exposing said tissue sample or cell sample to an effective amount of Apo2L/TRAIL. The steps in the methods for examining expression of GalNac-T14 may be conducted in a variety of assay formats, including assays detecting mRNA and/or protein expression, enzymatic activity, and others discussed herein. In optional embodiments, the methods also comprise examining the tissue or cell sample for expression of DR4, DR5, DcR1, or DcR2 receptors. Optionally, the tissue or cell sample comprises cancer tissue or cells. Optionally, the tissue or cell sample comprises non-small cell lung cancer cells, pancreatic cancer cells, breast cancer cells, or non-Hodgkin's lymphoma cells.

In a further aspect, the present invention provides the use of Apo2L/TRAIL in the manufacture of a medicament for the treatment of an immune related disorder or cancer in a mammalian subject, wherein a tissue or cell sample from the subject has been determined to express GalNac-T14 and expression of GalNac-T14 is predictive that said tissue or cell sample is sensitive to apoptosis-inducing activity of Apo2L/TRAIL.

In a further aspect, the present invention provides Apo2L/TRAIL for use in a method of treating cancer in a mammalian subject, wherein a tissue or cell sample from the subject has been determined to express GalNac-T14 and expression of GalNac-T14 is predictive that said tissue or cell sample is sensitive to apoptosis-inducing activity of Apo2L/TRAIL.

In a further aspect, the present invention provides the use of an antibody or a polynucleotide to detect expression of GalNac-T14 in a mammalian tissue or cell sample in the for predicting the sensitivity of a mammalian tissue or cell sample to Apo2L/TRAIL, wherein expression of said GalNac-T14 is predictive that said tissue or cell sample is sensitive to apoptosis-inducing activity of Apo2L/TRAIL

In carrying out these medical uses, examination of the expression of one or more biomarkers may be conducted in a variety of assay formats, including assays detecting mRNA and/or protein expression, enzymatic activity, and others discussed herein. In optional embodiments, the methods also comprise examining the tissue or cell sample for expression of DR4, DR5, DcR1, or DcR2 receptors. Optionally, the methods comprise treating cancer in a mammal.

Optionally, the medical uses comprise, in addition to the administration of an effective amount of Apo2L/TRAIL, the administration of chemotherapeutic agent(s) or radiation therapy to said mammal.

In further embodiments of the invention, the afore-mentioned methods may comprise examining mammalian tissue or cells for expression of other GalNac-T molecules, such as GalNac-T3.

**BRIEF DESCRIPTION OF THE FIGURES**

Figure 1 shows the nucleotide sequence of human Apo-2 ligand cDNA (SEQ ID NO:2) and its derived amino acid sequence (SEQ ID NO:1). The "N" at nucleotide position 447 is used to indicate the nucleotide base may be a "T" or "G".

Figures 2A and 2B show the nucleotide sequence of a cDNA (SEQ ID NOs 4 and 30, respectively, in order of appearance) for full length human DR4 and its derived amino acid sequence (SEQ ID NO:3). The respective nucleotide and amino acid sequences for human DR4 are also reported in Pan et al., Science, 276:111 (1997).

Figure 3A shows the 411 amino acid sequence (SEQ ID NO:5) of human DR5 as published in WO 98/51793 on November 19, 1998. A transcriptional splice variant of human DR5 is known in the art. This DR5 splice variant encodes the 440 amino acid sequence (SEQ ID NO:6) of human DR5 shown in Figures 3B and 3C as published in WO 98/35986 on August 20, 1998.

Figure 3D-1 - 3D-3 shows the nucleotide sequences of cDNA (SEQ ID NOs 7 and 31, respectively, in order of appearance) for full length human DcR1 and amino acid sequences derived from two different translation initiation sites in the (duplicated) DcR1 cDNA molecule shown in Figure 3D-1 - 3D-3 (SEQ ID NOs 8 and 32, respectively, in order of appearance). The respective nucleotide and amino acid sequences for human DcR1 (and particular domains thereof) are also shown and described in WO 98/58062.

Figure 3E shows the nucleotide sequences of cDNA (SEQ ID NO:9) for full length human DcR2 and its derived amino acid sequence (SEQ ID NO:10). The respective nucleotide and amino acid sequences for human DcR2 (and particular domains thereof) are also shown in WO 99/10484.

Figure 4A shows the nucleotide sequence of human GalNac-T14 (SEQ ID NO:11) and its derived amino acid sequence (SEQ ID NO:12). These sequences are also described in Wang et al., BBRC, 300:738-744 (2003).

Figure 4B shows the nucleotide sequence of human GalNac-T3 (SEQ ID NO:13) and its derived amino acid sequence (SEQ ID NO:14). These sequences are also described in Bennett et al., J. Biol. Chem., 271:17006-17012 (1996).

Figure 5 provides an IC50 summary chart of the data obtained in analyzing non-small cell lung cancer ("NSCLC") cell lines for sensitivity or resistance to apoptotic activity of Apo2L (+ 0.5% fetal bovine serum "FBS" or 10% FBS) or DR5 monoclonal antibody "DR5 ab", cross-linked "XL" or not crosslinked, + 0.5% fetal bovine serum "FBS" or 10% FBS) as measured in MTT cytotoxicity assays.

Figure 6 provides an IC50 summary chart of the data obtained in analyzing pancreatic cancer cell lines for sensitivity or resistance to apoptotic activity of Apo2L (+ 0.5% fetal bovine serum "FBS" or 10% FBS) or DR5 monoclonal antibody "DR5 ab", cross-linked "XL" or not crosslinked, + 0.5% fetal bovine serum "FBS" or 10% FBS) as measured in MTT cytotoxicity assays.

Figure 7 provides an IC50 summary chart of the data obtained in analyzing non-hodgkin's lymphoma cancer ("NHL") cell lines for sensitivity or resistance to apoptotic activity of Apo2L (+ 10% fetal bovine serum "FBS") or DR5 monoclonal antibody "DR5 ab", cross-linked "XL" or not crosslinked, (+ 10% fetal bovine serum "FBS") as measured in MTT cytotoxicity assays.

Figure 8 provides a comparison of sensitivity ("sen") or resistance ("RES") of select NSCLC, Pancreatic, and NHL cancer cell lines to DR5 antibody and the correlation to expression of GalNac-T14, as measured by GalNac-T14 mRNA expression.

Figure 9 provides a bar diagram graph of various NSCLC, pancreatic, and NHL cell lines ranked (in descending order) by levels of GalNac-T14 mRNA expression patterns.

Figure 10A-D illustrates differential expression of specific O-glycosylation enzymes in Apo2L/TRAIL-sensitive and -resistant cancer cell lines: (A) Cell viability was measured after incubation with varying doses of Apo2L/TRAIL. IC50 for each cell line was computed as the concentration of Apo2L/TRAIL that gives 50% loss of viability. Each cell viability experiment was repeated at least three times in presence of low (0.5%) and high (10%) fetal bovine serum. Black, grey, or open symbols depict cell lines that are highly sensitive, moderately sensitive, or resistant to Apo2L/TRAIL, respectively. (B) ppGalNAcT-14 mRNA expression levels (probe set 219271_at) in pancreatic and malignant melanoma cell lines. Cell lines are arranged by tissue type and sensitivity to Apo2L/TRAIL. Black, grey, or open bars depict cell lines as in A. (C) mRNA expression levels of Fut-6 (top panel, probe set 211885_x_at) and ppGalNAcT-3 (bottom panel, probe set 203397_s_at) in colorectal cancer cell lines. Cell lines are arranged as in B. The P values in panels B and C are based on a Fisher's test of the correlation between cell line sensitivity (including high and moderate) and mRNA expression above cutoff. (D) Effect of Apo2L/TRAIL on growth of established tumor xenografts. Athymic nude mice carrying GalNAcT-3/Fut-6-positive (left panel) or GalNACT-3/Fut-6-negative (right panel) tumors received vehicle or Apo2L/TRAIL (60 mg/kg/day i.p. on days 0-4) and tumor volume was monitored (mean$\pm$SE, N=10 mice/group).

Figure 11 illustrates modulation of particular O-glycosylation enzymes alters sensitivity to Apo2L/TRAIL. (A) Colo205 cells were preincubated with the pan O-glycosylation enzyme inhibitor benzyl-GalNAc (bGalNAC), treated with Apo2L/TRAIL for 24 h, and cell viability was determined (DMSO=vehicle control). (B) PSN-1 (pancreatic carcinoma) and Hs294T (melanoma) cells were transfected with caspase-8 or ppGalNACT-14 siRNAs for 48 h, incubated with Apo2L/TRAIL for another 24 h and cell viability was determined. siRNA duplexes against a non-targeting sequence (Dharmacon) were used as a control (NTC). (C) DLD-1 colorectal carcinoma cells were transfected with ppGalNACT-3 or Fut-6 by siRNAs and tested as in B. (D) HEK293 cells were co-transfected with plasmids encoding the indicated genes in combination with ppGalNAcT-14 or vector control. Apoptosis was measured at 24 h by Annexin V staining (left panel). H1569 melanoma cells were transduced with retrovirus directing ppGalNAcT-14 expression or control retrovirus; resulting cell line pools were treated with Apo2L/TRAIL for 24 h and cell viability was determined (right panel). Western blot analysis using anti-FLAG antibodies was used to verify expression of epitope-tagged ppGalNACT-14.

Figure 12 illustrates (A) Analysis of the caspase cascade induced by Apo2L/TRAIL. PSN-1 and DLD-1 cells were transfected with siRNAs against ppGalNACT-14 or Fut-6, respectively, for 48 h. The cells were treated with Apo2L/TRAIL for 4 or 8 h, and cell lysates were analyzed by immunoblot with antibodies specific for caspase-8, Bid, caspase-9, caspase-3, or actin as a loading control. (B) PSN-1 cells were transfected with ppGalNAcT-14 siRNA as in A, treated with Apo2L/TRAIL for 4 h, and caspase-3/7 enzymatic activity in cell lysates was determined. (C) Analysis of the Apo2L/TRAIL DISC. PSN-1 cells were transfected with ppGalNAcT-14 siRNA as in A. FLAG-Apo2L/TRAIL (1 mg/ml) was added for 0-60 min, the cells were lysed, and subjected to an immunoprecipitation with an anti-FLAG antibody. DISC-associated FADD, caspase-8, DR4, and were detected by immunoblot. (D) PSN-1 cells were transfected, treated, and subjected to DISC immunoprecipitation as in C, and DISC-associated caspase-8 enzymatic activity was measured as previously described (Sharp et al., J. Biol. Chem., 280:19401 (2005).

Figure 13 illustrates (A) Monosaccharide analysis of recombinant human DR5 (Long splice variant) produced in CHO cells, performed by HPAEC-PAD (high-performance anion-exchange chromatography with pulsed amperometric detection). (B) Sequence comparison of human Apo2L/TRAIL receptors (SEQ ID NOs 22, 33, 23, 34, 24, 35, 25, 36, 26, 37, 27, and 38-44, respectively, in order of appearance) (human DR5 long 440 aa form "hDR5L", human DR5 short form 411 aa "hDR5S" and hDR4), murine DR5 (mDR5), human Fas (hFas) and human TNFR1 (hTNFR1). Boxes indicate putative O-glycosylation sites. (C) Immunoblot analysis of total cell lysates corresponding to D. DRSL-5T and DR5S-5T are constructs containing 5 threonine-to-alanine substitutions and DR5L-5T3S and DR5S-5T3S are constructs containing 5 threonine-to-alanine and three serine-to-alanine substitutions, respectively, in residues that are potential 0-glycosylation sites. (D) HEK293 cells were co-transfected with the indicated DR5 constructs together with vector or ppGalNAcT-14 plasmid for 48 h and apoptosis was measured by Annexin V staining. (E) mRNA expression levels for ppGalNAcT-14 (Affymetrix chip, probe set 219271_at) in primary human tumor samples from cancers of the skin (SCC=squamous cell carcinoma), lung, pancreas (Panc), breast, ovarian (Ov), endometrium (Endo), bladder (Bla, TCC= transitional cell carcinoma) and NHL (FL=follicular lymphoma, DLBCL=diffuse large B-cell lymphoma). Median expression of samples is indicated by a grey horizontal bar for each class. A cutoff of 500 and 200 (melanoma) corresponding to the cell line data from Figure 10B is displayed.

Figure 14 illustrates (A) Reduction in mRNA expression of ppGalNAcT-14 or ppGalNAcT-3 in PSN-1 or DLD-1 cells after 48h siRNA knockdown by Taqman analysis. (B) GalNAcT-14 expression is reconstituted in PSN-1 cells by transfection of empty plasmid (Empty), wild-type GalNAcT-14 (GalNAcT-14) or GalNAcT-14 containing siRNA silent mutations (GalNAcT-14 si(1)Mut (SEQ ID NO: 28)) subsequent to siGalNAcT-14 (1) (SEQ ID NO: 29) mediated knock-down of ppGalNAcT-14. (C) Down-regulation of ppGalNAcT-3 or Fut-6 by interfering RNAs inhibits Apo2L/TRAIL induced cell death in C170 (colorectal cancer) cells. Experimental procedure as in 11C. (Table 1) A) Summary table of siRNA knockdown phenotypes. Cell lines, in which downregulation of GalNAcT-14 or ppGalNAcT-3 and Fut-6 resulted in protection from Apo2L/TRAIL, are marked indicating less (+) or greater than 50% (++) protection with at least one siRNA oligonucleotide tested. (0) indicates the absence of protection against Apo2L/TRAIL. (D), (E) Subsequent to a 48h knockdown with the indicated siRNAs, cells were treated with increasing doses of etoposide or staurosporine (STS) for 24h and subjected to a cell viability assay. (F) Retroviral ppGalNAcT-14 overexpressing PA-TU-8902 and PL-45 cell line pools were subjected to cell viability assays after Apo2L/TRAIL treatment. Western blot analysis using anti-FLAG antibodies indicates retroviral expressed ppGalNAcT-14 in these cells.

Figure 15 (A) Western blot analysis of the Apo2L/TRAIL induced caspase activation cascade in Apo2L/TLAIL sensitive Colo205 and resistant colorectal cancer cell lines, RKO and SW1417. Cells were treated with 1000ng/ml Apo2L/TRAIL for 8 and 24h and total cell lysates were subjected to western blot analysis using antibodies specific for caspase-8, Bid, caspase-9, caspase-3 and actin as a loading control. (B) Knockdown of Fut-6 reduced recruitment and activation of caspase-8 at the Apo2L/TRAIL DISC in DLD-1 cells. Experimental procedure accordingly to 12D. (C) Cell surface expression of DR4 and DR5 was measured by FACS analysis in cells that were subjected to a siRNA knockdown with the indicated genes.

## DETAILED DESCRIPTION OF THE INTENTION

The techniques and procedures described or referenced herein are generally well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

Before the present methods and assays are described, it is to be understood that this invention is not limited to the particular methodology, protocols, cell lines, animal species or genera, constructs, and reagents described as such may, of course, vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise. Thus, for example, reference to "a genetic alteration" includes a plurality of such alterations and reference to "a probe" includes reference to one or more probes and equivalents thereof known to those skilled in the art, and so forth.

## I. DEFINITIONS

The terms "Apo2L/TRAIL", "Apo-2L", and "TRAIL" are used herein to refer to a polypeptide sequence which includes amino acid residues 114-281, inclusive, 95-281, inclusive, residues 92-281, inclusive, residues 91-281, inclusive, residues 41-281, inclusive, residues 15-281, inclusive, or residues 1-281, inclusive, of the amino acid sequence shown in Figure 1, as well as biologically active fragments, deletional, insertional, or substitutional variants of the above sequences. In one embodiment, the polypeptide sequence comprises residues 114-281 of Figure 1, and optionally, consists of residues 114-281 of Figure 1. Optionally, the polypeptide sequence comprises residues 92-281 or residues 91-281 of Figure 1. The Apo-2L polypeptides may be encoded by the native nucleotide sequence shown in Figure 1. Optionally, the codon which encodes residue Pro119 (Figure 1) may be "CCT" or "CCG". In other embodiments, the fragments or variants are biologically active and have at least about 80% amino acid sequence identity, more preferably at least about 90% sequence identity, and even more preferably, at least 95%, 96%, 97%, 98%, or 99% sequence identity with any one of the above recited Apo2L/TRAIL sequences. Optionally, the Apo2L/TRAIL polypeptide is encoded by a nucleotide sequence which hybridizes under stringent conditions with the encoding polynucleotide sequence provided in Figure 1. The definition encompasses substitutional variants of Apo2L/TRAIL in which at least one of its native amino acids are substituted by an alanine residue. Particular substitutional variants of the Apo2L/TRAIL include those in which at least one amino acid is substituted by an alanine residue. These substitutional variants include those identified, for example, as "D203A"; "D218A" and "D269A." This nomenclature is used to identify Apo2L/TRAIL variants wherein the aspartic acid residues at positions 203, 218, and/or 269 (using the numbering shown in Figure 1) are substituted by alanine residues. Optionally, the Apo2L variants may comprise one or more of the alanine substitutions which are recited in Table I of published PCT application WO 01/00832. Substitutional variants include one or more of the residue substitutions identified in Table I of WO 01/00832 published January 4, 2001. The definition also encompasses a native sequence Apo2L/TRAIL isolated from an Apo2L/TRAIL source or prepared by recombinant or synthetic methods. The Apo2L/TRAIL of the invention includes the polypeptides referred to as Apo2L/TRAIL or TRAIL disclosed in PCT Publication Nos. WO97/01633 and WO97/25428. The terms "Apo2L/TRAIL" or "Apo2L" are used to refer generally to forms of the Apo2L/TRAIL which include monomer, dimer or trimer forms of the polypeptide. All numbering of amino acid residues referred to in the Apo2L sequence use the numbering according to Figure 1, unless specifically stated otherwise. For instance, "D203" or "Asp203" refers to the aspartic acid residue at position 203 in the sequence provided in Figure 1.

The term "Apo2L/TRAIL extracellular domain" or "Apo2L/TRAIL ECD" refers to a form of Apo2L/TRAIL which is essentially free of transmembrane and cytoplasmic domains. Ordinarily, the ECD will have less than 1% of such transmembrane and cytoplasmic domains, and preferably, will have less than 0.5% of such domains. It will be understood that any transmembrane domain(s) identified for the polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified. In preferred embodiments, the ECD will consist of a soluble, extracellular domain sequence of the polypeptide which is free of the transmembrane and cytoplasmic or intracellular domains (and is not membrane bound). Particular extracellular domain sequences of Apo-2L/TRAIL are described in PCT Publication Nos. WO97/01633 and WO97/25428.

The term "Apo2L/TRAIL monomer" or "Apo2L monomer" refers to a covalent chain of an extracellular domain sequence of Apo2L.

The term "Apo2L/TRAIL dimer" or "Apo2L dimer" refers to two Apo-2L monomers joined in a covalent linkage via a disulfide bond. The term as used herein includes free standing Apo2L dimers and Apo2L dimers that are within trimeric forms of Apo2L (i.e., associated with another, third Apo2L monomer).

The term "Apo2L/TRAIL trimer" or "Apo2L trimer" refers to three Apo2L monomers that are non-covalently associated.

The term "Apo2L/TRAIL aggregate" is used to refer to self-associated higher oligomeric forms of Apo2L/TRAIL, such as Apo2L/TRAIL trimers, which form, for instance, hexameric and nanomeric forms of Apo2L/TRAIL. Determination of the presence and quantity of Apo2L/TRAIL monomer, dimer, or trimer (or other aggregates) may be made using methods and assays known in the art (and using commercially available materials), such as native size exclusion HPLC ("SEC"), denaturing size exclusion using sodium dodecyl sulphate ("SDS-SEC"), reverse phase HPLC and capillary electrophoresis.

"Apo-2 ligand receptor" includes the receptors referred to in the art as "DR4" and "DR5" whose polynucleotide and polypeptide sequences are shown in Figures 2 and 3 respectively. Pan et al. have described the TNF receptor family member referred to as "DR4" (Pan et al., Science, 276:111-113 (1997); see also WO98/32856 published July 30, 1998; WO 99/37684 published July 29, 1999; WO 00/73349 published December 7, 2000; US 6,433,147 issued August 13, 2002; US 6,461,823 issued October 8, 2002, and US 6,342,383 issued January 29, 2002). Sheridan et al., Science, 277:818-821 (1997) and Pan et al., Science, 277:815-818 (1997) described another receptor for Apo2L/TRAIL (see also, WO98/51793 published November 19, 1998; WO98/41629 published September 24, 1998). This receptor is referred to as DR5 (the receptor has also been alternatively referred to as Apo-2; TRAIL-R, TR6, Tango-63, hAPO8, TRICK2 or KILLER; Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/1179 published March 11, 1999; US 2002/0072091 published August 13, 2002; US 2002/0098550 published December 7, 2001; US 6,313,269 issued December 6, 2001; US 2001/0010924 published August 2, 2001; US 2003/01255540 published July 3, 2003; US 2002/0160446 published October 31, 2002, US 2002/0048785 published April 25, 2002; US 6,569,642 issued May 27, 2003, US 6,072,047 issued June 6, 2000, US 6,642,358 issued November 4, 2003). As described above, other receptors for Apo-2L include DcR1, DcR2, and OPG (see, Sheridan et al., supra; Masters et al., supra; and Simonet et al., supra). The term "Apo-2L receptor" when used herein encompasses native sequence receptor and receptor variants. These terms encompass Apo-2L receptor expressed in a variety of mammals, including humans. Apo-2L receptor may be endogenously expressed as occurs naturally in a variety of human tissue lineages, or may be expressed by recombinant or synthetic methods. A "native sequence Apo-2L receptor" comprises a polypeptide having the same amino acid sequence as an Apo-2L receptor derived from nature. Thus, a native sequence Apo-2L receptor can have the amino acid sequence of naturally-occurring Apo-2L receptor from any mammal. Such native sequence Apo-2L receptor can be isolated from nature or can be produced by recombinant or synthetic means. The term "native sequence Apo-2L receptor" specifically encompasses naturally-occurring truncated or secreted forms of the receptor (e.g., a soluble form containing, for instance, an extracellular domain sequence), naturally-occurring variant forms (*e.g.*, alternatively spliced forms) and naturally-occurring allelic variants. Receptor variants may include fragments or deletion mutants of the native sequence Apo-2L receptor. Figure 3A shows the 411 amino acid sequence of human DR5 as published in WO 98/51793 on November 19, 1998. A transcriptional splice variant of human DR5 is known in the art. This DR5 splice variant encodes the 440 amino acid sequence of human DR5 shown in Figures 3B and 3C as published in WO 98/35986 on August 20, 1998.

"Death receptor antibody" is used herein to refer generally to antibody or antibodies directed to a receptor in the tumor necrosis factor receptor superfamily and containing a death domain capable of signalling apoptosis, and such antibodies include DR5 antibody and DR4 antibody.

"DR5 receptor antibody", "DR5 antibody", or "anti-DR5 antibody" is used in a broad sense to refer to antibodies that bind to at least one form of a DR5 receptor, such as the 1-411 sequence shown in Figures 3A or the 1-440 sequence shown in Figures 3B-3C, or extracellular domain thereof. Optionally the DR5 antibody is fused or linked to a heterologous sequence or molecule. Preferably the heterologous sequence allows or assists the antibody to form higher order or oligomeric complexes. Optionally, the DR5 antibody binds to DR5 receptor but does not bind or cross-react with any additional Apo-2L receptor (e.g. DR4, DcR1, or DcR2). Optionally the antibody is an agonist of DR5 signalling activity.

Optionally, the DR5 antibody of the invention binds to a DR5 receptor at a concentration range of about 0.1 nM to about 20 mM as measured in a BIAcore binding assay. Optionally, the DR5 antibodies of the invention exhibit an Ic 50 value of about 0.6 nM to about 18 mM as measured in a BIAcore binding assay.

"DR4 receptor antibody", "DR4 antibody", or "anti-DR4 antibody" is used in a broad sense to refer to antibodies that bind to at least one form of a DR4 receptor or extracellular domain thereof. Optionally the DR4 antibody is fused or linked to a heterologous sequence or molecule. Preferably the heterologous sequence allows or assists the antibody to form higher order or oligomeric complexes. Optionally, the DR4 antibody binds to DR4 receptor but does not bind or cross-react with any additional Apo-2L receptor (e.g. DR5, DcR1, or DcR2). Optionally the antibody is an agonist of DR4 signalling activity.

Optionally, the DR4 antibody of the invention binds to a DR4 receptor at a concentration range of about 0.1 nM to about 20 mM as measured in a BIAcore binding assay. Optionally, the DR4 antibodies of the invention exhibit an Ic 50 value of about 0.6 nM to about 18 mM as measured in a BIAcore binding assay.

The term "agonist" is used in the broadest sense, and includes any molecule that partially or fully enhances, stimulates or activates one or more biological activities of Apo2L/TRAIL, DR4 or DR5, *in vitro*, *in situ*, or *in vivo*. Examples of such biological activities are binding of Apo2L/TRAIL to DR4 or DR5, including apoptosis as well as those further reported in the literature. An agonist may function in a direct or indirect manner. For instance, the agonist may function to partially or fully enhance, stimulate or activate one or more biological activities of DR4 or DR5, *in vitro*, *in situ*, or *in vivo* as a result of its direct binding to DR4 or DR5, which causes receptor activation or signal transduction. The agonist may also function indirectly to partially or fully enhance, stimulate or activate one or more biological activities of DR4 or DR5, *in vitro*, *in situ*, or *in vivo* as a result of, e.g., stimulating another effector molecule which then causes DR4 or DR5 activation or signal transduction. It is contemplated that an agonist may act as an enhancer molecule which functions indirectly to enhance or increase DR4 or DR5 activation or activity. For instance, the agonist may enhance activity of endogenous Apo-2L in a mammal. This could be accomplished, for example, by pre-complexing DR4 or DR5 or by stabilizing complexes of the respective ligand with the DR4 or DR5 receptor (such as stabilizing native complex formed between Apo-2L and DR4 or DR5).

The term "biomarker" as used in the present application refers generally to a molecule, including a gene, protein, carbohydrate structure, or glycolipid, the expression of which in or on a mammalian tissue or cell can be detected by standard methods (or methods disclosed herein) and is predictive for a mammalian cell's or tissue's sensitivity to Apo2L/TRAIL or death receptor antibody. Such biomarkers contemplated by the present invention include but are not limited to molecules in the GalNac-T family of proteins. Members of the human N-acetylgalactosaminyltransferase ("GalNac-T") family of genes and proteins have been described (see, e.g, Hang et al., "The chemistry and biology of mucin-type O-linked glycosylation initiated by the polypeptide N-acetyl- -galactosaminyltransferases", Bioorganic & Medicinal Chemistry, (available May 2005 at www.sciencedirect.com) and references cited therein; Wang et al., BBRC, 300:738-744 (2003) and references cited therein), and are thought to function in determining the number and position of O-linked sugar chains in proteins. Optionally, the expression of such a biomarker is determined to be higher than that observed for a control tissue or cell sample. Optionally, for example, the expression of such a biomarker will be determined using a gene expression microarray, quantitative PCR or immunohistochemistry (IHC) assay. Optionally, expression of a GalNac-T biomarker, such as GalNac-T14 or GalNac-T3, will be detected at a level of at least 750, as measured by Affymetrix U133P microarray analysis, or 500-fold, or preferably at least 1000- fold higher, in the test tissue or cell sample than that observed for a control tissue or cell sample when detecting expression of the biomarker using quantitative PCR.

"UDP-N-acetyl-D-galactosamine:polypeptide N-acetylgalactosaminyltransferase-T14", "pp-GalNac-T14", "GalNac-T14", "GALNT14" are used herein to refer a type II membrane protein having characteristic features of the GalNac-T family of molecules comprising a N-terminal cytoplasmic domain, transmembrane domain, stem region and catalytic domain. In an optional embodiment, the human GalNac-T14 molecule contains 1659 base pairs encoding a 552 amino acid protein, as shown in Figure 4A. The full length human cDNA has been deposited in GenBank as Accession No. AB078144. As disclosed in Wang et al., BBRC, 300:738-744 (2003), spliced isoforms of GalNac-T14 have been identified which include (or do not include) particular exons, such as exons 2, 3, and/or 4. The present invention contemplates examining expression of any of such various isoforms of GalNac-T14, and that expression of any one such isoforms is predictive of the mammalian tissue or cell sample's sensitivity to Apo2L/TRAIL or death receptor antibody:

"UDP-N-acetyl-D-galactosamine:polypeptide N-acetylgalactosaminyltransferase-T3", "pp-GalNac-T3", "GalNac-T3", "GALNT3" are used herein to refer a type II membrane protein having characteristic features of the GalNac-T family of molecules comprising a N-terminal cytoplasmic domain, transmembrane domain, stem region and catalytic domain. In an optional embodiment, the human GalNac-T3 polypeptide comprises the amino acid sequence shown in Figure 4B. GalNac-T3 is further described in Bennett et al., J. Biol. Chemistry, 271:17006-17012 (1996).

By "subject" or "patient" is meant any single subject for which therapy is desired, including humans. Also intended to be included as a subject are any subjects involved in clinical research trials not showing any clinical sign of disease, or subjects involved in epidemiological studies, or subjects used as controls.

The term "mammal" as used herein refers to any mammal classified as a mammal, including humans, cows, horses, dogs and cats. In a preferred embodiment of the invention, the mammal is a human.

By "tissue or cell sample" is meant a collection of similar cells obtained from a tissue of a subject or patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a primary or metastatic tumor. The tissue sample may contain compounds which are not naturally intermixed with the tissue in nature such as preservatives, anticoagulants, buffers, fixatives, nutrients, antibiotics, or the like.

For the purposes herein a "section" of a tissue sample is meant a single part or piece of a tissue sample, e.g. a thin slice of tissue or cells cut from a tissue sample. It is understood that multiple sections of tissue samples may be taken and subjected to analysis according to the present invention, provided that it is understood that the present invention comprises a method whereby the same section of tissue sample is analyzed at both morphological and molecular levels, or is analyzed with respect to both protein and nucleic acid.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to various embodiments herein, one may use the results of an analytical assay such as mRNA expression or IHC to determine whether a specific therapeutic regimen using Apo2L/TRAIL or death receptor antibody should be performed.

By "nucleic acid" is meant to include any DNA or RNA. For example, chromosomal, mitochondrial, viral and/or bacterial nucleic acid present in tissue sample. The term "nucleic acid" encompasses either or both strands of a double stranded nucleic acid molecule and includes any fragment or portion of an intact nucleic acid molecule.

By "gene" is meant any nucleic acid sequence or portion thereof with a functional role in encoding or transcribing a protein or regulating other gene expression. The gene may consist of all the nucleic acids responsible for encoding a functional protein or only a portion of the nucleic acids responsible for encoding or expressing a protein. The nucleic acid sequence may contain a genetic abnormality within exons, introns, initiation or termination regions, promoter sequences, other regulatory sequences or unique adjacent regions to the gene.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable (*e.g.*, radioisotope labels or fluorescent labels) or, in the case of an enzymatic label, may catalyze chemical alteration of a substrate compound or composition which is detectable.

The term "antibody" herein is used in the broadest sense and specifically covers intact monoclonal antibodies, polyclonal antibodies, multispecific antibodies (*e.g.* bispecific antibodies) formed from at least two intact antibodies, and antibody fragments so long as they exhibit the desired biological activity.

"Antibody fragments" comprise a portion of an intact antibody, preferably comprising the antigen-binding or variable region thereof. Examples of antibody fragments include Fab, Fab', $F(ab')_2$, and Fv fragments; diabodies; linear antibodies; single-chain antibody molecules; and multispecific antibodies formed from antibody fragments.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable or complementary determining regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody-dependent cell-mediated cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an $F(ab')_2$ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the $V_H$-$V_L$ dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), *e.g.*, IgG1, IgG2, IgG3, IgG4, IgA, and IgA2. The heavy-chain constant domains that correspond to the different classes of antibodies are called α, δ, ε, γ, and μ, respectively. The subunit structures and three-dimensional configurations of different classes of immunoglobulins are well known.

"Single-chain Fv" or "scFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the scFv to form the desired structure for antigen binding. For a review of scFv see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, *e.g.*, U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (*e.g.* old World Monkey, such as baboon, rhesus or cynomolgus monkey) and human constant region sequences (US Pat No. 5,693,780).

"Humanized" forms of non-human (*e.g.*, murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

The term "hypervariable region" when used herein refers to the amino acid residues of an antibody which are responsible for antigen-binding. The hypervariable region comprises amino acid residues from a "complementarity determining region" for "CDR" (*e.g.* residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*e.g.* residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). "Framework" or "FR" residues are those variable domain residues other than the hypervariable region residues as herein defined.

An antibody "which binds" an antigen of interest is one capable of binding that antigen with sufficient affinity and/or avidity such that the antibody is useful as a therapeutic or diagnostic agent for targeting a cell expressing the antigen.

For the purposes herein, "immunotherapy" will refer to a method of treating a mammal (preferably a human patient) with an antibody, wherein the antibody may be an unconjugated or "naked" antibody, or the antibody may be conjugated or fused with heterologous molecule(s) or agent(s), such as one or more cytotoxic agent(s), thereby generating an "immunoconjugate".

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino-acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The expression "effective amount" refers to an amount of an agent (e.g. Apo2L/TRAIL, anti-DR4 or DR5 antibody etc.) which is effective for preventing, ameliorating or treating the disease or condition in question.

The terms "treating", "treatment" and "therapy" as used herein refer to curative therapy, prophylactic therapy, and preventative therapy. Consecutive treatment or administration refers to treatment on at least a daily basis without interruption in treatment by one or more days. Intermittent treatment or administration, or treatment or administration in an intermittent fashion, refers to treatment that is not consecutive, but rather cyclic in nature.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors; platelet-growth factor; transforming growth factors (TGFs) such as TGF-$\alpha$

and TNF-β; insulin-like growth factor-I and -II; erythropoietin (EPO); osteoinductive factors; interferons such as interferon-α, -β, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-2, IL-3, IL-4, IL-5, IL-6, IL-7, IL-8, IL-9, IL-11, IL-12, IL-13, IL-17; and other polypeptide factors including LIF and kit ligand (KL). As used herein, the term cytokine includes proteins from natural sources or from recombinant cell culture and biologically active equivalents of the native sequence cytokines.

The term "cytotoxic agent" as used herein refers to a substance that inhibits or prevents the function of cells and/or causes destruction of cells. The term is intended to include radioactive isotopes (*e.g.*, $I^{131}$, $I^{125}$, $Y^{90}$ and $Re^{186}$), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin gammaII and calicheamicin phiI1, see, e.g., Agnew, Chem Intl. Ed. Engl., 33:183-186 (1994); dynemicin, including dynemicin A; bisphosphonates, such as clodronate; an esperamicin; as well as neocarzinostatin chromophore and related chromoprotein enediyne antiobiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (Adriamycin™) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK®; razoxane; rhizoxin; sizofiran; spirogermanium; tenuazonic acid; triaziquone; 2, 2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, NJ) and doxetaxel (TAXOTERE®, Rhône-Poulenc Rorer, Antony, France); chlorambucil; gemcitabine (Gemzar™); 6-thioguanine; mercaptopurine; methotrexate; platinum analogs such as cisplatin and carboplatin; vinblastine; platinum; etoposide (VP-16); ifosfamide; mitoxantrone; vincristine; vinorelbine (Navelbine™); novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included in this definition are anti-hormonal agents that act to regulate or inhibit hormone action on tumors such as anti-estrogens and selective estrogen receptor modulators (SERMs), including, for example, tamoxifen (including Nolvadex™), raloxifene, droloxifene, 4-hydroxytamoxifen, trioxifene, keoxifene, LY117018, onapristone, and toremifene (Fareston™); aromatase inhibitors that inhibit the enzyme aromatase, which regulates estrogen production in the adrenal glands, such as, for example, 4(5)-imidazoles, aminoglutethimide, megestrol acetate (Megace™), exemestane, formestane, fadrozole, vorozole (Rivisor™), letrozole (Femara™), and anastrozole (Arimidex™); and anti-androgens such as flutamide, nilutamide, bicalutamide, leuprolide, and goserelin; and pharmaceutically acceptable salts, acids or derivatives of any of the above.

A "growth inhibitory agent" when used herein refers to a compound or composition which inhibits growth of a cell, especially cancer cell overexpressing any of the genes identified herein, either *in vitro* or *in vivo*. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays (such as Alamar blue assays or MTT assays), FACS analysis, caspase activation, DNA fragmentation (see, for example, Nicoletti et al., J. Immunol. Methods, 139:271-279 (1991), and poly-ADP ribose polymerase, "PARP", cleavage assays known in the art.

As used herein, the term "disorder" in general refers to any condition that would benefit from treatment with the compositions described herein, including any disease or disorder that can be treated by effective amounts of Apo2L/TRAIL, an anti-DR4 antibody, and/or an anti-DR5 antibody. This includes chronic and acute disorders, as well as those pathological conditions which predispose the mammal to the disorder in question. Non-limiting examples of disorders to be treated herein include benign and malignant cancers; inflammatory, angiogenic, and immunologic disorders, autoimmune disorders, arthritis (including rheumatoid arthritis), multiple sclerosis, and HIV/AIDS.

The terms "cancer", "cancerous", or "malignant" refer to or describe the physiological condition in mammals that is typically characterized by unregulated cell growth. Examples of cancer include but are not limited to, carcinoma, lymphoma, leukemia, blastoma, and sarcoma. More particular examples of such cancers include squamous cell carcinoma, myeloma, small-cell lung cancer, non-small cell lung cancer, glioma, hodgkin's lymphoma, non-hodgkin's lymphoma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, stomach cancer, bladder cancer, hepatoma, breast cancer, colon carcinoma, and head and neck cancer.

The term "tagged" when used herein refers to a chimeric molecule comprising an antibody or polypeptide fused to a "tag polypeptide". The tag polypeptide has enough residues to provide an epitope against which an 28 antibody can be made or to provide some other function, such as the ability to oligomerize (e.g. as occurs with peptides having leucine zipper domains), yet is short enough such that it generally does not interfere with activity of the antibody or polypeptide. The tag polypeptide preferably also is fairly unique so that a tag-specific antibody does not substantially cross-react with other epitopes. Suitable tag polypeptides generally have at least six amino acid residues and usually between about 8 to about 50 amino acid residues (preferably, between about 10 to about 20 residues).

The term "divalent metal ion" refers to a metal ion having two positive charges. Examples of divalent metal ions include but are not limited to zinc, cobalt, nickel, cadmium, magnesium, and manganese. Particular forms of such metals that may be employed include salt forms (e.g., pharmaceutically acceptable salt forms), such as chloride, acetate, carbonate, citrate and sulfate forms of the above mentioned divalent metal ions. Optionally, a divalent metal ion for use in the present invention is zinc, and preferably, the salt form, zinc sulfate or zinc chloride.

"Isolated," when used to describe the various peptides or proteins disclosed herein, means peptide or protein that has been identified and separated and/or recovered from a component of its natural environment. Contaminant components of its natural environment are materials that would typically interfere with diagnostic or therapeutic uses for the peptide or protein, and may include enzymes, hormones, and other proteinaceous or nonproteinaceous solutes. In preferred embodiments, the peptide or protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain, or (3) to homogeneity by mass spectroscopic or peptide mapping techniques. Isolated material includes peptide or protein in situ within recombinant cells, since at least one component of its natural environment will not be present. Ordinarily, however, isolated peptide or protein will be prepared by at least one purification step.

"Percent (%) amino acid sequence identity" with respect to the sequences identified herein is defined as the percentage of amino acid residues in a candidate sequence that are identical with the amino acid residues in the reference sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent amino acid identity values can be obtained using the sequence comparison computer program, ALIGN-2, which was authored by Genentech, Inc. and the source code of which has been filed with user documentation in the US Copyright Office, Washington, DC, 20559, registered under the US Copyright Registration No. TXU510087. The ALIGN-2 program is publicly available through Genentech, Inc., South San Francisco, CA. All sequence comparison parameters are set by the ALIGN-2 program and do not vary.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired identity between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"High stringency conditions", as defined herein, are identified by those that: (1) employ low ionic strength and high temperature for washing; 0.015 M sodium chloride/0.0015 M sodium citrate/0.1% sodium dodecyl sulfate at 50°C; (2) employ during hybridization a denaturing agent; 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "primer" or "primers" refers to oligonucleotide sequences that hybridize to a complementary RNA or DNA target polynucleotide and serve as the starting points for the stepwise synthesis of a polynucleotide from mononucleotides by the action of a nucleotidyltransferase, as occurs for example in a polymerase chain reaction.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.*, in a animal model such as that disclosed in Clynes et al. PNAS (USA) 95:652-656 (1998) .

"Human effector cells" are leukocytes which express one or more FcRs and perform effector functions. Preferably, the cells express at least FcγRIII and carry out ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and Fcγ RIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see Daëron, Annu. Rev. Immunol. 15:203-234 (1997))., FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126: 330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)). FcRs herein include polymorphisms such as the genetic dimorphism in the gene that encodes FcγRIIIa resulting in either a phenylalanine (F) or a valine (V) at amino acid position 158, located in the region of the receptor that binds to IgGl. The homozygous valine FcγRIIIa (FcγRIIIa-158V) has been shown to have a higher affinity for human IgG1 and mediate increased ADCC *in vitro* relative to homozygous phenylalanine FcγRIIIa (FcγRIIIa-158F) or heterozygous (FcγRIIIa-158F/V) receptors.

"Complement dependent cytotoxicity" or "CDC" refer to the ability of a molecule to lyse a target in the presence of complement. The complement activation pathway is initiated by the binding of the first component of the complement system (C1q) to a molecule (*e.g.* an antibody) complexed with a cognate antigen. To assess complement activation, a CDC assay, *e.g.* as described in Gazzano-Santoro et al., J. Immunol. Methods 202:163 (1996), may be performed.

**II. EXEMPLARY METHODS AND MATERIALS OF THE INVENTION**

The methods and assays disclosed herein are directed to the examination of expression of one or more biomarkers in a mammalian tissue or cell sample, wherein the determination of that expression of one or more such biomarkers is predictive or indicative of whether the tissue or cell sample will be sensitive to agents such as Apo2L/TRAIL and/or death receptor antibodies such as anti-DR5 agonist antibodies or anti-DR4 agonist antibodies. The methods and assays include those which examine expression of members of the GalNac-T family of molecules, including GalNac-T14 and GalNac-T3.

As discussed above, there are some populations of diseased human cell types (such as certain populations of cancer cells) which are resistant to the cell death inducing effects of Apo2L/TRAIL or death receptor antibodies. It is therefore believed that the disclosed methods and assays can provide for convenient, efficient, and potentially cost-effective means to obtain data and information useful in assessing appropriate or effective therapies for treating patients. For example, a patient having been diagnosed with cancer or an immune related condition could have a biopsy performed to obtain a tissue or cell sample, and the sample could be examined by way of various *in vitro* assays to determine whether the patient's cells would be sensitive to a therapeutic agent such as Apo2L/TRAIL or death receptor antibody.

The invention provides methods for predicting the sensitivity of a mammalian tissue or cell sample (such as a cancer cell) to Apo2L/TRAIL or a death receptor agonist antibody. Optionally, a mammalian tissue or cell sample is obtained and examined for expression of GalNac-T14. The methods may be conducted in a variety of assay formats, including assays detecting mRNA expression, protein expression (such as immunohistochemistry assays) and biochemical assays detecting enzymatic UDP-N-acetyl-D-galactosamine:polypeptide N-acetylgalactosaminyltransferase activity. Determination of expression of such GalNac-T14 biomarkers in (or on) said tissues or cells will be predictive that such tissues or cells will be sensitive to the biological effects of Apo2L/TRAIL and/or death receptor antibody. Applicants surprisingly found that the expression of GalNac-T14 correlates with the sensitivity of such tissues and cells to Apo2L/TRAIL and death receptor agonist antibodies.

As discussed below, expression of various biomarkers such as GalNac-T14 in a sample can be analyzed by a number of methodologies, many of which are known in the art and understood by the skilled artisan, including but not limited to, immunohistochemical and/or Western analysis, quantitative blood based assays (as for example Serum ELISA) (to examine, for example, levels of protein expression), biochemical enzymatic activity assays, *in situ* hybridization, Northern analysis and/or PCR analysis of mRNAs, and genomic Southern analysis (to examine, for example, gene deletion or amplification), as well as any one of the wide variety of assays that can be performed by gene and/or tissue array analysis. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis).

The protocols below relating to detection of GalNac-T14 in a sample are provided below for illustrative purposes.

Optional methods of the invention include protocols which examine or test for presence of GalNac-T14 in a mammalian tissue or cell sample. A variety of methods for detecting GalNac-T14 can be employed and include, for example, immunohistochemical analysis, immunoprecipitation, Western blot analysis, molecular binding assays, ELISA, ELIFA, fluorescence activated cell sorting (FACS), and immunoprecipitation followed by MS, monosaccharide analysis. For example, an optional method of detecting the expression of GalNac-T14 in a tissue or sample comprises contacting the sample with an anti-GalNac-T14 antibody and then detecting the binding of the antibody to GalNac-T14 in the sample.

In particular embodiments of the invention, the expression of GalNac-T14 in a sample is examined using immunohistochemistry and staining protocols. Immunohistochemical staining of tissue sections has been shown to be a reliable method of assessing or detecting presence of proteins in a sample. Immunohistochemistry ("IHC") techniques utilize an antibody to probe and visualize cellular antigens *in situ*, generally by chromogenic or fluorescent methods.

For sample preparation, a tissue or cell sample from a mammal (typically a human patient) may be used. Examples of samples include, but are not limited to, cancer cells such as colon, breast, prostate, ovary, lung, stomach, pancreas, lymphoma, and leukemia cancer cells. Optionally, the samples include non-small cell lung cancer cells, pancreatic cancer cells or non-hodgkin's lymphoma cancer cells. The sample can be obtained by a variety of procedures known in the art including, but not limited to surgical excision, aspiration or biopsy. The tissue may be fresh or frozen. In one embodiment, the sample is fixed and embedded in paraffin or the like.

The tissue sample may be fixed (*i.e.* preserved) by conventional methodology (*See e.g.*, "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C.). One of skill in the art will appreciate that the choice of a fixative is determined by the purpose for which the sample is to be histologically stained or otherwise analyzed. One of skill in the art will also appreciate that the length of fixation depends upon the size of the tissue sample and the fixative used. By way of example, neutral buffered formalin, Bouin's or paraformaldehyde, may be used to fix a sample.

Generally, the sample is first fixed and is then dehydrated through an ascending series of alcohols, infiltrated and embedded with paraffin or other sectioning media so that the tissue sample may be sectioned. Alternatively, one may section the tissue and fix the sections obtained. By way of example, the tissue sample may be embedded and processed in paraffin by conventional methodology (See *e.g.*, "Manual of Histological Staining Method of the Armed Forces Institute of Pathology", *supra*). Examples of paraffin that may be used include, but are not limited to, Paraplast, Broloid, and Tissuemay. Once the tissue sample is embedded, the sample may be sectioned by a microtome or the like (See *e.g.*, "Manual of Histological Staining Method of the Armed Forces Institute of Pathology", *supra*). By way of example for this procedure, sections may range from about three microns to about five microns in thickness. Once sectioned, the sections may be attached to slides by several standard methods. Examples of slide adhesives include, but are not limited to, silane, gelatin, poly-L-lysine and the like. By way of example, the paraffin embedded sections may be attached to positively charged slides and/or slides coated with poly-L-lysine.

If paraffin has been used as the embedding material, the tissue sections are generally deparaffinized and rehydrated to water. The tissue sections may be deparaffinized by several conventional standard methodologies. For example, xylenes and a gradually descending series of alcohols may be used (See *e.g.*, "Manual of Histological Staining Method of the Armed Forces Institute of Pathology", *supra*). Alternatively, commercially available deparaffinizing non-organic agents such as Hemo-De7 (CMS, Houston, Texas) may be used.

Optionally, subsequent to the sample preparation, a tissue section may be analyzed using IHC. IHC may be performed in combination with additional techniques such as morphological staining and/or fluorescence *in-situ* hybridization. Two general methods of IHC are available; direct and indirect assays. According to the first assay, binding of antibody to the target antigen (e.g., GalNac-T14) is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for immunohistochemistry typically will be labeled with a detectable moiety. Numerous labels are available which can be generally grouped into the following categories:

(a) Radioisotopes, such as $^{35}S$, $^{14}C$, $^{125}I$, $^{3}H$, and $^{131}I$. The antibody can be labeled with the radioisotope using the techniques described in Current Protocols in Immunology, Volumes 1 and 2, Coligen et al., Ed. Wiley-Interscience, New York, New York, Pubs. (1991) for example and radioactivity can be measured using scintillation counting.

(b) Colloidal gold particles.

(c) Fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above. The fluorescent labels can be conjugated to the antibody using the techniques disclosed in *Current Protocols in Immunology, supra,* for example. Fluorescence can be quantified using a fluorimeter.

(d) Various enzyme-substrate labels are available and U.S. Patent No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (*e.g.*, firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g.*, glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed. J. Langone & H. Van Vunakis), Academic press, New York, 73:147-166 (1981).

Examples of enzyme-substrate combinations include, for example:

(i) Horseradish peroxidase (HRPO) with hydrogen peroxidase as a substrate, wherein the hydrogen peroxidase oxidizes a dye precursor (*e.g.*, orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (*e.g.*, p-nitrophenyl-β-D-galactosidase) or fluorogenic substrate (*e.g.*, 4-methylumbelliferyl-β-D-galactosidase).

Numerous other enzyme-substrate combinations are available to those skilled in the art. For a general review of these, see U.S. Patent Nos. 4,275,149 and 4,318,980. Sometimes, the label is indirectly conjugated with the antibody. The skilled artisan will be aware of various techniques for achieving this. For example, the antibody can be conjugated with biotin and any of the four broad categories of labels mentioned above can be conjugated with avidin, or *vice versa.* Biotin binds selectively to avidin and thus, the label can be conjugated with the antibody in this indirect manner. Alternatively, to achieve indirect conjugation of the label with the antibody, the antibody is conjugated with a small hapten and one of the different types of labels mentioned above is conjugated with an anti-hapten antibody. Thus, indirect conjugation of the label with the antibody can be achieved.

Aside from the sample preparation procedures discussed above, further treatment of the tissue section prior to, during or following IHC may be desired, For example, epitope retrieval methods, such as heating the tissue sample in citrate buffer may be carried out (see, *e.g.*, Leong et al. Appl. Immunohistochem. 4(3):201 (1996)).

Following an optional blocking step, the tissue section is exposed to primary antibody for a sufficient period of time and under suitable conditions such that the primary antibody binds to the target protein antigen in the tissue sample. Appropriate conditions for achieving this can be determined by routine experimentation. The extent of binding of antibody to the sample is determined by using any one of the detectable labels discussed above. Preferably, the label is an enzymatic label (*e.g.* HRPO) which catalyzes a chemical alteration of the chromogenic substrate such as 3,3'-diaminobenzidine chromogen. Preferably the enzymatic label is conjugated to antibody which binds specifically to the primary antibody (*e.g.* the primary antibody is rabbit polyclonal antibody and secondary antibody is goat anti-rabbit antibody).

Optionally, the antibodies employed in the IHC analysis to detect expression of GalNac-T14 are anti-GalNac-T14 antibodies. Alternatively, antibodies to other GalNac-T antigens which have cross-reactivity with GalNac-T14 may be employed. Optionally, the anti-GalNac-T14 antibody is a monoclonal antibody.

Specimens thus prepared may be mounted and coverslipped. Slide evaluation is then determined, *e.g.* using a microscope, and staining intensity criteria, routinely used in the art, may be employed. Staining intensity criteria may be evaluated as follows:

**TABLE 1**

| Staining Pattern | Score |
|---|---|
| No staining is observed in cells. | 0 |
| Faint/barely perceptible staining is detected in more than 10% of the cells. | 1+ |
| Weak to moderate staining is observed in more than 10% of the cells. | 2+ |
| Moderate to strong staining is observed in more than 10% of the cells. | 3+ |
| | |

Typically, a staining pattern score of about 2+ or higher in such an IHC assay is believed to be predictive or indicative of sensitivity of a mammalian cell (such as a mammalian cancer cell) to Apo2L/TRAIL or a death receptor agonist antibody.

In alternative methods, the sample may be contacted with an antibody specific for said biomarker under conditions sufficient for an antibody-biomarker complex to form, and then detecting said complex. The presence of the biomarker may be accomplished in a number of ways, such as by Western blotting (with or without immunoprecipitation) and ELISA procedures for assaying a wide variety of tissues and samples, including plasma or serum. A wide range of immunoassay techniques using such an assay format are available, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labelled antibody to a target biomarker.

Sandwich assays are among the most useful and commonly used assays. A number of variations of the sandwich assay technique exist, and all are intended to be encompassed by the present invention. Briefly, in a typical forward assay, an unlabelled antibody is immobilized on a solid substrate, and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labelled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40°C such as between 25° C and 32° C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, - galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is.also possible to employ fluorogenic substrates, which yield a fluorescent product rather than the chromogenic substrates noted above. In all cases, the enzyme-labelled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

It is contemplated that the above described techniques may also be employed to detect expression of GalNac-T14.

Methods of the invention further include protocols which examine the presence and/or expression of GalNac-T14 mRNA in a tissue or cell sample. Methods for the evaluation of mRNAs in cells are well known and include, for example, hybridization assays using complementary DNA probes (such as *in situ* hybridization using labeled GalNac-T14 riboprobes, Northern blot and related techniques) and various nucleic acid amplification assays (such as RT-PCR using complementary primers specific for GalNac-T14, and other amplification type detection methods, such as, for example, branched DNA, SISBA, TMA and the like).

Tissue or cell samples from mammals can be conveniently assayed for, e.g., GalNac-T14 mRNAs using Northern, dot blot or PCR analysis. For example, RT-PCR assays such as quantitative PCR assays are well known in the art. In an illustrative embodiment of the invention, a method for detecting a GalNac-T14 mRNA in a biological sample comprises producing cDNA from the sample by reverse transcription using at least one primer; amplifying the cDNA so produced using a GalNac-T14 polynucleotide as sense and antisense primers to amplify GalNac-T14 cDNAs therein; and detecting the presence of the amplified GalNac-T14 cDNA. In addition, such methods can include one or more steps that allow one to determine the levels of GalNac-T14 mRNA in a biological sample (e.g. by simultaneously examining the levels a comparative control mRNA sequence of a "housekeeping" gene such as an actin family member). Optionally, the sequence of the amplified GalNac-T14 cDNA can be determined.

Material embodiments of this aspect of the invention include GalNac-T14 primers and primer pairs, which allow the specific amplification of the polynucleotides of the invention or of any specific parts thereof, and probes that selectively or specifically hybridize to nucleic acid molecules of the invention or to any part thereof. Probes may be labeled with a detectable marker, such as, for example, a radioisotope, fluorescent compound, bioluminescent compound, a chemiluminescent compound, metal chelator or enzyme. Such probes and primers can be used to detect the presence of GalNac-T14 polynucleotides in a sample and as a means for detecting a cell expressing GalNac-T14 proteins. As will be understood by the skilled artisan, a great many different primers and probes may be prepared based on the sequences provided in herein and used effectively to amplify, clone and/or determine the presence and/or levels of GalNac-T14 mRNAs.

Optional methods of the invention include protocols which examine or detect mRNAs, such as GalNac-T14 mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes that have potential to be expressed in certain disease states may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment. (see, e.g., WO 01/75166 published October 11, 2001; (See, for example, U.S. 5,700,637, U.S. Patent 5,445,934, and U.S. Patent 5,807,522, Lockart, Nature Biotechnology, 14:1675-1680 (1996); Cheung, V.G. et al., Nature Genetics 21(Suppl):15-19 (1999) for a discussion of array fabrication). DNA microarrays are miniature arrays containing gene fragments that are either synthesized directly onto or spotted onto glass or other substrates. Thousands of genes are usually represented in a single array. A typical microarray experiment involves the following steps: 1. preparation of fluorescently labeled target from RNA isolated from the sample, 2. hybridization of the labeled target to the microarray, 3. washing, staining, and scanning of the array, 4. analysis of the scanned image and 5. generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (in situ).

The Affymetrix GeneChip® system is a commerically available microarray system which comprises arrays fabricated by direct synthesis of oligonucleotides on a glass surface. Probe/Gene Arrays: Oligonucleotides, usually 25 mers, are directly synthesized onto a glass wafer by a combination of semiconductor-based photolithography and solid phase chemical synthesis technologies. Each array contains up to 400,000 different oligos and each oligo is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligo. The Microarray Suite software subtracts the hybridization intensities of the mismatch probes from those of the perfect match probes to determine the absolute or specific intensity value for each probe set. Probes are chosen based on current information from Genbank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

Fluorescent in-situ hybridization (FISH) assay may also be used to detect expression of the biomarker mRNA using labeled probes. Such assays are known in the art (see, e.g., Kallioniemi et al., 1992; US patent 6,358,682).

The expression of a selected biomarker may also be assessed by examining gene deletion or gene amplification. Gene deletion or amplification may be measured by any one of a wide variety of protocols known in the art, for example, by conventional Southern blotting, Northern blotting to quantitate the transcription of mRNA (Thomas, Proc. Natl. Acad. Sci. USA, 77:5201-5205 (1980)), dot blotting (DNA analysis), or *in situ* hybridization (e.g., FISH), using an appropriately labeled probe, cytogenetic methods or comparative genomic hybridization (CGH) using an appropriately labeled probe. By way of example, these methods may be employed to detect deletion of amplification of GalNac-T14 genes.

Additionally, one can examine the methylation status of the biomarker, such as GalNac-T14 gene, in a tissue or cell sample. Aberrant demethylation and/or hypermethylation of CpG islands in gene 5' regulatory regions frequently occurs in immortalized and transformed cells, and can result in altered expression of various genes. A variety of assays for examining methylation status of a gene are well known in the art. For example, one can utilize, in Southern hybridization approaches, methylation-sensitive restriction enzymes which cannot cleave sequences that contain methylated CpG sites to assess the methylation status of CpG islands. In addition, MSP (methylation specific PCR) can rapidly profile the methylation status of all the CpG sites present in a CpG island of a given gene. This procedure involves initial modification of DNA by sodium bisulfite (which will convert all unmethylated cytosines to uracil) followed by amplification using primers specific for methylated versus unmethylated DNA. Protocols involving methylation interference can also be found for example in Current Protocols In Molecular Biology, Unit 12, Frederick M. Ausubel et al. eds., 1995; De Marzo et al., Am. J. Pathol. 155(6): 1985-1992 (1999); Brooks et al, Cancer Epidemiol. Biomarkers Prev., 1998, 7: 531-536); and Lethe et al., Int. J. Cancer 76(6): 903-908 (1998).

Expression of GalNac-T14 in a tissue or cell sample may also be examined by way of functional or activity-based assays. For instance, one may conduct assays known in the art to determine or detect the presence of the given enzymatic N-aetylgalactosaminyltransferase activity in the tissue or cell sample. (see, e.g., Bennett et al., J. Biol. Chem., 271:17006-17012 (1996); Wang et al., BBRC, 300:738-744 (2003); Hang et al., supra, available May 2005 at www.sciencedirect.com.

In the methods of the present invention, it is contemplated that the tissue or cell sample may also be examined for the expression of Apo2L/TRAIL or receptors in the sample which bind Apo2L/TRAIL. As described above and in the art, it is presently believed Apo2L/TRAIL binds to at least five different receptors: DR4, DR5, DcR1, DcR2, and OPG. Using methods known in the art, including those described herein, the expression of Apo2L/TRAIL, DR4, DR5, DcR1, DcR2 and/or OPG can be detected on the mRNA level and on the protein level. By way of example, the IHC techniques described above may be employed to detect the presence of one of more such molecules in the sample. It is contemplated that in methods in which a tissue or sample is being examined not only for the presence of GalNac-T14 marker, but also for the presence, e.g., DR4, DR5 or DcR1, separate slides may be prepared from the same tissue or sample, and each slide tested with a reagent specific for each specific biomarker or receptor. Alternatively, a single slide may be prepared from the tissue or cell sample, and antibodies directed to each biomarker or receptor may be used in connection with a multi-color staining protocol to allow visualization and detection of the respective biomarkers or receptors.

Subsequent to the determination that the tissue or cell sample expresses GalNac-T14 indicating the tissue or cell sample will be sensitive to Apo2L/TRAIL, it is contemplated that an effective amount of the Apo2L/TRAIL may be administered to the mammal to treat a disorder, such as cancer which is afflicting the mammal. Diagnosis in mammals of the various pathological conditions described herein can be made by the skilled practitioner. Diagnostic techniques are available in the art which allow, e.g., for the diagnosis or detection of cancer or immune related disease in a mammal.

For instance, cancers may be identified through techniques, including but not limited to, palpation, blood analysis, x-ray, NMR and the like. Immune related diseases can also be readily identified.

The Apo2L/TRAIL can be administered in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering Apo2L/TRAIL or death receptor antibody may be determined empirically, and making such determinations is within the skill in the art. Single or multiple dosages may be employed. It is presently believed that an effective dosage or amount of Apo2L/TRAIL used alone may range from about 1 μg/kg to about 100 mg/kg of body weight or more per day. Interspecies scaling of dosages can be performed in a manner known in the art, e.g., as disclosed in Mordenti et al., Pharmaceut. Res., 8:1351 (1991).

When *in vivo* administration of Apo2L/TRAIL is employed, normal dosage amounts may vary from about 10 ng/kg to up to 100 mg/kg of mammal body weight or more per day, preferably about 1 μg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

It is contemplated that yet additional therapies may be employed in the methods. The one or more other therapies may include but are not limited to, administration of radiation therapy, cytokine(s), growth inhibitory agent(s), chemotherapeutic agent(s), cytotoxic agent(s), tyrosine kinase inhibitors, ras farnesyl transferase inhibitors, angiogenesis inhibitors, and cyclin-dependent kinase inhibitors which are known in the art and defined further with particularity above. It is contemplated that such other therapies may be employed as an agent separate from the Apo2L/TRAIL or death receptor antibody. In addition, therapies based on therapeutic antibodies that target tumor antigens such as Rituxan™ or Herceptin™ as well as anti-angiogenic antibodies such as anti-VEGF.

Preparation and dosing schedules for chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the Apo2L/TRAIL or death receptor antibody, or may be given simultaneously therewith.

It may be desirable to also administer antibodies against other antigens, such as antibodies which bind to CD20, CD11a, CD18, CD40, ErbB2, EGFR, ErbB3, ErbB4, vascular endothelial factor (VEGF), or other TNFR family members (such as OPG, TNFR1, TNFR2, GITR, Apo-3, TACI, BCMA, BR3).

Sometimes, it may be beneficial to also administer one or more cytokines to the patient. Following administration, treated cells *in vitro* can be analyzed. Where there has been *in vivo* treatment, a treated mammal can be monitored in various ways well known to the skilled practitioner. For instance, tumor cells can be examined pathologically to assay for necrosis or serum can be analyzed for immune system responses.

For use in the applications described or suggested above, kits or articles of manufacture may be used. Such kits may comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Such probe may be an antibody or polynucleotide specific for GalNac-T14 protein or a GalNac-T14 gene or message, respectively. Where the kit utilizes nucleic acid hybridization to detect the target nucleic acid, the kit may also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label.

The kit will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for either *in vivo* or *in vitro* use, such as those described above.

The kits have a number of embodiments. A typical embodiment is a kit comprising a container, a label on said container, and a composition contained within said container; wherein the composition includes a primary antibody that binds to a GalNac-T14 polypeptide sequence, the label on said container indicates that the composition can be used to evaluate the presence of GalNac-T14 proteins in at least one type of mammalian cell, and instructions for using the GalNac-T14 antibody for evaluating the presence of proteins in at least one type of mammalian cell. The kit can further comprise a set of instructions and materials for preparing a tissue sample and applying antibody and probe to the same section of a tissue sample. The kit may include both a primary and secondary antibody, wherein the secondary antibody is conjugated to a label, *e.g.*, an enzymatic label.

Another embodiment is a kit comprising a container, a label on said container, and a composition contained within said container; wherein the composition includes a polynucleotide that hybridizes to a complement of the GalNac-T14 polynucleotide under stringent conditions, the label on said container indicates that the composition can be used to evaluate the presence of GalNac-T14 in at least one type of mammalian cell, and instructions for using the GalNac-T14 polynucleotide for evaluating the presence of GalNac-T14 RNA or DNA in at least one type of mammalian cell.
Other optional components in the kit include one or more buffers (*e.g.*, block buffer, wash buffer, substrate buffer, etc), other reagents such as substrate (*e.g.*, chromogen) which is chemically altered by an enzymatic label, epitope retrieval solution, control samples (positive and/or negative controls), control slide(s) etc.

## EXAMPLES

Various aspects of the invention are further described and illustrated by way of the examples that follow, none of which are intended to limit the scope of the invention.

### METHODS AND MATERIALS:

#### Cell culture and cell lines.

The following human cell lines: Non-small cell lung cancer (NSCLC) lines: H2122, A427, H647, SK-MES-1, H838, H358, H2126, H460, H1703, H2405, H650, H1568, H1666, H322T, SW1573, H292, H1650, H522, EKVX, H661, H23, LXFL 529, H226, A549, H1781, H1299, HOP 62, H2009, HOP 92, H1793, H1975, H1651, calu-1, H1435, HOP 18, H520, H441, H2030, H1155, H1838, H596, HLFa; Pancreatic cancer lines: Panc 05.04, BxPC3, HPAC, SU.86.86, HuP-T3, PSN1, Panc 08.13, MiaPaCa-2, PA-TU-8988T, Panc 03.27, Capan-1, SW 1990, CFPAC-1, PA-TU-8902, Panc 02.03, Panc 04.03, PL45, Aspc-1, Hs766T, Panc 10.05, Panc1, Capan-2, HPAF-II and NHL: JEKO-1, SU-DHL-4, OCI-LY-19, SR, Farage, DOHH-2, Toledo, WSU-NHL, KARPAS-422, GRANTA-519, Pfeiffer, HT, SC-1, DB. The cell lines were obtained from ATCC Depository (Manassas, Virginia), DSMZ (German Collection of Microorganisms and Cell Cultures), JCRB (Japanese Cell Resources Bank) or ECACC (European Collection of Cell Cultures) and cultured in RPMI-1640 media supplemented with 10% heat inactivated fetal bovine serum, 2 mM L-glutamine and 10 mM HEPES.

#### Cytotoxicity assays.

The MTT assay (CellTiter 96 ® Non-Radioactive Cell Proliferation Assay from Promega), which is a colorimetric assay based on the ability of viable cells to reduce a soluble yellow tetrazolium salt (MTT) to blue formazan crystals), was used to determine the amount of viable cells after treatment with Apo2L/TRAIL or DR5 antibody. The MTT assay was performed by the addition of a premixed optimized dye solution to culture wells of a 96-well plate containing various concentrations (0 to 1000 ng/ml) of Apo2L/TRAIL or DR5 antibody. During a 4-hour incubation, living cells convert the tetrazolium component of the dye solution into a formazan product. The solubilization/stop solution was then added to the culture wells to solubilize the formazan product, and the absorbance at 570nm was recorded using a 96-well plate reader (SpectraMax). The 570nm absorbance reading is directly proportional to the number of cells normally used in proliferation assays. Although the absorbance maximum for the formazan product is 570nm and pure solutions appear blue, the color at the end of the assay may not be blue and depends on the quantity of formazan present relative to other components (including serum, acidified phenol red and unreduced MTT) in the culture medium.
Cell numbers were optimized by performing a cell titration to produce an assay signal near the high end of the linear range of the assay. Since different cell types have different levels of metabolic activity, this was done for each cell line separately. For most tumor cells examined, 5,000 cells per well to 20,000 cells per well were used.
The following is a step by step description of the assays employed:

1. Cells used for bioassay were from stock cultures.
2. Determination of cell number and trypan blue viability, and suspension of the cells to a final number of 5,000 to 20,000 cells per well.
3. Dispensed 50μl of the cell suspension into 96-well plate.
4. Incubation of the plates at 37°C in a humidified 5% $CO_2$ atmosphere over night.
5. Addition of 50μl culture medium containing various concentrations ranging from 0 to 1000 ng/ml of Apo2L/TRAIL or DR5 antibody to samples of the 96-well plate. The controls were 50μl of culture medium (without Apo2L/TRAIL or DR5 antibody) and 100μl culture medium (without cells).

Every experiment was performed in a triplicate set of wells and at three independent days. The total volume of the wells was 100μl/well.

6. Incubation of the plates at 37°C for 72 hours in a humidified 5% $CO_2$ atmosphere.
7. Addition of 15μl of dye solution to each well.
8. Incubation of the plates at 37°C for up to 4 hours in a humidified 5% $CO_2$ atmosphere.
9. Addition of 100μl of the solubilization/stop solution to each well.
10. Incubation of the plates overnight at 37°C overnight.
11. Record the absorbance at 570nm wavelength using a 96-well plate reader. A reference wavelength of 750 nm was used to reduce background contributed by cell debris, fingerprints and other nonspecific absorbance.
12. The average of the absorbance values for the negative control was used as a blank value and subtracted from all other readings. The average of the absorbance values for each concentration of Apo2L/TRAIL or DR5 antibody was divided by the average of the absorbance values of the positive control (100% viable cells - untreated) to calculate the amount of viable cells (in %).
13. Percent viable cells (Y axis) versus concentration of Apo2L/TRAIL or DR5 antibody (X axis, log scale) was plotted and the IC50 value was determined by locating the X-axis value (ng/ml) corresponding to 50 % viable cells.

**Affymetrix Labeling Protocol**

An OD260/280 reading was taken for all samples, and samples were run on the BioAnalyzer. 5 μg high quality Total RNA was used.

A. First Strand cDNA Synthesis:

1. Primer hybridization

| | | |
|---|---|---|
| DEPC-H2O | x μl | Mix by vortexing. Quick spin. |
| RNA (5 ug) | y μl | Incubate at 70°C for 10 minutes. |
| Spike (1:4 dil of stock for 5 ug) | 1 μl | Quick spin and put on ice |
| T7-(dT)24 primer | 1 μl | |
| volume | 12 μl | |

2. Temperature adjustment

| | | |
|---|---|---|
| 5X-1st strand cDNA buffer | 4 μl | |
| Add 7 μl (of the mix to the left) to each sample. | | |
| 0.1 M DTT | 2 μl | Mix by vortexing. Quick spin. |
| 10 mM dNTP mix | 1 μl | Incubate at 42°C for 2 minutes. |
| volume | 7 μl | |

3. First Strand Synthesis

| | | |
|---|---|---|
| Add 1 μl SSII RT to each sample. | | |
| SSII RT | 1 μl | Mix by pipetting up and down -OR- vortex lightly. |
| Quick spin. | | |
| Total volume | 20 μl | Incubate at 42°C for 1 hour. |

B. Second Strand cDNA Synthesis

1. Place First Strand reactions on ice. Centrifuge briefly to bring down condensation on sides of tube.
2. Make the following Second strand master-mix.

| | |
|---|---|
| DEPC-treated H2O | 91 μl |
| 5X-2nd Strand Reaction Buffer | 30 μl |
| 10 mM dNTP mix | 3 μl |
| 10 U/μl DNA Ligase | 1 μl |
| 10 U/μl DNA Polymerase I | 4 μl |
| 2 U/μl RNase H | 1 μl |
| Total volume | 130 μl |

3. Add 130 μl Second strand master-mix to the 20 μl First strand cDNA. (Final volume = 150μl)
4. Mix by pipetting up and down -OR- by vortexing lightly. Quick spin.
5. Incubate at 16°C for 2 hours in a cooling waterbath.
6. Add 2 μl [10 U] T4 DNA Polymerase. Mix by pipetting up and down -OR- vortex lightly. Quick spin.
7. Incubate for 5 minutes at 16°C.
8. Add 10 μl 0.5 M EDTA. Vortex lightly. Quick spin.
9. Proceed to cleanup procedure for cDNA -OR- store at -20°C for later use.

Cleanup of Double-Stranded cDNA (GeneChip Sample Cleanup Module)

1. Add 600 μl cDNA Binding Buffer to the 162 μl final double-stranded cDNA synthesis preparation. Mix by vortexing for 3 seconds.
2. Check that the color of the mixture is yellow (similar to cDNA Binding Buffer w/o the cDNA synthesis rxn.) If the color of the mixture is orange or violet, add 10 μl of 3 M sodium acetate, pH5.0 and mix. The color of the mixture will turn to yellow.
3. Apply 500 μl of the sample to the cDNA Cleanup Spin Column sitting in a 2 ml Collection Tube, and centrifuge for 1 minute at ≥8,000 x g (≥10,000 rpm). Discard flow-through as *hazardous waste.
4. Reload the spin column with the remaining mixture (262 μl) and centrifuge as above. Discard flow-through as *hazardous waste and discard the Collection Tube.
5. Transfer spin column into a new 2 ml Collection Tube (supplied). Pipet 750 μl cDNA Wash Buffer onto the spin column. Centrifuge for 1 minute at ≥8,000 x g (≥10,000 rpm). Discard flow-through.
6. Open the cap of the spin column and centrifuge for 5 minutes at maximum speed (≤ 25,000 x g). Place columns into the centrifuge using every second bucket. Position caps over the adjoining bucket so that they are oriented in the opposite direction to the rotation, i.e., if rotation is clockwise, orient caps in a counter-clockwise direction. This avoids damage to caps. Discard flow-through and Collection Tube.
7. Transfer spin column into a 1.5 ml Collection Tube. Pipet 10 μl of cDNA Elution Buffer directly onto the spin column membrane. Ensure that the cDNA Elution buffer is dispensed directly onto the membrane. Incubate for 1 minute at RT and centrifuge 1 minute at max. speed (≤ 25,000xg) to elute.

Setting Up and Running the IVT Reaction

Enzo: Bioarray HighYield RNA transcript Labeling Kit (Part No. 900182)
1. Use 10 μl of the Cleaned-up Double-stranded cDNA
2. Make the following IVT master-mix:

| | |
|---|---|
| Distilled or Deionized H2O | 12 μl |
| 10X HY Reaction buffer | 4 μl |
| 10x Biotin labeled Ribonucleotides | 4 μl |
| 10X DTT | 4 μl |
| 10X RNase Inhibitor Mix | 4 μl |
| 20X T7 RNA Polymerase | 2 μl |
| Total volume: | 30 μl |

3. Add 30 μl of the IVT master-mix to 10 μl double-stranded cDNA. (Total volume = 40 μl)
4. Mix by pipetting up and down -OR- by vortexing lightly. Quick spin.
5. Immediately place the tube in a 37°C water bath. Incubate for 5 hours.
6. Store at -20°C if not purifying RNA immediately.

Cleanup of Biotin-Labeled cRNA (GeneChip Sample Cleanup Module)

1. Add 60 μl H2O to the IVT reaction and mix by vortexing for 3 seconds.
2. Add 350 μl IVT cRNA Binding Buffer to the sample, mix by vortexing for 3 seconds.
3. Add 250 μl ethanol (96-100%) to the lysate, and mix well by pipetting. Do not centrifuge.
4. Apply sample (700 μl) to the IVT cRNA Cleanup Spin Column sitting in a 2 ml collection tube. Centrifuge for 15 seconds at ≥8,000xg (≥10,000 rpm).

5. Pass the eluate through the column once more.
Centrifuge for 15 seconds at ≥8,000xg (≥10,000 rpm).
Discard the flow-through as **hazardous waste and discard the collection tube.
6. Transfer the spin column into a new 2-ml collection tube (supplied).
7. Add 500 μl IVT cRNA Wash Buffer and centrifuge for 15 seconds at ≥8,000xg (≥10,000 rpm) to wash.
Discard the flow-through.
8. Pipet 500 μl 80% (v/v) ethanol onto the spin column, and centrifuge for 15 seconds at ≥8,000x (≥10,000 rpm). Discard flow-though.
9. Open the cap of the spin column and centrifuge for 5 minutes at max. speed (≤25,000 x g).
Discard flow-through and Collection Tube.
10. Transfer the spin column into a new 1.5 ml collection tube.
11. Pipet 11 μl RNase-free water directly onto the spin column membrane. Let stand for 1 minute.
Centrifuge for 1 minute at maximum speed (≤25,000 x g) to elute.
12. Pipet 10 μl RNase-free water directly onto the spin column membrane. Let stand for 1 minute.
Centrifuge for 1 minute at maximum speed (≤25,000 x g) to elute.

Quantifying the cRNA (IVT Product)

Use spectrophotometric analysis to determine the RNA yield. Apply the convention that 1 OD at 260 nm equals 40 μg/ml RNA.
Check the OD at 260 nm and 280 nm to determine sample concentration and purity.
Maintain the A260/A280 ratio close to 2.0 for pure RNA (ratios between 1.9 and 2.1 are acceptable).
For quantification of cRNA when using total RNA as starting material, an adjusted cRNA yield must be calculated to reflect carryover of unlabeled total RNA. Using an estimate of 100% carryover, use the formula below to determine adjusted cRNA yield:

```
adjusted cRNA yield = RNAm - (total RNAi)(y)
     RNAm = amount of cRNA measured after IVT (µg)
     total RNAi = starting amount of total RNA (µg)
     y = fraction of cDNA reaction used in IVT
```

Fragmenting the cRNA for Target Preparation

For fragmentation, use the adjusted cRNA concentration.
1. Add 2 μl of 5x Fragmentation Buffer for every 8 μl of RNA plus H2O.

| | |
|---|---|
| 20 μg cRNA | 1 to 32 μl |
| 5X Fragmentation Buffer | 8 μl |
| RNase-free water to | 40 μl |
| Total volume: | 40 μl |

2. Incubate at 94°C for 30 minutes. Immediately, put on ice following the incubation.

Preparing the Hybridization Target

1. Heat the 20X Eukaryotic Hybridization Controls and the Oligo B2 for 5 minutes at 65°C.
Affymetrix GeneChip Eukaryotic Hybridization Control Kit, Part #900362 (for 150 rxns)
2. Lightly vortex, spin down.
3. Master mix (Assuming the fragmented cRNA concentration is 0.5 μg/μl):

| Standard Array (μl) | Final Conc. |
|---|---|
| Fragmented cRNA 15 μg 30 | 0.05 μg/μl |
| Oligo B2 (3 nM) 5 | 50 pM |
| 20x Control Spike 15 (Bio B, C, D, Cre) | 1.5, 5, 25, 100 pM |

(continued)

| Standard Array (μl) | Final Conc. |
|---|---|
| Herring Sperm DNA 3 | 0.1 mg/ml |
| Acetylated BSA 3 | 0.5 mg/ml |
| Hu cot-1 DNA (1 mg/ml) 30 | 0.1 mg/ml |
| 2X MES Hyb Buffer 150 | 1X |
| H2O 64 | |
| Final Volume 300 | |

4. Aliquot 270 μl master mix into tubes and add 30 μl of fragmented cRNA to each. This is the Hybridization Mix.
5. Equilibrate the probe arrays to room temperature immediately before use.
6. Fill the probe array with 1x MES Hyb Buffer, and incubate in the rotisserie oven for 10 minutes at 45°C, 60 rpm.
7. Heat the Hybridization Mix in a 99°C waterbath for 5 minutes.
8. Transfer the Hybridization Mix to a 45°C waterbath for 5 minutes.
9. Centrifuge the Hybridization Mix for 5 minutes at maximum speed.
10. Remove the 1x MES Hyb Buffer from the probe arrays.
11. Fill the probe array with the top 200 μl of the Hybridization Mix.
12. Seal the septa with Tough-Spots.
13. Hybridize the probe array at 45°C, 60 RPM for 19 hours.
14. Wash, stain and scan the probe array according to the Affymetrix protocols.

**Affymetrix Materials**

Item Vendor Catalog #

$T_7$-(dT)24 primer Biosearch Technolgies custom

Control spikes in-house -

Superscript II/5X First Strand Buffer/0.1 M DTT Invitrogen 18064-014

5X Second Strand Buffer Invitrogen 10812-014

10 mM dNTP Invitrogen 18427-088

10 U/ul E. coli DNA Ligase Invitrogen 18052-019

10 U/ul E. coli DNA Polymerase I Invitrogen 18010-025

2 U/ul RNase H Invitrogen 18021-071

10 U/ul T4 DNA Polymerase Invitrogen 18005-025

0.5 M EDTA Sigma E-7889

ENZO High Yield RNA Transcript labeling kit Affymetrix or ENZO 900182 (ENZO)

GeneChip Sample Cleanup Module Affymetrix 900371

Acetylated Bovine Serum Albumin Invitrogen 15561-020

Goat IgG - Reagent Grade Sigma 1-5256

Anti-streptavidin antibody (goat), biotinylated Vector Labs BA-0500

R-Phycoerythrin Streptavidin Molecular Probes S-866

20X SSPE BioWhittaker 51214

Eukaryotic Control Kit Affymetrix 900362

Water, Molecular Biology Grade Ambion 9934

Human Cot-1 DNA Roche 1-581-074

5 M NaCl RNase-free, DNase-free Ambion 9760

Antifoam 0-30 Sigma A-8082

10% Tween-20 Pierce Chemical 28320

MES Free Acid Monohydrate Sigma M5287

MES Sodium Salt Sigma M3885

EDTA Disodium Salt, 0.5 M solution Sigma E7889

Tough Spots, Label Dots USA Scientific 9902

GeneChip Hybridization Oven 640 Affymetrix 800139

GeneChip Scanner 3000 w/Workstation Affymetrix 00-0074

Fluidics Station Affymetrix 00-0081

Autoloader w/External Barcode Reader Affymetrix 00-0129

**Quantitative PCR**

cDNA Synthesis :

| Component | Volume (uL) |
|---|---|
| 10X RT Buffer | 10 |
| 25X dNTP mixture | 4 |
| 10X Random Primers | 10 |
| MultiScribe RT (50U/uL) | 5 |
| RNase-free H2O | 21 |
| RNA (100ng) | 50 |
| Final Volume | 100 |

Incubation Conditions :

25° for 10 minutes

37° for 2 hours

TaqMan Reaction using the ABI Prism 7700 Sequencing Detector:

| Component | Volume (uL) |
|---|---|
| TaqMan Universal PCR Master Mix (2X) | 25 |
| TaqMan probe (20X) (Assays-on-Demand™) | 2.5 |
| cDNA (100ng) | 2 |
| H2O | 20.5 |
| Final Volume | 50 |

Thermal Cycling Conditions :

95° for 10 minutes

40 cycles : 95° for 15 seconds
60° for 1 minute

- TaqMan probes : Assays-on-Demand™ (TaqMan® MGB probes, FAM™ dye-labeled)
- Amplification of the endogenous control, GAPDH (probe concentration 100nM, forward & reverse primer concentrations 200nM), was performed to standardize the amount of sample RNA (cDNA) added to each reaction.

Relative quantitation was performed using the standard curve method. For quantitation normalized to an endogenous control, standard curves were prepared for both the target and the endogenous reference. For each experimental sample, the amount of target and endogenous reference was determined from the appropriate standard curve. Then, the target amount was divided by the endogenous reference amount to obtain a normalized target value. One of the experimental samples served as the calibrator, or 1x sample. Each of the normalized target values was then divided by the calibrator normalized target value to generate the relative expression levels.

**Experimental Results:**

Experiments were conducted using the methods and materials described above. Results of these experiments are illustrated in Figures 5-9, as discussed below.

Figure 5 provides an IC50 summary chart of the data obtained in analyzing non-small cell lung cancer ("NSCLC") cell lines for sensitivity or resistance to apoptotic activity of Apo2L (+ 0.5% fetal bovine serum "FBS" or 10% FBS) or DR5 monoclonal antibody "mab", cross-linked "XL" or not crosslinked, + 0.5% fetal bovine serum "FBS" or 10% FBS) as measured in MTT cytotoxicity assays.

Figure 6 provides an IC50 summary chart of the data obtained in analyzing pancreatic cancer cell lines for sensitivity or resistance to apoptotic activity of Apo2L (+ 0.5% fetal bovine serum "FBS" or 10% FBS) or DR5 monoclonal antibody "mab", cross-linked "XL" or not crosslinked, + 0.5% fetal bovine serum "FBS" or 10% FBS) as measured in MTT cytotoxicity assays.

Figure 7 provides an IC50 summary chart of the data obtained in analyzing non-hodgkin's lymphoma cancer ("NHL") cell lines for sensitivity or resistance to apoptotic activity of Apo2L (+ 10% fetal bovine serum "FBS") or DR5 monoclonal antibody "mab", cross-linked "XL" or not crosslinked, + 0.5% fetal bovine serum "FBS" or 10% FBS) as measured in MTT cytotoxicity assays.

Figure 8 provides a comparison of sensitivity ("sen") or resistance ("RES") of select NSCLC, Pancreatic, and NHL cancer cell lines to DR5 antibody and the correlation to expression of GalNac-T14, as measured by GalNac-T14 mRNA expression.

Figure 9 provides a bar diagram graph of various NSCLC, pancreatic, and NHL cell lines ranked (in descending order) by levels of GalNac-T14 mRNA expression patterns.

The apoptotic cell death program plays important roles in the development and homeostasis of multicellular organisms (Danial et al., Cell, 116:205 (2004)). Intracellular stimuli can trigger apoptosis through the cell-intrinsic pathway, which relies on members of the Bcl-2 gene superfamily to activate the apoptotic caspase machinery (Cory et al., Nat.

Rev. Cancer, 2:647 (2002)). Certain cytokines that belong to the tumor necrosis factor (TNF) superfamily can activate apoptosis through the cell-extrinsic pathway, by interacting with some receptors that contain a functional apoptosis-inducing 'death domain' (DD) (Ashkenazi et al., Science, 281:1305 (1998)). Fas ligand (FasL) stimulates apoptosis through Fas (Apol/CD95), while Apo-2 ligand/TNF-related apoptosis-inducing ligand (Apo2L/TRAIL) triggers apoptosis through DR4 (TRAIL-R1) and/or DR5 (TRAIL-R2) (LeBlanc et al., Cell Death Differ., 10:66 (2003)). Upon binding their cognate ligand, these receptors bind the adaptor molecule FADD (Fas associated death domain), which recruits the apoptosis-initiator caspase-8 to form the death-inducing signaling complex (DISC) (see, eg, Kischkel et al., EMBO J., 14:5579 (1995); Kischkel et al., Immunity, 12:611 (2000)). DISC-association stimulates caspase-8, which in turn cleaves and activates effector proteases such as caspase-3, 6, and 7 to execute the apoptotic death program. In many cell types, cross-talk to the cell-intrinsic pathway can further amplify the cell-extrinsic death signal (Scaffidi et al., J. Biol. Chem., 274:1541 (1999)). Apo2L/TRAIL induces apoptosis in a variety of tumor cell types with little or no effect on normal tissues, suggesting that it may be useful for cancer therapy (see, eg, Ashkenazi, Nat. Rev. Cancer, 2:420 (2002)'; Kelley et al., Curr. Opin. Pharmacol., 4:333 (2004)). Alterations in various components of the apoptosis pathways can reduce Apo2L/TRAIL sensitivity in specific cancer cell lines (Igney et al., Nat. Rev. Cancer, 2:277 (2002)).

Various experiments were performed according to the methods and protocols set forth below, and the data is provided in Figures 10-15.

To examine sensitivity to receptor activation, cell survival was tested as a function of Apo2L/TRAIL concentration in a panel of human cancer cell lines, including 23 pancreatic adenocarcinomas, 18 malignant melanomas, and 36 colorectal adenocarcinomas (Fig. 10A and data not shown). This analysis classified 29/77 (38%) of the cell lines as highly or moderately sensitive to Apo2L/TRAIL. The Apo2L/TRAIL concentration required for achieving 50% cell death in these 29 cell lines ranged from 3 to 800 ng/mL, with a mean of 250 ng/mL.

The cell line panel was also examined by gene-expression profiling, using a microarray of 54, 613 gene-probe sets. Albeit with some exceptions, pancreatic cancer and melanoma cell lines that displayed strong or intermediate sensitivity to Apo2L/TRAIL expressed significantly higher mRNA levels of the O-glycosylation enzyme ppGalNAcT-14 than corresponding resistant cell lines ($p=0.5x10^{-1}$ by Fisher's exact test, cutoff assigned iteratively at 750 for pancreatic carcinoma and 300 for melanoma) (Fig. 10B) . Most of the Apo2L/TRAIL-sensitive colorectal cancer cell lines showed high mRNA expression of the related O-glycosylation enzyme ppGalNAcT-3, although several resistant cell lines also expressed this gene, resulting in a weaker, yet significant difference (p=0.026, cutoff assigned at 2000) (Fig. 10C, bottom). Exceptions in the entire panel were: (a) 5/29 (17%) cell lines that were sensitive, yet expressed ppGalNAcT-14 or ppGalNAcT-3 below cutoff levels; (b) 16/48 (33%) resistant cell lines that nonetheless had ppGalNAcT-14 or ppGalNAcT-3 levels above cutoff. By examining mRNA expression of other O-glycosylation enzymes in the colorectal cell lines, higher levels of Fut-6 were detected in 10/12 (83%) sensitive as compared to 6/24 (25%) resistant cell lines (p=0.013; cutoff assigned at 200) (Fig. 10C, top). The combined expression of ppGalNAcT-14 in pancreatic cancer and melanoma with Fut-6 in colorectal cancer cell lines correlated very strongly with Apo2L/TRAIL sensitivity ($p=1.83x10^{-7}$, N=77). This gene-set correctly predicted sensitivity or resistance for 23/32 (72%) marker-positive and 39/45 (87%) marker-negative cell lines, respectively.

Apo2L/TRAIL sensitivity was also examined in vivo using tumor xenografts. A 5-day Apo2L/TRAIL treatment of mice harboring tumors derived from the Fut-6-positive colorectal cancer cell lines Colo205 and DLD-1 caused tumor regression followed by a greatly delayed tumor progression (Fig. 10D). In contrast, tumors derived from the Fut-6-negative colorectal cancer cell lines Colo320 and RKO did not respond to this treatment.

Preincubation of the ppGalNAcT-3/Fut-6-positive Colo205 cell line with the pan O-glycosyl transferase inhibitor benzyl-GalNAc (Delannoy et al., Glycoconj., 13:717 (1996)) markedly reduced sensitivity to Apo2L/TRAIL (Fig. 11A), suggesting a functional link between O-glycosylation and Apo2L/TRAIL signaling. To examine this further, specific small interfering (si)RNA oligonucleotides were used that target ppGalNacT-14, ppGalNacT-3, or Fut-6 mRNA. To exclude off-target effects, multiple, non-overlapping siRNAs were synthesized for each gene and verified their ability to reduce target expression by quantitative RT-PCR (fig. 14A). We confirmed siRNA specificity further with a mutant ppGalNacT-14 plasmid containing 6 'silent' nucleotide changes within the siRNA-targeted region '(Editorial, Nat. Cell. Biol., 5:489 (2003))(fig. 14B). Transfection of the ppGalNAcT-14-positive PSN-1 pancreatic carcinoma and Hs294T melanoma cell lines with ppGalNAcT-14 siRNA substantially reduced sensitivity to Apo2L/TRAIL, while caspase-8 siRNA, as expected, provided essentially complete protection (Fig. 11B). Similarly, transfection of DLD-1 or C170 colorectal cancer cells with GalNAcT-3 or Fut-6 siRNA significantly diminished sensitivity to Apo2L/TRAIL (Fig. 11C and fig. 14C). In sum, GalNAcT-14 siRNA reduced sensitivity to Apo2L/TRAIL in 4/5 pancreatic cancer and 2/2 melanoma cell lines, while ppGalNAcT-3 or Fut-6 siRNA each reduced sensitivity in 2/3 colorectal cancer cell lines. By contrast, transfection of PSN-1 or Hs294T cells with GalNAcT-14 siRNA did not alter sensitivity to the topoisomerase II inhibitor etoposide (fig. 14D). Similarly, transfection of PSN-1 or C170 cells with GalNAcT-14 or GalNAcT-3 siRNA did not affect sensitivity to the broad-spectrum protein kinase inhibitor staurosporine (fig. 14E). Because both etoposide and staurosporine stimulate apoptosis through the cell-intrinsic pathway (Wei et al., Science, 292:727 (2001), these studies suggested that O-glycosylation enzymes may modulate apoptosis signaling through the cell-extrinsic pathway.

Transfection of HEK293 cells with ppGalNAcT-14 revealed cell death when cotransfected with DR4 or DR5, but not the related receptors Fas and TNFR1 or the cell-intrinsic pathway agonist Bax (Fig. 11D). Furthermore, ppGalNAcT-14 transfection increased the Apo2L/TRAIL sensitivity of the resistant cell lines H1568 melanoma (Fig. 11E) and PA-TU-8902 and PL-45 pancreatic carcinoma (Fig. 14F), but did not alter sensitivity to etoposide (data not shown). In total, GalNAcT-14 overexpression sensitized 4/7 cell lines to Apo2L/TRAIL.

The effect of siRNA knockdown of ppGalNacT-14 or Fut-6 was examined on Apo2L/TRAIL-induced caspase processing. In PSN-1 and DLD-1 cells transfected with control siRNA, Apo2L/TRAIL induced essentially complete processing of caspase-8, leading to cleavage of Bid, caspase-9 and caspase-3 (Fig. 12A). Transfection with caspase-8 siRNA prevented these events. Knockdown of ppGalNAcT-14 in PSN-1 cells or Fut-6 in DLD-1 cells also markedly attenuated Apo2L/TRAIL-induced processing of caspase-8, Bid, caspase-9, and caspase-3 (Fig. 12A), and stimulation of caspase-3/7 activity (Fig. 12B). The Apo2L/TRAIL-resistant RKO and SW1417 colorectal cancer cell lines, which express low levels of ppGalNAcT-3 and Fut-6, showed a similar block at the level of caspase-8 processing (fig. 15A). Thus, O-glycosylation enzymes may modulate the Apo2L/TRAIL pathway upstream of events that lead to caspase-8 activation.

Caspase-8 activation requires DISC assembly (Ashkenazi et al., Science, 281:1305 (1998)). Analysis of the Apo2L/TRAIL DISC (Kischkel et al., Immunity, 12:611 (2000)) in PSN-1 and DLD-1 cells indicated that knockdown of ppGalNAcT-14 or Fut-6 reduced the recruitment of FADD and caspase-8 to the DISC, the processing of DISC-bound caspase-8, and the stimulation of DISC-associated caspase-8 enzyme activity (Sharp et al., J. Biol. Chem., 280:19401 (2005)) (Fig. 12C, 12D, and fig. 15B). Neither ppGalNacT-14 nor Fut-6 siRNA substantially altered the amount of DR4 and DR5 in the DISC, or the dose-dependent binding of Apo2L/TRAIL to PSN-1 or DLD-1 cells, which express both DR4 and DR5 (Fig. 12C, fig. 15B, and data not shown). Thus, ppGalNAcT-14 and Fut-6 do not appear to modulate apoptosis by affecting cell-surface receptor levels or Apo2L/TRAIL binding. Consistent with this, Apo2L/TRAIL sensitivity in the 77-cell line panel did not show significant correlation with cell-surface expression of the cognate signaling receptors DR4 and DR5 or decoy receptors DcR1 and DcR2 (data not shown). Furthermore, most siRNAs against ppGalNAcT-14, ppGalNacT-3, or Fut-6 did not alter the levels of DR4 and DR5 on PSN-1, C170, or DLD-1 cells (fig. 15C). Two siRNAs did cause a detectable decrease in DR4 and DR5 in certain cell lines (fig. 15C). However, other siRNAs against the same enzymes inhibited Apo2L/TRAIL-induced apoptosis without affecting receptor levels.

The extracellular domain (ECD) of human DR5 was expressed in chinese hamster ovary cells, the secreted protein purified, subjected to acid hydrolysis, and the associated monosaccharides were analyzed (Fig. 13A). Consistent with the absence of predicted N-glycosylation sites in the DR5 ECD, we did not detect N-linked glycans. However, two samples from 2 independent experiments displayed 3 moles of GalNAc and 3 moles of Gal per mole of DR5 ECD (Fig. 13A), suggesting O-linked modification of three sites on DR5 with the core glycan GalNAc-Gal.

Protein O-glycosylation modifies serines or threonines. Using a previously established bioinformatics tool for prediction of potential O-glycosylation sites (http://www.cbs.dtu.dk/services/NetOGlyc) (Julenius et al., Glycobiology, 15:153 (2005)), we identified two such regions in the common ECD sequence of the long (DR5-L) and short (DR5-S) splice variants of human DR5, and a third site within the alternatively spliced region (Fig. 13B). The first amino acid segment (74-77) contains 3 serines; the second (130-144) has 5 threonines, while the third has 4 threonines and 3 serines. Murine DR5 has sequences similar to the first 2 segments, with 2 serines and 4 threonines, respectively, while human DR4 also has 2 similar sequences containing 1 serine and 5 threonines. To test whether these sites might be important for post-translational modification of DR5, a set of DR5L and DR5S mutants were made, replaced by alanines either the 5 threonines of segment 130-144 (DR5L-5T, DR5S-5T) or these same 5 threonines as well as the 3 serines of segment 74-77 (DR5L-5T3S, DR5S-5T3S). DR5 antibody immunoblot of lysates from HEK293 cells transfected with DR5L or DR5S revealed the presence of the expected DR5L or DR5S bands (Fig. 13C). The antibody also detected DR5 bands of higher molecular weight (MW), which became more abundant upon cotransfection of DR5L or DR5S with ppGalNAcT-14 as compared to control (Fig. 13C, asterisks). The abundance of these higher MW bands and their augmentation by ppGalNAcT-14 were significantly diminished with DR5L-5T or DR5S-5T and nearly abolished with DR5L-5T3S or DR5S-5T3S, as compared to the wild type constructs. These results suggest that the higher MW bands represent O-glycosylated forms of DR5: ppGalNAcT-14 promotes their formation, and progressive elimination of the predicted O-glycosylation sites by alanine substitution gradually reverses this effect. Transfection of HEK293 cells with murine DR5 or human DR4, DR5L, or DR5S revealed cell death (Fig. 13D); each DR5 mutant displayed less activity than its corresponding wildtype construct, with DR5S-5T3S (which lacks all three sites) having the weakest activity. Cotransfection with ppGalNAcT-14 markedly enhanced cell death by all the DR4 and DR5 constructs except DR5S-5T3S, which showed significantly less activity.

A majority of normal-tissue and tumor samples from cancers of the skin, lung, pancreas, breast, ovary, endometrium, and bladder, or from non-Hodgkin's lymphoma displayed ppGalNAcT-14 mRNA expression below cutoff values (determined at 500 for most cancers and at 200 for skin cancers, Fig. 13E). However, a significant subset of the tumor samples, ranging from 10% in lobular breast cancer to 30% in lung cancer and diffuse large B-cell lymphoma, showed overexpression of ppGalNAcT-14. Some cancer samples had mRNA expression levels more than 1000-fold above the corresponding normal tissues. The dynamic expression of ppGalNAcT-14 in cancer suggests that this gene, and possibly other related enzymes, may provide useful biomarkers for identifying tumors with greater sensitivity to Apo2L/TRAIL.

O-linked glycans display extensive structural diversity, and they modulate various aspects of plasma membrane protein biology, including conformation, aggregation, trafficking, half-life, as well as cell adhesion and signaling activity (Hang et al., Bioorg. Med. Chem., 13:5021 (2005); Hanisch, Biol. Chem., 382:143 (2001)). Cancer cells often exhibit dramatic alterations in O-glycan profiles, creating unique tumor-associated carbohydrate antigens (Brockhausen, Biochim. Biophys. Acta, 1473:67 (1999); Dube et al., Nat. Rev. Drug Discovery, 4:477 (2005); Fuster et al., Nat. Rev. Cancer, 5:526 (2005)). O-glycosylation also plays an important role in the homing of tumor cells to specific sites of metastasis (Fuster et al., Cancer Res., 63:2775 (2003); Ohyama et al., EMBO J., 18:1516 (1999); Takada et al., Cancer Res., 53: 354 (1993)). A significant subset of primary tumor samples from a variety of human cancers shows elevated expression of the O-glycosylation enzyme ppGalNAcT-14, including colon and colorectal cell samples, melanoma cell samples and chondrosarcoma cell samples.

## METHODS

### Materials.

Cell culture reagents were purchased from Gibco (Invitrogen/Gibco, Carlsbad, CA), nontagged soluble Apo2L/TRAIL was prepared as described earlier (Lawrence et al., Nat. Med., 7:383 (2001)), the O-linked glycosylation inhibitor Benzyl-a-GalNAc was purchased from Calbiochem and all other chemicals (including etoposide and staurosporine) were from Sigma Aldrich (St. Louis, MO).

### Cell culture and cell lines.

All 119 human carcinoma cell lines (for names and catalog numbers see supplemental data) were obtained from ATCC or DSMZ (Braunschweig, Germany) and cultured at 37°C and 5% $CO_2$ in RPMI1640 supplemented with 10% heat inactivated fetal bovine serum, 2 mM L-glutamine and 10 mM HEPES without antibiotics like penicillin/streptomycin. 293 human embryonic kidney cells (catalog number CRL-1573) were also obtained from ATCC and cultured in 100% Dulbecco's modified Eagle's medium supplemented with 10% FBS. The O-glycosylation mutant CHO cell line, ldlD CHO, was licensed from Dr. Monty Kreiger, MIT (Boston MA).

### Cell viability assays and apoptosis assays.

To determine IC50 for Apo2L/TRAIL, cells were plated in triplicate in 96 well plates, allowed to adhere for 24 hours and then treated with recombinant human Apo2L/TRAIL in increasing concentrations, up to 1000 ng/ml. After a 72h incubation, they were then subjected to a viability assay - MTT assay (Pierce) or CellTiter-Glo Luminescent Cell Viability Assay (Promega) - as per the manufacturer's protocol. Each cell viability experiment was repeated at least three times in low (0.5%) and high (10% FBS) serum and intermediate sensitive cell lines are defined by variability between the IC50s of independent experiments or between low and high serum. We defined a cell line as sensitive based on apoptosis induction of at least 50% of the cells at an Apo2L/TRAIL concentration of 1 ug/ml and as intermediately sensitive based on variability of the amount of apoptosis induced in independent experiments or in presence of low (0.5%) versus high (10%) serum. Apoptosis was quantified by flow cytometric analysis of the average percentage of harvested cells (adherent + floating in the medium) stained with Annexin V (BD Pharmingen).

### Microarray Hybridization and data analysis.

Total cellular RNA was prepared from untreated cells (3 x $10^6$) using the RNeasy Kit (Quiagen). Labeled cRNA was prepared and hybridized to oligonucleotide microarrays (U133P GeneChip; Affymetrix Incorporated, Santa Clara, CA) as described previously (Hoffman et al., Nat. Rev. Genetics, 5:229 (2004); Yauch et al., Clin. Cancer Res., 11:8686 (2005)). Scanned image files were analyzed with GENECHIP 3.1 (Affymetrix), Spotfire, GenePattern and Cluster/TreeView. To identify the most differentially expressed genes between sensitive and resistant cell lines, we subjected gene-expression values to a variation filter that excluded genes with minimal variation across the samples being analyzed by testing for a fold-change and absolute variation over samples, comparing ratio of max and min (max/min) and difference between max and min (max-min) with predefined values and excluding genes not obeying both conditions.

**Expression constructs and retroviral transduction.**

A DNA fragment encoding ppGalNAcT-14 was cloned from cDNA pooled from Apo2L/TRAIL sensitive cell lines and inserted into the expression plasmid pcDNA3.1 (Invitrogen) with an N-terminal Flag tag. This construct was then subjected to site directed mutagenesis (Quikchange Mutagenesis kit, Stratagene) to generate siRNA silent mutants that had 4-6 wobble basepair alterations in the sequence homologous to the siRNA, without changing the protein sequence. The mutations spanned a region of 10bp in the center of the 19bp siRNA binding sequence. The DNA sequences for DR5Long and DR5Short, DR4, murine TRAIL receptor, DR4, Fas (variant 1), TNFR1 and Bax (beta variant) were cloned from cDNA pools and inserted into the pRK expression vector (Genentech). O-glycosylation mutants of DR5L and DR5S were generated by site-specific mutation of four threonine to alanine residues, Mut4xTA (T130, T131, T132, T135) or five threonine to alanine residues Mut5xTA (T130, T131, T132, T135, T143). Transient transfection into HEK293 cells with expression constructs of proapoptotic molecules were done in 6 well plates at a concentration of 0.5 ug/well of the proapoptotic molecule and 2.0 ug of ppGalNAcT-14 or a vector control. Cells were transfected using Lipofectamine 2000 according to the manufacturer's protocol. Following a 48h incubation, cells were subjected to apoptosis analysis.

To generate retroviral constructs ppGalNAcT-14 and mutants were cloned into the pQCXIP retroviral vector (Clontech). High titer retroviral supernatants were generated using the ΦNX-Ampho helper cell line. Packaging cells were transfected using Calcium Phosphate (Invitrogen). Supernatants were isolated 48h after transfection and added to target cells along with 10microg/ml polybrene, followed by a 1h centrifugation step at 2700 rpm to enhance infection. Following transduction, cells were subjected to selection with 2microg/ml puromycin.

**siRNA design and transfection protocols.**

The siRNAs against ppGalNAcT-14, ppGalNAcT-3, Caspase-8 and DR5 were designed by Dharmacon (Lafayette, CO) using their proprietary selection criteria. The selected sequences were:

siGalNAcT-14 (1): 5' GACCATCCGCAGTGTATTA-dTdT 3' (=14-4) (SEQ ID NO:15)
siGalNAcT-14 (2): 5' ATACAGATATGTTCGGTGA-dTdT 3' (=14-6) (SEQ ID NO:16)
siGalNAcT-3 (1): 5' CCATAGATCTGAACACGTT-dTdT 3' (=3-2) (SEQ ID NO:17)
siGalNAcT-3 (2): 5' GCAAGGATATTATACAGCA-dTdT 3' (=3-7) (SEQ ID NO:18)
siFut-6 (1) 5' GUACCAGACACGCGGCAUA-dTdT 3' (=6-1) (SEQ ID NO:19)
siFut-6 (2) 5' ACCGAGAGGUCAUGUACAA-dTdT 3' (=6-2) (SEQ ID NO:20)
siCaspase-8: 5' GGACAAAGTTTACCAAATG-dTdT 3' (SEQ ID NO:21)

siRNAs were purchased as double stranded RNA oligonucleotides and transfected into the respective cell lines at a final concentration of 25 nM for each siRNA. siRNA duplexes against a non-targeting sequence (Dharmacon) was used as a control. Cells were transfected using Lipofectamine2000 (Invitrogen) by reverse transfection where cells are added in suspension to the pre-plated lipid-siRNA complexes. Concentrations for Lipofectamine2000 were as per the manufacturer's protocol. After 48h incubation, cells were harvested for mRNA analysis or incubated with recombinant human Apo2L/TRAIL, etoposide or staurosporine for an additional 24-72h for viability assays or for 4, 8 or 24h for Western blot analysis.

**Inhibition of O-glycosylation with Benzyl-a-GalNAc.**

Colo205 cells were grown in the presence of Benzyl-a-GalNAc (2mM or 4mM) for 72 hours. At this point they were replated into 96 well plates, allowed to adhere for 24 hours, while still in the presence of the inhibitor. They were then stimulated with increasing concentrations of Apo2L/TRAIL as indicated and subjected to a viability assay.

**Quantitative PCR.**

GalNacT-14 and GalNacT-3 transcript expression levels were assessed by quantitative RT-PCR using standard Taqman techniques. Transcript levels were normalized to the housekeeping gene, GAPDH and results are expressed as normalized expression values (=$2^{-DCt}$). Primer/probe sets for the GalNacT-14 (cat#: Hs00226180_ml_GT14), GalNacT-3 (cat#:Hs00237084_ml_GT3) and GAPDH (cat#: 402869) were purchased from Applied Biosystems (Foster City, CA).

**Immunoprecipitation, Western blot analysis and antibodies.**

IP: Anti-Apo2L (2E11; ATCC Accession No. HB-12256), anti-DR4 (3G1 and 4G7, ATCC Accession No. PTA-99) and anti-DR5 (3H3, ATCC Accession No. 12534, and 5C7) monoclonal antibodies were generated at Genentech, Inc. using receptor-Fc fusion proteins as antigens. Anti-DR4 (4G7) and anti-DR5 (5C7) monoclonal antibodies, used to immunoprecipitate the Apo2L/TRAIL DISC, were conjugated to agarose using the ImmunoPure Protein G IgG Plus orientation kit (catalog number 44990) from Pierce. The anti-DR4 (3G1) and anti-DR5 (3H3) monoclonal antibodies, used for immunodetection of DR4/5 in DISC immunoprecipitations, were biotinylated using EZ-link Sulfo-NHS-LC biotinulation kit (catalog number 21217) from Pierce. FLAG-tagged Apo2L/TRAIL was prepared and cross-linked with anti-FLAG antibody M2 (Sigma) as described (Kischkel, Immunity, 12:611 (2000)). These experiments were done as previously described for Apo2L/TRAIL-FLAG + anti-FLAG DISC analysis (Kischkel, supra). The DR4/5 DISC immunoprecipitation experiments also were performed as described, except that anti-DR4 (4G7) and anti-DR5 (5C7) monoclonal antibodies were directly conjugated to agarose for the immunoprecipitation (Sharp et al., J. Biol. Chem., 280:19401 (2005)).

WB: $5x10^5$ cells per well were seeded in 6 well plates. For RNAi knock-down experiments, cells were treated with different siRNAs for 48h followed by Apo2L/TRAIL for 4, 8 or 24h. After the indicated periods of time, cells were washed in ice cold PBS and lysed in 1% Triton X-100 containing hypotonic lysis buffer (20 mM HEPES pH 7.5, 10 mM KCL, 1.5 mM $MgCl_2$, 1 mM EDTA and 1 mM DTT). For each sample 40 μg protein was separated under reducing conditions on 10% or 10-20% gradient SDS-polyacrylamide gels. After transfer to nitrocellulose membranes (Schleicher and Schuell) the membranes were incubated for 1h in 10% non-fat milk powder followed by a 1h incubation with the following primary antibodies: goat anti-caspase-3 antibody (1:1000, R&D) rabbit anti-caspase-8 antibody (1:1000, Pharmingen), mouse anti-caspase-9 antibody 5B4 (1:1000, MBL), rabbit anti-Bid antibody (1:1000, Pharmingen), rabbit anti-DR5 antibody (1:500, Cayman) or goat anti-actin antibody (1:200, Santa Cruz Biotechnology). Membranes were washed five times with TBS / 0.05% Tween and then incubated with the respective peroxidase conjugated affinity purified secondary antibody (1:5000, Biorad) for 30 min. The membranes were washed again five times and developed using enhanced chemiluminescence (ECL, Amersham) and exposed to Kodak Biomax films.

**Flow cytometry/FACS analysis:**

Surface expression of the TNF family receptors DR4 and DR5 was determined by florescence-activated cell sorting (FACS) using a FACS Calibur flow cytometer (Becton Dickinson Immunocytometry System, San Jose, CA). C170 and PSN-1 cells, transfected with indicated siRNAs for 48h, were stained with10 μg/ml primary antibody, 4G7 (anti-DR4) or 3H3 (anti-DR5) or a mouse IgG control antibody for 1 h at 4°C. Cells were then washed with PBS and then incubated with a fluorescein (FITC) conjugated goat anti-mouse secondary antibody (Jackson Laboratories) for 30 min at 4°C. Cells were then analyzed by flow cytometry using a FACS Calibur flow cytometer.

**Caspase assays.**

Caspase-3/-7 activities were assayed at 37°C in 40μl of caspase buffer (50mM HEPES pH 7.4, 100 mM NaCl, 10 % sucrose, 1mM EDTA, 0.1% CHAPS and 10 mM DTT) containing 100 μM of the fluorogenic peptide Ac-DEVD-AFC. Activity was measured continuously over the indicated time by the release of AFC from DEVD-AFC using a Molecular Devices fluorometer in the kinetic mode and with the 405-510 filter pair. For the assessment of caspase activity 20 μg of total cell protein (Triton X-100 extracts) was used in 40 μl of caspase buffer (containing 100 μM DEVD-AFC).

**Carbohydrate analysis of CHO-derived DR5.**

Monosaccharide composition of CHO-cell-derived DR5 was obtained after hydrolysis with 4 N TFA. Analysis of the released monosaccharides was carried out on a Dionex BioLC HPLC system using high-performance anion-exchange chromatography coupled to a pulsed amperometric detector.

**Animals and s.c. xenograft studies.**

Female athymic nude mice (The Jackson Laboratory, Bar Harbor, ME, USA) were acclimated to Genentech's animal housing facility for a minimum of 1 week before placed on experimental study. All of the experimental procedures were approved by Genentech's Institutional Animal Care and Use Committee (IACAUC). Mice were inoculated s.c. with $5 \times 10^6$ cells/mouse of Colo205, DLD-1 and RKO or $20 \times 10^6$ cells/mouse of Colo320HSR human colon carcinoma cells (American Type Culture Collection). Tumor measurements were collected by a digital caliper and tumor volumes calculated using the formula □~□ (A= length) $(B=width)^2$. Once tumors reached a volume of ~150 - 200 $mm^3$, mice were randomly grouped and treatment administered intraperitoneally (i.p) with vehicle or Apo2L/TRAIL (60 mg/kg/day) on days 0 - 4.

## Claims

1. A method for predicting the sensitivity of a mammalian cancer tissue or cell sample to Apo2L/TRAIL, the method comprising examining the tissue sample or cell sample to detect expression of GalNac-T14, wherein expression of said GalNac-T14 is predictive that said tissue sample or cell sample is sensitive to apoptosis-inducing activity of Apo2L/TRAIL.

2. The method of claim 1, wherein said expression of GalNac-T14 is examined by detecting expression of GalNac-T14 mRNA.

3. The method of claim 1, wherein said expression of GalNac-T14 is examined by immunohistochemistry.

4. The method of any one of claims 1 to 3, further comprising the step of examining expression of DR4, DR5, DcR1, or DcR2 receptors in said tissue or cell sample.

5. The method of any one of the preceding claims, wherein said cancer cells or tissue are pancreatic, lymphoma, non-small cell lung cancer, colon cancer, colorectal cancer, melanoma, or chondrosarcoma cells or tissue.

6. The method of claim 5, wherein said cancer cells or tissue are colon or colorectal or non-small cell lung cancer cells or tissue.

7. A method for inducing apoptosis in a mammalian cancer tissue sample or cell sample which comprises:

   examining the tissue sample or cell sample to detect expression of GalNac-T14, and
   subsequent to detecting expression of said GalNac-T14, exposing said tissue sample or cell sample to an effective amount of Apo2L/TRAIL.

8. The method of claim 7, wherein said expression of GalNac-T14 is examined by testing for expression of GalNac-T14 mRNA.

9. The method of claim 7, wherein said expression of GalNac-T14 is examined by immunohistochemistry.

10. The method of any one of claims 7 to 9, further comprising the step of examining expression of DR4, DR5, DcR1 or DcR2 receptors in said tissue or cell sample.

11. The method of any one of claims 7 to 10, wherein said cancer cells or tissue are pancreatic, lymphoma, non-small cell lung cancer, colon cancer, colorectal cancer, melanoma, or chondrosarcoma cells or tissue.

12. The method of claim 11, wherein said cancer cells or tissue are colon or colorectal or non-small cell lung cancer cells or tissue.

13. The method of any one of claims 7 to 12, wherein said cells are exposed to an effective amount of Apo2L/TRAIL polypeptide comprising amino acids 114-281 of Figure 1.

14. The method of any one of claims 7 to 12, wherein said cells are exposed to an effective amount of Apo2L/TRAIL polypeptide comprising amino acids 41-281 of Figure 1 (SEQ ID NO: 1), or a fragment thereof which has apoptotic activity.

15. The method of any one of claims 7 to 14, wherein said Apo2L/TRAIL polypeptide is linked to a polyethylene glycol molecule.

16. Use of Apo2L/TRAIL in the manufacture of a medicament for the treatment of cancer in a mammalian subject, wherein a tissue sample or cell sample from the subject has been determined to express GalNac-T14 and expression of GalNac-T14 is predictive that said tissue or cell sample is sensitive to apoptosis-inducing activity of Apo2L/TRAIL.

17. The use of Apo2L/TRAIL of claim 16, wherein said expression of GalNac-T14 is examined by detecting expression of GalNac-T14 mRNA.

**18.** The use of Apo2L/TRAIL of claim 16, wherein said expression of GalNac-T14 is examined by immunohistochemistry.

**19.** The use of Apo2L/TRAIL of any one of claims 16 to 18, further comprising the step of examining expression of DR4, DR5, DcR1 or DcR2 receptors in said tissue or cell.

**20.** The use of Apo2L/TRAIL of any one of claims 16 to 19, wherein said cancer cells or tissue comprises pancreatic, lymphoma, non-small cell lung cancer, colon cancer, colorectal cancer, melanoma, or chondrosarcoma cells or tissue.

**21.** The use of Apo2L/TRAIL of claim 20, wherein said cancer cells or tissue are colon or colorectal or non-small cell lung cancer cells or tissue.

**22.** The use of Apo2L/TRAIL of any one of claims 16 to 21, wherein the Apo2L/TRAIL polypeptide comprises amino acids 114-281 of Figure 1.

**23.** The use of Apo2L/TRAIL of any one of claims 16 to 22, wherein said cells are exposed to an effective amount of Apo2L/TRAIL polypeptide comprising amino acids 41-281 of Figure 1 (SEQ ID NO: 1), or a fragment thereof which has apoptotic activity.

**24.** The use of Apo2L/TRAIL of any one of claims 16 to 23, wherein the Apo2L/TRAIL is for administration with a chemotherapeutic agent(s) or radiation therapy.

**25.** The use of Apo2L/TRAIL of any one of claims 16 to 24, wherein the Apo2L/TRAIL is for administration with a cytokine, cytotoxic agent or growth inhibitory agent.

**26.** The use of Apo2L/TRAIL of any one of claims 16 to 25, wherein said Apo2L/TRAIL polypeptide is linked to a polyethylene glycol molecule.

**27.** Apo2L/TRAIL for use in a method of treating cancer in a mammalian subject, wherein a tissue sample or cell sample from the subject has been determined to express GalNac-T14 and expression of GalNac-T14 is predictive that said tissue sample or cell sample is sensitive to apoptosis-inducing activity of Apo2L/TRAIL.

**28.** The Apo2L/TRAIL for use in a method of treating cancer of claim 27, wherein said expression of GalNac-T14 is examined by detecting expression of GalNac-T14 mRNA.

**29.** The Apo2L/TRAIL for use in a method of treating cancer of claim 27, wherein said expression of GalNac-T14 is examined by immunohistochemistry.

**30.** The Apo2L/TRAIL for use in a method of treating cancer of claim 27 or claim 28, wherein the expression of DR4, DR5, DcR1 or DcR2 receptors is determined in said tissue or cell sample.

**31.** The Apo2L/TRAIL for use in a method of treating cancer of any one of claims 27 to 30, wherein said cancer cells or tissue comprises pancreatic, lymphoma, non-small cell lung cancer, colon cancer, colorectal cancer, melanoma, or chondrosarcoma cells or tissue.

**32.** The Apo2L/TRAIL for use in a method of treating cancer of claim 31, wherein said cancer cells or tissue are colon or colorectal or non-small cell lung cancer cells or tissue.

**33.** The Apo2L/TRAIL for use in a method of treating cancer of any one of claims 27 to 32, wherein the Apo2L/TRAIL polypeptide comprises amino acids 114-281 of Figure 1.

**34.** The Apo2L/TRAIL for use in a method of treating cancer of any one of claims 27 to 32, wherein said cells are exposed to an effective amount of Apo2L/TRAIL polypeptide comprising amino acids 41-281 of Figure 1 (SEQ ID NO: 1), or a fragment thereof which has apoptotic activity.

**35.** The Apo2L/TRAIL for use in a method of treating cancer of any one of claims 27 to 34, wherein the Apo2L/TRAIL is for administration with a chemotherapeutic agent(s) or radiation therapy.

**36.** The Apo2L/TRAIL for use in a method of treating cancer of any one of claims 27 to 35, wherein the Apo2L/TRAIL is for administration with a cytokine, cytotoxic agent or growth inhibitory agent.

**37.** The Apo2L/TRAIL for use in a method of treating cancer of any one of claims 27 to 36, wherein said Apo2L/TRAIL polypeptide is linked to a polyethylene glycol molecule.

**38.** In vitro Use of an antibody or a polynucleotide to detect expression of GalNac-T14 in a mammalian tissue or cell sample in the for predicting the sensitivity of a mammalian tissue sample or cell sample to Apo2L/TRAIL, wherein expression of said GalNac-T14 is predictive that said tissue sample or cell sample is sensitive to apoptosis-inducing activity of Apo2L/TRAIL.

**39.** The use of claim 38, wherein said expression of GalNac-T14 is examined by detecting mRNA expression of GalNac-T14.

**40.** The use of claim 38, wherein said expression of GalNac-T14 is examined by immunohistochemistry to detect expression of GalNac-T14.

**41.** The use of any one of claims 38 to 40, wherein the detection further comprises examining the expression of DR4, DR5, DcR1, or DcR2 receptors in said tissue or cell sample.

**42.** The use of any one of claims 38 to 41, wherein said cancer cells or tissue are colon, colorectal, pancreatic, melanoma, lymphoma, chondrosarcoma, or non-small cell lung cancer cells or tissue.

**43.** The use of claim 42, wherein said cancer cells or tissue are colon or colorectal or non-small cell lung cancer cells or tissue.

**44.** The use of any one of claims 38 to 43, wherein said Apo2L/TRAIL is a polypeptide comprising amino acids 114-281 of Figure 1 (SEQ ID NO: 1).

**45.** The use of any one of claims 38 to 43, wherein said Apo2L/TRAIL is a polypeptide comprising amino acids 41-281 of Figure 1 (SEQ ID NO: 1) or a biologically active fragment thereof which has apoptotic activity.

**Patentansprüche**

**1.** Verfahren zur Vorhersage der Empfindlichkeit eines Säugetierkrebsgewebes oder einer Zellprobe gegenüber Apo2L/ TRAIL, wobei das Verfahren das Prüfen der Gewebeprobe oder der Zellprobe zum Detektieren der Expression von GalNac-T14 umfasst, worin die Expression des GalNac-T14 die Prognose zulässt, dass die Gewebeprobe oder Zellprobe gegenüber Apoptose induzierender Aktivität von Apo2L/ TRAIL empfindlich ist.

**2.** Verfahren nach Anspruch 1, worin anhand des Detektierens von Expression von GalNac-T14-mRNA auf die Expression von GalNac-T14 geprüft wird.

**3.** Verfahren nach Anspruch 1, worin durch Immunhistochemie auf die Expression von GalNac-T14 geprüft wird.

**4.** Verfahren nach einem der Ansprüche 1 bis 3, das weiters den Schritt des Prüfens auf Expression von DR4-, DR5-, DcR1- oder DcR2-Rezeptoren in der Gewebe- oder Zellprobe umfasst.

**5.** Verfahren nach einem der vorangegangenen Ansprüche, worin es sich bei den Krebszellen oder dem Krebsgewebe um Zellen oder Gewebe von Bauchspeicheldrüse, Lymphomen, nicht-kleinzelligem Lungenkrebs, Kolonkrebs, Kolorektalkrebs, Melanomen oder Chondrosarkomen handelt.

**6.** Verfahren nach Anspruch 5, worin es sich bei den Krebszellen oder dem Krebsgewebe um Zellen oder Gewebe von Kolon-, Kolorektal- oder nicht-kleinzelligem Lungenkrebs handelt.

**7.** Verfahren zur Induktion von Apoptose bei einer Säugetierkrebsgewebeprobe oder -zellprobe, das Folgendes umfasst:

das Prüfen der Gewebeprobe oder Zellprobe, um Expression von GalNac-T14 zu detektieren, und

anschließend an eine Detektion der Expression von GalNac-T14, das Aussetzen der Gewebeprobe oder Zellprobe gegenüber einer wirksamen Menge von Apo2L/TRAIL.

**8.** Verfahren nach Anspruch 7, worin durch Testen auf Expression von GalNac-T14-mRNA auf die Expression von GalNac-T14 geprüft wird.

**9.** Verfahren nach Anspruch 7, worin durch Immunhistochemie auf die Expression von GalNac-T14 geprüft wird.

**10.** Verfahren nach einem der Ansprüche 7 bis 9, das weiters den Schritt des Prüfens auf Expression der Rezeptoren DR4, DR5, DcR1 oder DcR2 in der Gewebe- oder Zellprobe umfasst.

**11.** Verfahren nach einem der Ansprüche 7 bis 10, worin es sich bei den Krebszellen oder dem Krebsgewebe um Zellen oder Gewebe von Bauchspeicheldrüse, Lymphomen, nicht-kleinzelligem Lungenkrebs, Kolonkrebs, Kolorektalkrebs, Melanomen oder Chondrosarkomen handelt.

**12.** Verfahren nach Anspruch 11, worin es sich bei den Krebszellen oder dem Krebsgewebe um Zellen oder Gewebe von Kolon-, Kolorektal- oder nicht-kleinzelligem Lungenkrebs handelt.

**13.** Verfahren nach einem der Ansprüche 7 bis 12, worin die Zellen einer wirksamen Menge an Apo2L/TRAIL-Polypeptid, das die Aminosäuren 114 bis 281 aus Figur 1 umfasst, ausgesetzt werden.

**14.** Verfahren nach einem der Ansprüche 7 bis 12, worin die Zellen einer wirksamen Menge an Apo2L/TRAIL-Polypeptid, das die Aminosäuren 41 bis 281 aus Figur 1 umfasst (Seq.-ID Nr. 1), oder eines Fragments davon, das Apoptoseaktivität aufweist, ausgesetzt werden.

**15.** Verfahren nach einem der Ansprüche 7 bis 14, worin das Apo2L/TRAIL-Polypeptid an ein Polyethylenglykolmolekül gebunden wird.

**16.** Verwendung von Apo2L/TRAIL bei der Herstellung eines Medikaments zur Behandlung von Krebs bei einem Säugetierindividuum, worin von einer Gewebeprobe oder Zellprobe des Individuums bestimmt wurde, dass GalNac-T14 exprimiert wird, und die Expression von GalNac-T14 die Prognose zulässt, dass die Gewebe- oder Zellprobe gegenüber Apoptose induzierender Aktivität von Apo2L/TRAIL empfindlich ist.

**17.** Verwendung von Apo2L/TRAIL nach Anspruch 16, worin anhand des Detektierens von Expression von GalNac-T14-mRNA auf die Expression von GalNac-T14 geprüft wird.

**18.** Verwendung von Apo2L/TRAIL nach Anspruch 16, worin durch Immunhistochemie auf die Expression von GalNac-T14 geprüft wird.

**19.** Verwendung von Apo2L/TRAIL nach einem der Ansprüche 16 bis 18, das weiters den Schritt des Prüfens auf Expression der Rezeptoren DR4, DR5, DcR1 oder DcR2 in dem Gewebe oder der Zelle umfasst.

**20.** Verwendung von Apo2L/TRAIL nach einem der Ansprüche 16 bis 19, worin es sich bei den Krebszellen oder dem Krebsgewebe um Zellen oder Gewebe von Bauchspeicheldrüse, Lymphomen, nicht-kleinzelligem Lungenkrebs, Kolonkrebs, Kolorektalkrebs, Melanomen oder Chondrosarkomen handelt.

**21.** Verwendung von Apo2L/TRAIL nach Anspruch 20, worin es sich bei den Krebszellen oder dem Krebsgewebe um Zellen oder Gewebe von Kolon-, Kolorektal- oder nicht-kleinzelligem Lungenkrebs handelt.

**22.** Verwendung von Apo2L/TRAIL nach einem der Ansprüche 16 bis 21, worin die Zellen einer wirksamen Menge an Apo2L/TRAIL-Polypeptid, das die Aminosäuren 114 bis 281 aus Figur 1 umfasst, ausgesetzt werden.

**23.** Verwendung von Apo2L/TRAIL nach einem der Ansprüche 16 bis 22, worin die Zellen einer wirksamen Menge an Apo2L/TRAIL-Polypeptid, das die Aminosäuren 41 bis 281 aus Figur 1 umfasst (Seq.-ID Nr. 1), oder eines Fragments davon, das Apoptoseaktivität aufweist, ausgesetzt werden.

**24.** Verwendung von Apo2L/TRAIL nach einem der Ansprüche 16 bis 23, worin das Apo2L/TRAIL zur Verabreichung zusammen mit einem oder mehreren chemotherapeutischen Mitteln oder mit Strahlentherapie dient.

**25.** Verwendung von Apo2L/TRAIL nach einem der Ansprüche 16 bis 24, worin das Apo2L/TRAIL zur Verabreichung zusammen mit einem Zytokin, einem zytotoxischen Mittel oder einem wachstumshemmenden Mittel dient.

**26.** Verwendung von Apo2L/TRAIL nach einem der Ansprüche 16 bis 25, worin das Apo2L/TRAIL-Polypeptid an ein Polyethylenglykolmolekül gebunden ist.

**27.** Apo2L/TRAIL zur Verwendung in einem Verfahren zur Behandlung von Krebs bei einem Säugetierindividuum, worin von einer Gewebeprobe oder Zellprobe des Individuums bestimmt wurde, dass sie GalNac-T14 exprimiert, und die Expression von GalNac-T14 die Prognose zulässt, dass die Gewebe- oder Zellprobe gegenüber Apoptose induzierender Aktivität von Apo2L/TRAIL empfindlich ist.

**28.** Apo2L/TRAIL zur Verwendung in einem Verfahren zur Behandlung von Krebs nach Anspruch 27, worin anhand des Detektierens von Expression von GalNac-T14-mRNA auf die Expression von GalNac-T14 geprüft wird.

**29.** Apo2L/TRAIL zur Verwendung in einem Verfahren zur Behandlung von Krebs nach Anspruch 27, worin durch Immunhistochemie auf die Expression von GalNac-T14 geprüft wird.

**30.** Apo2L/TRAIL zur Verwendung in einem Verfahren zur Behandlung von Krebs nach Anspruch 27 oder Anspruch 28, worin die Expression der Rezeptoren DR4, DR5, DcR1 oder DcR2 in der Gewebe- oder Zellprobe bestimmt wird.

**31.** Apo2L/TRAIL zur Verwendung in einem Verfahren zur Behandlung von Krebs nach einem der Ansprüche 27 bis 30, worin es sich bei den Krebszellen oder dem Krebsgewebe um Zellen oder Gewebe von Bauchspeicheldrüse, Lymphomen, nicht-kleinzelligem Lungenkrebs, Kolonkrebs, Kolorektalkrebs, Melanomen oder Chondrosarkomen handelt.

**32.** Apo2L/TRAIL zur Verwendung in einem Verfahren zur Behandlung von Krebs nach Anspruch 31, worin es sich bei den Krebszellen oder dem Krebsgewebe um Zellen oder Gewebe von Kolon-, Kolorektal- oder nicht-kleinzelligem Lungenkrebs handelt.

**33.** Apo2L/TRAIL zur Verwendung in einem Verfahren zur Behandlung von Krebs nach einem der Ansprüche 27 bis 32, worin das Apo2L/TRAIL-Polypeptid die Aminosäuren 114 bis 281 aus Figur 1 umfasst.

**34.** Apo2L/TRAIL zur Verwendung in einem Verfahren zur Behandlung von Krebs nach einem der Ansprüche 27 bis 32, worin die Zellen einer wirksamen Menge an Apo2L/TRAIL-Polypeptid, das die Aminosäuren 41 bis 281 aus Figur 1 (Seq.-ID Nr. 1) umfasst, oder eines Fragments davon, das Apoptoseaktivität aufweist, ausgesetzt werden.

**35.** Apo2L/TRAIL zur Verwendung in einem Verfahren zur Behandlung von Krebs nach einem der Ansprüche 27 bis 34, worin der Apo2L/TRAIL zur Verabreichung zusammen mit einem oder mehreren chemotherapeutischen Mitteln oder mit Strahlentherapie dient.

**36.** Apo2L/TRAIL zur Verwendung in einem Verfahren zur Behandlung von Krebs nach einem der Ansprüche 27 bis 35, worin der Apo2L/TRAIL zur Verabreichung zusammen mit einem Zytokin, einem zytotoxischen Mittel oder einem wachstumshemmenden Mittel dient.

**37.** Apo2L/TRAIL zur Verwendung in einem Verfahren zur Behandlung von Krebs nach einem der Ansprüche 27 bis 36, worin das Apo2L/TRAIL-Polypeptid an ein Polyethylenglykolmolekül gebunden ist.

**38.** In-vitro-Verwendung eines Antikörpers oder eines Polynucleotids zum Detektieren der Expression von GalNac-T14 in einer Säugetiergewebe- oder -zellprobe zur Vorhersage der Empfindlichkeit einer Säugetiergewebeprobe oder -zellprobe gegenüber Apo2L/TRAIL, worin die Expression des GalNac-T14 die Prognose zulässt, dass die Gewebeprobe oder Zellprobe gegenüber Apoptose induzierender Aktivität von Apo2L/TRAIL empfindlich ist.

**39.** Verwendung nach Anspruch 38, worin anhand des Detektierens von Expression von GalNac-T14-mRNA auf die Expression von GalNac-T14 geprüft wird.

**40.** Verwendung nach Anspruch 38, worin durch Immunhistochemie auf die Expression von GalNac-T14 geprüft wird, um die Expression von GalNac-T14 zu detektieren.

**41.** Verwendung nach einem der Ansprüche 38 bis 40, worin die Detektion weiters das Prüfen auf Expression der Rezeptoren DR4, DR5, DcR1 oder DcR2 in der Gewebe- oder Zellprobe umfasst.

**42.** Verwendung nach einem der Ansprüche 38 bis 41, worin es sich bei den Krebszellen oder dem Krebsgewebe um Zellen oder Gewebe von Bauchspeicheldrüse, Lymphomen, nicht-kleinzelligem Lungenkrebs, Kolonkrebs, Kolorektalkrebs, Melanomen oder Chondrosarkomen handelt.

**43.** Verwendung nach Anspruch 42, worin es sich bei den Krebszellen oder dem Krebsgewebe um Zellen oder Gewebe von Kolon-, Kolorektal- oder nicht-kleinzelligem Lungenkrebs handelt.

**44.** Verwendung nach einem der Ansprüche 38 bis 43, worin der Apo2L/TRAIL ein Polypeptid ist, das die Aminosäuren 114 bis 281 aus Figur 1 umfasst.

**45.** Verwendung nach einem der Ansprüche 38 bis 43, worin der Apo2L/TRAIL ein Polypeptid, das die Aminosäuren 41 bis 281 aus Figur 1 (Seq.-ID Nr. 1) umfasst, oder ein Fragment davon ist, das Apoptoseaktivität aufweist.

**Revendications**

**1.** Procédé pour prévoir la sensibilité d'un échantillon de tissu cancéreux ou de cellules cancéreuses d'un mammifère à Apo2L/TRAIL, le procédé comprenant l'étude de l'échantillon de tissu ou de l'échantillon de cellules pour détecter l'expression de GalNac-T14, dans lequel l'expression dudit GalNac-T14 permet de prévoir que ledit échantillon de tissu ou échantillon de cellules est sensible à l'activité inductrice d'apoptose d'Apo2L/TRAIL.

**2.** Procédé suivant la revendication 1, dans lequel ladite expression de GalNac-T14 est étudiée en détectant l'expression de l'ARNm de GalNac-T14.

**3.** Procédé suivant la revendication 1, dans lequel ladite expression de GalNac-T14 est étudiée par immunohistochimie.

**4.** Procédé suivant l'une quelconque des revendications 1 à 3, comprenant en outre l'étape d'étude de l'expression de récepteurs DR4, DR5, DcR1 ou DcR2 dans ledit échantillon de tissu ou échantillon de cellules.

**5.** Procédé suivant l'une quelconque des revendications précédentes, dans lequel lesdites cellules cancéreuses ou ledit tissu cancéreux sont des cellules ou un tissu du pancréas, d'un lymphome, d'un cancer pulmonaire non à petites cellules, d'un cancer du côlon, d'un cancer colorectal, d'un mélanome ou d'un chondrosarcome.

**6.** Procédé suivant la revendication 5, dans lequel lesdites cellules cancéreuses ou ledit tissu cancéreux sont des cellules cancéreuses ou un tissu cancéreux du côlon ou du système colorectal ou bien du cancer pulmonaire non à petites cellules.

**7.** Procédé pour induire une apoptose dans un échantillon de tissu cancéreux ou échantillon de cellules cancéreuses de mammifère, qui comprend :

l'étude de l'échantillon de tissu ou de l'échantillon de cellules pour détecter l'expression de GalNac-T14, et après la détection de l'expression dudit GalNac-T14, l'exposition dudit échantillon de tissu ou dudit échantillon de cellules à une quantité efficace d'Apo2L/TRAIL.

**8.** Procédé suivant la revendication 7, dans lequel ladite expression de GalNac-T14 est étudiée en testant l'expression de l'ARNm de GalNac-T14.

**9.** Procédé suivant la revendication 7, dans lequel ladite expression de GalNac-T14 est étudiée par immunohistochimie.

**10.** Procédé suivant l'une quelconque des revendications 7 à 9, comprenant en outre l'étape d'étude de l'expression de récepteurs DR4, DR5, DcR1 ou DcR2 dans ledit échantillon de tissu ou échantillon de cellules.

**11.** Procédé suivant l'une quelconque des revendications 7 à 10, dans lequel lesdites cellules cancéreuses ou ledit tissu cancéreux sont des cellules ou un tissu du pancréas, d'un lymphome, d'un cancer pulmonaire non à petites cellules, d'un cancer du côlon, d'un cancer colorectal, d'un mélanome ou d'un chondrosarcome.

**12.** Procédé suivant la revendication 11, dans lequel lesdites cellules cancéreuses ou ledit tissu cancéreux sont des cellules ou un tissu de cancer du côlon ou du système colorectal ou bien du cancer pulmonaire non à petites cellules.

**13.** Procédé suivant l'une quelconque des revendications 7 à 12, dans lequel lesdites cellules sont exposées à une quantité efficace de polypeptide Apo2L/TRAIL comprenant les aminoacides 114-281 de la figure 1.

**14.** Procédé suivant l'une quelconque des revendications 7 à 12, dans lequel lesdites cellules sont exposées à une quantité efficace de polypeptide Apo2L/TRAIL comprenant les aminoacides 41-281 de la figure 1 (SEQ ID NO:1), ou d'un de ses fragments ayant une activité apoptosique.

**15.** Procédé suivant l'une quelconque des revendications 7 à 14, dans lequel ledit polypeptide Apo2L/TRAIL est lié à une molécule de polyéthylèneglycol.

**16.** Utilisation d'Apo2L/TRAIL dans la production d'un médicament pour le traitement d'un cancer chez un sujet mammifère, dans laquelle il a été déterminé qu'un échantillon de tissu ou échantillon de cellules provenant du sujet exprime GalNac-T14, et l'expression de GalNac-T14 permet de prévoir que ledit échantillon de tissu ou échantillon de cellules est sensible à l'activité inductrice d'apoptose d'Apo2L/TRAIL.

**17.** Utilisation d'Apo2L/TRAIL suivant la revendication 16, dans laquelle ladite expression de GalNac-T14 est étudiée en détectant l'expression de l'ARNm de GalNac-T14.

**18.** Utilisation d'Apo2L/TRAIL suivant la revendication 16, dans laquelle ladite expression de GalNac-T14 est étudiée par immunohistochimie.

**19.** Utilisation d'Apo2L/TRAIL suivant l'une quelconque des revendications 16 à 18, comprenant en outre l'étape d'étude de l'expression de récepteurs DR4, DR5, DcR1 ou DcR2 dans ledit tissu ou ladite cellule.

**20.** Utilisation d'Apo2L/TRAIL suivant l'une quelconque des revendications 16 à 19, dans laquelle lesdites cellules cancéreuses ou ledit tissu cancéreux comprennent des cellules ou un tissu du pancréas, d'un lymphome, d'un cancer pulmonaire non à petites cellules, d'un cancer du côlon, d'un cancer colorectal, d'un mélanome ou d'un chondrosarcome.

**21.** Utilisation d'Apo2L/TRAIL suivant la revendication 20, dans laquelle lesdites cellules cancéreuses ou ledit tissu cancéreux sont des cellules ou un tissu de cancer du côlon ou du système colorectal ou bien du cancer pulmonaire non à petites cellules.

**22.** Utilisation d'Apo2L/TRAIL suivant l'une quelconque des revendications 16 à 21, dans laquelle le polypeptide Apo2L/TRAIL comprend les aminoacides 114-281 de la figure 1.

**23.** Utilisation d'Apo2L/TRAIL suivant l'une quelconque des revendications 16 à 22, dans laquelle lesdites cellules sont exposées à une quantité efficace de polypeptide Apo2L/TRAIL comprenant les aminoacides 41-281 de la figure 1 (SEQ ID NO:1), ou d'un de ses fragments ayant une activité apoptosique.

**24.** Utilisation d'Apo2L/TRAIL suivant l'une quelconque des revendications 16 à 23, dans laquelle l'Apo2L/TRAIL est utilisé pour l'administration avec un ou plusieurs agents chimiothérapeutiques ou avec une radiothérapie.

**25.** Utilisation d'Apo2L/TRAIL suivant l'une quelconque des revendications 16 à 24, dans laquelle l'Apo2L/TRAIL est utilisé pour l'administration avec une cytokine, un agent cytotoxique ou un agent inhibiteur de croissance.

**26.** Utilisation d'Apo2L/TRAIL suivant l'une quelconque des revendications 16 à 25, dans laquelle ledit polypeptide Apo2L/TRAIL est lié à une molécule de polyéthylèneglycol.

**27.** Apo2L/TRAIL pour une utilisation dans une méthode de traitement du cancer chez un sujet mammifère, où il a été déterminé qu'un échantillon de tissu ou échantillon de cellules provenant du sujet exprime GalNac-T14, et l'expression de GalNac-T14 permet de prévoir que ledit échantillon de tissu ou échantillon de cellules est sensible à l'activité inductrice d'apoptose d'Apo2L/TRAIL.

**28.** Apo2L/TRAIL pour une utilisation dans une méthode de traitement du cancer suivant la revendication 27, où ladite expression de GalNac-T14 est étudiée en détectant l'expression de l'ARNm de GalNac-T14.

**29.** Apo2L/TRAIL pour une utilisation dans une méthode de traitement du cancer suivant la revendication 27, où ladite expression de GalNac-T14 est étudiée par immunohistochimie.

**30.** Apo2L/TRAIL pour une utilisation dans une méthode de traitement du cancer suivant la revendication 27 ou la revendication 28, où l'expression de récepteurs DR4, DR5, DcR1 ou DcR2 est déterminée dans ledit échantillon de tissu ou échantillon de cellules.

**31.** Apo2L/TRAIL pour une utilisation dans une méthode de traitement du cancer suivant l'une quelconque des revendications 27 à 30, où lesdites cellules cancéreuses ou ledit tissu cancéreux comprennent des cellules ou un tissu du pancréas, d'un lymphome, d'un cancer pulmonaire non à petites cellules, d'un cancer du côlon, d'un cancer colorectal, d'un mélanome ou d'un chondrosarcome.

**32.** Apo2L/TRAIL pour une utilisation dans une méthode de traitement du cancer suivant la revendication 31, où lesdites cellules cancéreuses ou ledit tissu cancéreux sont des cellules ou un tissu du côlon ou du système colorectal ou bien d'un cancer pulmonaire non à petites cellules.

**33.** Apo2L/TRAIL pour une utilisation dans une méthode de traitement du cancer suivant l'une quelconque des revendications 27 à 32, où le polypeptide Apo2L/TRAIL comprend les aminoacides 114-281 de la figure 1.

**34.** Apo2L/TRAIL pour une utilisation dans une méthode de traitement du cancer suivant l'une quelconque des revendications 27 à 32, où lesdites cellules sont exposées à une quantité efficace de polypeptide Apo2L/TRAIL comprenant les aminoacides 41-281 de la figure 1 (SEQ ID NO:1), ou d'un de ses fragments ayant une activité apoptosique.

**35.** Apo2L/TRAIL pour une utilisation dans une méthode de traitement du cancer suivant l'une quelconque des revendications 27 à 34, où l'Apo2L/TRAIL est utilisé pour l'administration avec un ou plusieurs agents chimiothérapeutiques ou une radiothérapie.

**36.** Apo2L/TRAIL pour une utilisation dans une méthode de traitement du cancer suivant l'une quelconque des revendications 27 à 35, où l'Apo2L/TRAIL est utilisé pour l'administration avec une cytokine, un agent cytotoxique ou un agent inhibiteur de croissance.

**37.** Apo2L/TRAIL pour une utilisation dans une méthode de traitement du cancer suivant l'une quelconque des revendications 27 à 36, où ledit polypeptide Apo2L/TRAIL est lié à une molécule de polyéthylèneglycol.

**38.** Utilisation in vitro d'un anticorps ou d'un polynucléotide pour détecter l'expression de GalNac-T14 dans un échantillon de tissu ou de cellules d'un mammifère pour prévoir la sensibilité d'un échantillon de tissu ou échantillon de cellules de mammifère à Apo2L/TRAIL, dans laquelle l'expression dudit GalNac-T14 permet de prévoir que ledit échantillon de tissu ou échantillon de cellules est sensible à l'activité inductrice d'apoptose d'Apo2L/TRAIL.

**39.** Utilisation suivant la revendication 38, dans laquelle ladite expression de GalNac-T14 est étudiée en détectant l'expression de l'ARNm de GalNac-T14.

**40.** Utilisation suivant la revendication 38, dans laquelle ladite expression de GalNac-T14 est étudiée par immunohistochimie pour détecter l'expression de GalNac-T14.

**41.** Utilisation suivant l'une quelconque des revendications 38 à 40, dans laquelle la détection comprend en outre l'étude de l'expression de récepteurs DR4, DR5, DcR1 ou DcR2 dans ledit échantillon de tissu ou échantillon de cellules.

**42.** Utilisation suivant l'une quelconque des revendications 38 à 41, dans laquelle lesdites cellules cancéreuses ou ledit tissu cancéreux sont des cellules ou un tissu du côlon, du système colorectal, du pancréas, d'un mélanome, d'un lymphome, d'un chondrosarcome ou d'un cancer pulmonaire non à petites cellules.

**43.** Utilisation suivant la revendication 42, dans laquelle lesdites cellules cancéreuses ou ledit tissu cancéreux sont des cellules ou un tissu du côlon ou du système colorectal ou d'un cancer pulmonaire non à petites cellules.

**44.** Utilisation suivant l'une quelconque des revendications 38 à 43, dans laquelle ledit Apo2L/TRAIL est un polypeptide comprenant les aminoacides 114-281 de la figure 1 (SEQ ID NO:1).

**45.** Utilisation suivant l'une quelconque des revendications 38 à 43, dans laquelle ledit Apo2L/TRAIL est un polypeptide comprenant les aminoacides 41-281 de la figure 1 (SEQ ID NO:1) ou un de ses fragments biologiquement actifs ayant une activité apoptosique.

```
1    TTTCCTCACTGACTATAAAAGAATAGAGAAGGAAGGGCTTCAGTGACCGGCTGCCTGGCTGACTTACAGCAGTCAGACTCTGACAGGATC

1    ATGGCTATGATGGAGGTCCAGGGGGGACCCAGCCTGGGACAGACCTGCGTGCTGATCGTGATCTTCACAGTGCTCCTGCAGTCTCTCTGT
1    MetAlaMetMetGluValGlnGlyGlyProSerLeuGlyGlnThrCysValLeuIleValIlePheThrValLeuLeuGlnSerLeuCys

181  GTGGCTGTAACTTACGTGTACTTTACCAACGAGCTGAAGCAGATGCAGGACAAGTACTCCAAAAGTGGCATTGCTTGTTTCTTAAAAGAA
31   ValAlaValThrTyrValTyrPheThrAsnGluLeuLysGlnMetGlnAspLysTyrSerLysSerGlyIleAlaCysPheLeuLysGlu

271  GATGACAGTTATTGGGACCCCAATGACGAAGAGAGTATGAACAGCCCCTGCTGGCAAGTCAAGTGGCAACTCCGTCAGCTCGTTAGAAAG
61   AspAspSerTyrTrpAspProAsnAspGluGluSerMetAsnSerProCysTrpGlnValLysTrpGlnLeuArgGlnLeuValArgLys

361  ATGATTTTGAGAACCTCTGAGGAAACCATTTCTACAGTTCAAGAAAAGCAACAAAATATTTCTCCCCTAGTGAGAGAAAGAGGTCCNCAG
91   MetIleLeuArgThrSerGluGluThrIleSerThrValGlnGluLysGlnGlnAsnIleSerProLeuValArgGluArgGlyProGln
                                                                                    *
451  AGAGTAGCAGCTCACATAACTGGGACCAGAGGAAGAAGCAACACATTGTCTTCTCCAAACTCCAAGAATGAAAAGGCTCTGGGCCGCAAA
121  ArgValAlaAlaHisIlethrGlyThrArgGlyArgSerAsnThrLeuSerSerProAsnSerLysAsnGluLysAlaLeuGlyArgLys

541  ATAAACTCCTGGGAATCATCAAGGAGTGGGCATTCATTCCTGAGCAACTTGCACTTGAGGAATGGTGAACTGGTCATCCATGAAAAAGGG
151  IleAsnSerTrpGluSerSerArgSerGlyHisSerPheLeuSerAsnLeuHisLeuArgAsnGlyGluLeuValIleHisGluLysGly

631  TTTTACTACATCTATTCCCAAACATACTTTCGATTTCAGGAGGAAATAAAAGAAAACACAAAGAACGACAAACAAATGGTCCAATATATT
181  PheTyrTyrIleTyrSerGlnThrTyrPheArgPheGlnGluGluIleLysGluAsnThrLysAsnAspLysGlnMetValGlnTyrIle

721  TACAAATACACAAGTTATCCTGACCCTATATTGTTGATGAAAAGTGCTAGAAATAGTTGTTGGTCTAAAGATGCAGAATATGGACTCTAT
211  TyrLysTyrThrSerTyrProAspProIleLeuLeuMetLysSerAlaArgAsnSerCysTrpSerLysAspAlaGluTyrGlyLeuTyr

811  TCCATCTATCAAGGGGGAATATTTGAGCTTAAGGAAAATGACAGAATTTTTGTTTCTGTAACAAATGAGCACTTGATAGACATGGACCAT
241  SerIleTyrGlnGlyGlyIlePheGluLeuLysGluAsnAspArgIlePheValSerValThrAsnGluHisLeuIleAspMetAspHis

901  GAAGCCAGTTTTTTCGGGGCCTTTTTAGTTGGCTAACTGACCTGGAAAGAAAAAGCAATAACCTCAAAGTGACTATTCAGTTTTCAGGAT
271  GluAlaSerPhePheGlyAlaPheLeuValGlyStp

991  GATACACTATGAAGATGTTTCAAAAAATCTGACCAAAACAAACAAACAGAAA
```

*FIG. 1*

```
  1 ATGGCGCCAC CACCAGCTAG AGTACATCTA GGTGCGTTCC TGGCAGTGAC
    TACCGCGGTG GTGGTCGATC TCATGTAGAT CCACGCAAGG ACCGTCACTG
  1 MetAlaProP roProAlaAr gValHisLeu GlyAlaPheL euAlaValTh

 51 TCCGAATCCC GGGAGCGCAG CGAGTGGGAC AGAGGCAGCC GCGGCCACAC
    AGGCTTAGGG CCCTCGCGTC GCTCACCCTG TCTCCGTCGG CGCCGGTGTG
    rProAsnPro GlySerAlaA laSerGlyTh rGluAlaAla AlaAlaThrPro

101 CCAGCAAAGT GTGGGGCTCT TCCGCGGGGA GGATTGAACC ACGAGGCGGG
    GGTCGTTTCA CACCCCGAGA AGGCGCCCCT CCTAACTTGG TGCTCCGCCC
 35   SerLysVa lTrpGlySer SerAlaGlyA rgIleGluPr oArgGlyGly

151 GGCCGAGGAG CGCTCCCTAC CTCCATGGGA CAGCACGGAC CCAGTGCCCG
    CCGGCTCCTC GCGAGGGATG GAGGTACCCT GTCGTGCCTG GGTCACGGGC
    GlyArgGlyA laLeuProTh rSerMetGly GlnHisGlyP roSerAlaArg

201 GGCCCGGGCA GGGCGCGCCC CAGGACCCAG GCCGGCGCGG GAAGCCAGCC
    CCGGGCCCGT CCCGCGCGGG GTCCTGGGTC CGGCCGCGCC CTTCGGTCGG
 68   AlaArgAla GlyArgAlaP roGlyProAr gProAlaArg GluAlaSerP

251 CTCGGCTCCG GGTCCACAAG ACCTTCAAGT TTGTCGTCGT CGGGGTCCTG
    GAGCCGAGGC CCAGGTGTTC TGGAAGTTCA AACAGCAGCA GCCCCAGGAC
    roArgLeuAr gValHisLys ThrPheLysP heValValVa lGlyValLeu

301 CTGCAGGTCG TACCTAGCTC AGCTGCAACC ATGATCAATC AATTGGCACA
    GACGTCCAGC ATGGATCGAG TCGACGTTGG TAGTTTGAAG TACTAGTTAG
101 LeuGlnValV alProSerSe rAlaAlaThr IleLysLeuH isAspGlnSe

351 AATTGGCACA CAGCAATGGG AACATAGCCC TTTGGGAGAG TTGTGTCCAC
    TTAACCGTGT GTCGTTACCC TTGTATCGGG AAACCCTCTC AACACAGGTG
    rIleGlyThr GlnGlnTrpG luHisSerPr oLeuGlyGlu LeuCysProPro

401 CAGGATCTCA TAGATCAGAA CGTCCTGGAG CCTGTAACCG GTGCACAGAG
    GTCCTAGAGT ATCTAGTCTT GCAGGACCTC GGACATTGGC CACGTGTCTC
135   GlySerHi sArgSerGlu ArgProGlyA laCysAsnAr gCysThrGlu

451 GGTGTGGGTT ACACCAATGC TTCCAACAAT TTGTTTGCTT GCCTCCCATG
    CCACACCCAA TGTGGTTACG AAGGTTGTTA AACAAACGAA CGGAGGGTAC
    GlyValGlyT yrThrAsnAl aSerAsnAsn LeuPheAlaC ysLeuProCys

501 TACAGCTTGT AAATCAGATG AAGAAGAGAG AAGTCCCTGC ACCACGACCA
    ATGTCGAACA TTTAGTCTAC TTCTTCTCTC TTCAGGGACG TGGTGCTGGT
168   ThrAlaCys LysSerAspG luGluGluAr gSerProCys ThrThrThrA

551 GGAACACAGC ATGTCAGTGC AAACCAGGAA CTTTCCGGAA TGACAATTCT
    CCTTGTGTCG TACAGTCACG TTTGGTCCTT GAAAGGCCTT ACTGTTAAGA
    rgAsnThrAl aCysGlnCys LysProGlyT hrPheArgAs nAspAsnSer

601 GCTGAGATGT GCCGGAAGTG CAGCACAGGG TGCCCCAGAG GGATGGTCAA
    CGACTCTACA CGGCCTTCAC GTCGTGTCCC ACGGGGTCTC CCTACCAGTT
201 AlaGluMetC ysArgLysCy sSerThrGly CysProArgG lyMetValLy

651 GGTCAAGGAT TGTACGCCCT GGAGTGACAT CGAGTGTGTC CACAAAGAAT
    CCAGTTCCTA ACATGCGGGA CCTCACTGTA GCTCACACAG GTGTTTCTTA
    sValLysAsp CysThrProT rpSerAspIl eGluCysVal HisLysGluSer
```

*FIG. 2A*

```
701  CAGGCAATGG ACATAATATA TGGGTGATTT TGGTTGTGAC TTTGGTTGTT
     GTCCGTTACC TGTATTATAT ACCCACTAAA ACCAACACTG AAACCAACAA
235    GlyAsnGl yHisAsnIle TrpValIleL euValValTh rLeuValVal

751  CCGTTGCTGT TGGTGGCTGT GCTGATTGTC TGTTGTTGCA TCGGCTCAGG
     GGCAACGACA ACCACCGACA CGACTAACAG ACAACAACGT AGCCGAGTCC
     ProLeuLeuL euValAlaVa lLeuIleVal CysCysCysI leGlySerGly

801  TTGTGGAGGG GACCCCAAGT GCATGGACAG GGTGTGTTTC TGGCGCTTGG
     AACACCTCCC CTGGGGTTCA CGTACCTGTC CCACACAAAG ACCGCGAACC
268   CysGlyGly AspProLysC ysMetAspAr gValCysPhe TrpArgLeuG

851  GTCTCCTACG AGGGCCTGGG GCTGAGGACA ATGCTCACAA CGAGATTCTG
     CAGAGGATGC TCCCGGACCC CGACTCCTGT TACGAGTGTT GCTCTAAGAC
     lyLeuLeuAr gGlyProGly AlaGluAspA snAlaHisAs nGluIleLeu

901  AGCAACGCAG ACTCGCTGTC CACTTTCGTC TCTGAGCAGC AAATGGAAAG
     TCGTTGCGTC TGAGCGACAG GTGAAAGCAG AGACTCGTCG TTTACCTTTC
301  SerAsnAlaA spSerLeuSe rThrPheVal SerGluGlnG lnMetGluSe

951  CCAGGAGCCG GCAGATTTGA CAGGTGTCAC TGTACAGTCC CCAGGGGAGG
     GGTCCTCGGC CGTCTAAACT GTCCACATGT ACATGTCAGG GGTCCCCTCC
     rGlnGluPro AlaAspLeuT hrGlyValTh rValGlnSer ProGlyGluAla

1001 CACAGTGTCT GCTGGGACCG GCAGAAGCTG AAGGGTCTCA GAGGAGGAGG
     GTGTCACAGA CGACCCTGGC CGTCTTCGAC TTCCCAGAGT CTCCTCCTCC
335    GlnCysLe uLeuGlyPro AlaGluAlaG luGlySerGl nArgArgArg

1051 CTGCTGGTTC CAGCAAATGG TGCTGACCCC ACTGAGACTC TGATGCTGTT
     GACGACCAAG GTCGTTTACC ACGACTGGGG TGACTCTGAG ACTACGACAA
     LeuLeuValP roAlaAsnGl yAlaAspPro ThrGluThrL euMetLeuPhe

1101 CTTTGACAAG TTTGCAAACA TCGTGCCCTT TGACTCCTGG GACCAGCTCA
     GAAACTGTTC AAACGTTTGT AGCACGGGAA ACTGAGGACC CTGGTCGAGT
368   PheAspLys PheAlaAsnI leValProPh eAspSerTrp AspGlnLeuM

1151 TGAGGCAGCT GGACCTCACG AAAAATGAGA TCGATGTGGT CAGAGCTGGT
     ACTCCGTCGA CCTGGAGTGC TTTTTACTCT AGCTACACCA GTCTCGACCA
     etArgGlnLe uAspLeuThr LysAsnGluI leAspValVa lArgAlaGly

1201 ACAGCAGGCC CAGGGGATGC CTTGTATGCA ATGCTGATGA AATGGGTCAA
     TGTCGTCCGG GTCCCCTACG GAACATACGT TACGACTACT TTACCCAGTT
401  ThrAlaGlyP roGlyAspAl aLeuTyrAla MetLeuMetL ysTrpValAs

1251 CAAAACTGGA CGGAACGCCT CGATCCACAC CCTGCTGGAT GCCTTGGAGA
     GTTTTGACCT GCCTTGCGGA GCTAGGTGTG GGACGACCTA CGGAACCTCT
     nLysThrGly ArgAsnAlaS erIleHisTh rLeuLeuAsp AlaLeuGluArg

1301 GGATGGAAGA GAGACATGCA AAAGAGAAGA TTCAGGACCT CTTGGTGGAC
     CCTACCTTCT CTCTGTACGT TTTCTCTTCT AAGTCCTGGA GAACCACCTG
435    MetGluGl uArgHisAla LysGluLysI leGlnAspLe uLeuValAsp

1351 TCTGGAAAGT TCATCTACTT AGAAGATGGC ACAGGCTCTG CCGTGTCCTT
     AGACCTTTCA AGTAGATGAA TCTTCTACCG TGTCCGAGAC GGCACAGGAA
     SerGlyLysP heIleTyrLe uGluAspGly ThrGlySerA laValSerLeu

1401 GGAGTGA
     CCTCACT
468   GluOP*
```

*FIG. 2B*

```
  1 MEQRGQNAPAASGARKRHGPGPREARGARPGLRVPKTLVLVVAAVLLLVSAESALITQQD
 61 LAPQQRAAPQQKRSSPSEGLCPPGHHISEDGRDCISCKYGQDYSTHWNDLLFCLRCTRCD
121 SGEVELSPCTTTRNTVCQCEEGTFREEDSPEMCRKCRTGCPRGMVKVGDCTPWSDIECVH
181 KESGIIIGVTVAAVVLIVAVFVCKSLLWKKVLPYLKGICSGGGGDPERVDRSSQRPGAED
241 NVLNEIVSILQPTQVPEQEMEVQEPAEPTGVNMLSPGESEHLLEPAEAERSQRRRLLVPA
301 NEGDPTETLRQCFDDFADLVPFDSWEPLMRKLGLMDNEIKVAKAEAAGHRDTLYTMLIKW
361 VNKTGRDASVHTLLDALETLGERLAKQKIEDHLLSSGKFMYLEGNADSALS
```

*FIG. 3A*

Met Glu Gln Arg Gly Gln Asn Ala Pro Ala Ala Ser Gly Ala Arg Lys
1 5 10 15

Arg His Gly Pro Gly Pro Arg Glu Ala Arg Gly Ala Arg Pro Gly Pro
20 25 30

Arg Val Pro Lys Thr Leu Val Leu Val Val Ala Ala Val Leu Leu Leu
35 40 45

Val Ser Ala Glu Ser Ala Leu Ile Thr Gln Gln Asp Leu Ala Pro Gln
50 55 60

Gln Arg Ala Ala Pro Gln Gln Lys Arg Ser Ser Pro Ser Glu Gly Leu
65 70 75 80

Cys Pro Pro Gly His His Ile Ser Glu Asp Gly Arg Asp Cys Ile Ser
85 90 95

Cys Lys Tyr Gly Gln Asp Tyr Ser Thr His Trp Asn Asp Leu Leu Phe
100 105 110

Cys Leu Arg Cys Thr Arg Cys Asp Ser Gly Glu Val Glu Leu Ser Pro
115 120 125

Cys Thr Thr Thr Arg Asn Thr Val Cys Gln Cys Glu Glu Gly Thr Phe
130 135 140

Arg Glu Glu Asp Ser Pro Glu Met Cys Arg Lys Cys Arg Thr Gly Cys
145 150 155 160

Pro Arg Gly Met Val Lys Val Gly Asp Cys Thr Pro Trp Ser Asp Ile
165 170 175

Glu Cys Val His Lys Glu Ser Gly Thr Lys His Ser Gly Glu Ala Pro
180 185 190

Ala Val Glu Glu Thr Val Thr Ser Ser Pro Gly Thr Pro Ala Ser Pro
195 200 205

Cys Ser Leu Ser Gly Ile Ile Ile Gly Val Thr Val Ala Ala Val Val
210 215 220

Leu Ile Val Ala Val Phe Val Cys Lys Ser Leu Leu Trp Lys Lys Val
225 230 235 240

Leu Pro Tyr Leu Lys Gly Ile Cys Ser Gly Gly Gly Gly Asp Pro Glu
245 250 255

Arg Val Asp Arg Ser Ser Gln Arg Pro Gly Ala Glu Asp Asn Val Leu
260 265 270

Asn Glu Ile Val Ser Ile Leu Gln Pro Thr Gln Val Pro Glu Gln Glu
275 280 285

*FIG. 3B*

```
Met Glu Val Gln Glu Pro Ala Glu Pro Thr Gly Val Asn Met Leu Ser
    290                 295                 300

Pro Gly Glu Ser Glu His Leu Leu Glu Pro Ala Glu Ala Glu Arg Ser
305                 310                 315                 320

Gln Arg Arg Arg Leu Leu Val Pro Ala Asn Glu Gly Asp Pro Thr Glu
                325                 330                 335

Thr Leu Arg Gln Cys Phe Asp Asp Phe Ala Asp Leu Val Pro Phe Asp
            340                 345                 350

Ser Trp Glu Pro Leu Met Arg Lys Leu Gly Leu Met Asp Asn Glu Ile
        355                 360                 365

Lys Val Ala Lys Ala Glu Ala Ala Gly His Arg Asp Thr Leu Tyr Thr
    370                 375                 380

Met Leu Ile Lys Trp Val Asn Lys Thr Gly Arg Asp Ala Ser Val His
385                 390                 395                 400

Thr Leu Leu Asp Ala Leu Glu Thr Leu Gly Glu Arg Leu Ala Lys Gln
                405                 410                 415

Lys Ile Glu Asp His Leu Leu Ser Ser Gly Lys Phe Met Tyr Leu Glu
            420                 425                 430

Gly Asn Ala Asp Ser Ala Met Ser  *
        435                 440
```

*FIG. 3C*

```
  1 GCTGTGGGAA CCTCTCCACG CGCACGAACT CAGCCAACGA TTTCTGATAG ATTTTTGGGA GTTTGACCAG AGATGCAAGG GGTGAAGGAG CGCTTCCTAC
    CGACACCCTT GGAGAGGTGC GCGTGCTTGA GTCGGTTGCT AAAGACTATC TAAAAACCCT CAAACTGGTC TCTACGTTCC CCACTTCCTC GCGAAGGATG

101 CGTTAGGGAA CTCTGGGGAC AGAGCGCCCC GGCCGCCTGA TGGCCGAGGC AGGGTGCGAC CCAGGACCCA GGACGGCGTC GGGAACCATA CCATGGCCCG
    GCAATCCCTT GAGACCCCTG TCTCGCGGGG CCGGCGGACT ACCGGCTCCG TCCCACGCTG GGTCCTGGGT CCTGCCGCAG CCCTTGGTAT GGTACCGGGC
  1                                                                                                    MetAlaArg

201 GATCCCCAAG ACCCTAAAGT TCGTCGTCGT CATCGTCGCG GTCCTGCTGC CAGTCCTAGC TTACTCTGCC ACCACTGCCC GGCAGGAGGA AGTTCCCCAG
    CTAGGGGTTC TGGGATTTCA AGCAGCAGCA GTAGCAGCGC CAGGACGACG GTCAGGATCG AATGAGACGG TGGTGACGGG CCGTCCTCCT TCAAGGGGTC
  4  IleProLys ThrLeuLysP heValValVa lIleValAla ValLeuLeuP roValLeuAl aTyrSerAla ThrThrAlaA rgGlnGluGl uValProGln

301 CAGACAGTGG CCCCACAGCA ACAGAGGCAC AGCTTCAAGG GGGAGGAGTG TCCAGCAGGA TCTCATAGAT CAGAACATAC TGGAGCCTGT AACCCGTGCA
    GTCTGTCACC GGGGTGTCGT TGTCTCCGTG TCGAAGTTCC CCCTCCTCAC AGGTCGTCCT AGAGTATCTA GTCTTGTATG ACCTCGGACA TTGGGCACGT
 37 GlnThrValA laProGlnGl nGlnArgHis SerPheLysG lyGluGluCy sProAlaGly SerHisArgS erGluHisTh rGlyAlaCys AsnProCysThr

401 CAGAGGGTGT GGATTACACC AACGCTTCCA ACAATGAACC TTCTTGCTTC CCATGTACAG TTTGTAAATC AGATCAAAAA CATAAAAGTT CCTGCACCAT
    GTCTCCCACA CCTAATGTGG TTGCGAAGGT TGTTACTTGG AAGAACGAAG GGTACATGTC AAACATTTAG TCTAGTTTTT GTATTTTCAA GGACGTGGTA
 71   GluGlyVa lAspTyrThr AsnAlaSerA snAsnGluPr oSerCysPhe ProCysThrV alCysLysSe rAspGlnLys HisLysSerS erCysThrMet

501 GACCAGAGAC ACAGTGTGTC AGTGTAAAGA AGGCACCTTC CGGAATGAAA ACTCCCCAGA GATGTGCCGG AAGTGTAGCA GGTGCCCTAG TGGGGAAGTC
    CTGGTCTCTG TGTCACACAG TCACATTTCT TCCGTGGAAG GCCTTACTTT TGAGGGGTCT CTACACGGCC TTCACATCGT CCACGGGATC ACCCCTTCAG
104  ThrArgAsp ThrValCysG lnCysLysGl uGlyThrPhe ArgAsnGluA snSerProGl uMetCysArg LysCysSerA rgCysProSe rGlyGluVal

601 CAAGTCAGTA ATTGTACGTC CTGGGATGAT ATCCAGTGTG TTGAAGAATT TGGTGCCAAT GCCACTGTGG AAACCCCAGC TGCTGAAGAG ACAATGAACA
    GTTCAGTCAT TAACATGCAG GACCCTACTA TAGGTCACAC AACTTCTTAA ACCACGGTTA CGGTGACACC TTTGGGGTCG ACGACTTCTC TGTTACTTGT
137 GlnValSerA snCysThrSe rTrpAspAsp IleGlnCysV alGluGluPh eGlyAlaAsn AlaThrValG luThrProAl aAlaGluGlu ThrMetAsnThr

701 CCAGCCCGGG GACTCCTGCC CCAGCTGCTG AAGAGACAAT GAACACCAGC CCAGGGACTC CTGCCCCAGC TGCTGAAGAG ACAATGACCA CCAGCCCGGG
    GGTCGGGCCC CTGAGGACGG GGTCGACGAC TTCTCTGTTA CTTGTGGTCG GGTCCCTGAG GACGGGGTCG ACGACTTCTC TGTTACTGGT GGTCGGGCCC
171   SerProGl yThrProAla ProAlaAlaG luGluThrMe tAsnThrSer ProGlyThrP roAlaProAl aAlaGluGlu ThrMetThrT hrSerProGly
```

*FIG. 3D-1*

```
 801 GACTCCTGCC CCAGCTGCTG AAGAGACAAT GACCACCAGC CCGGGGACTC CTGCCCCAGC TGCTGAAGAG ACAATGACCA CCAGCCCGGG GACTCCTGCC
     CTGAGGACGG GGTCGACGAC TTCTCTGTTA CTGGTGGTCG GGCCCCTGAG GACGGGGTCG ACGACTTCTC TGTTACTGGT GGTCGGGCCC CTGAGGACGG
 204  ThrProAla ProAlaAlaG luGluThrMe tThrThrSer ProGlyThrP roAlaProAl aAlaGluGlu ThrMetThrT hrSerProGl yThrProAla

 901 TCTTCTCATT ACCTCTCATG CACCATCGTA GGGATCATAG TTCTAATTGT GCTTCTGATT GTGTTTGTTT GAAAGACTTC ACTGTGGAAG AAATTCCTTC
     AGAAGAGTAA TGGAGAGTAC GTGGTAGCAT CCCTAGTATC AAGATTAACA CGAAGACTAA CACAAACAAA CTTTCTGAAG TGACACCTTC TTTAAGGAAG
 237 SerSerHisT yrLeuSerCy sThrIleVal GlyIleIleV alLeuIleVa lLeuLeuIle ValPheVal

1001 CTTACCTGAA AGGTTCAGGT AGGCGCTGGC TGAGGGCGGG GGGCGCTGGA CACTCTCTGC CCTGCCTCCC TCTGCTGTGT TCCCACAGAC AGAAACGCCT
     GAATGGACTT TCCAAGTCCA TCCGCGACCG ACTCCCGCCC CCCGCGACCT GTGAGAGACG GGACGGAGGG AGACGACACA AGGGTGTCTG TCTTTGCGGA

1101 GCCCCTGCCC CAAAAAAAAA AAAAAAAAAA AAAAAAAAAA AAAAAAAAAA AAAAAAAAAA AAAAAAAAAA AAAAAAAAAA
     CGGGGACGGG GTTTTTTTTT TTTTTTTTTT TTTTTTTTTT TTTTTTTTTT TTTTTTTTTT TTTTTTTTTT TTTTTTTTTT


   1 GCTGTGGGAA CCTCTCCACG CGCACGAACT CAGCCAACGA TTTCTGATAG ATTTTTGGGA GTTTGACCAG AGATGCAAGG GGTGAAGGAG CGCTTCCTAC
     CGACACCCTT GGAGAGGTGC GCGTGCTTGA GTCGGTTGCT AAAGACTATC TAAAAACCCT CAAACTGGTC TCTACGTTCC CCACTTCCTC GCGAAGGATG
 -40                                                                          MetGlnGl yValLysGlu ArgPheLeuPro

 101 CGTTAGGGAA CTCTGGGGAC AGAGCGCCCC GGCCGCCTGA TGGCCGAGGC AGGGTGCGAC CCAGGACCCA GGACGGCGTC GGGAACCATA CCATGGCCCG
     GCAATCCCTT GAGACCCCTG TCTCGCGGGG CCGGCGGACT ACCGGCTCCG TCCCACGCTG GGTCCTGGGT CCTGCCGCAG CCCTTGGTAT GGTACCGGGC
 -30   LeuGlyAs nSerGlyAsp ArgAlaProA rgProProAs pGlyArgGly ArgValArgP roArgThrGl nAspGlyVal GlyAsnHisT hrMetAlaArg

 201 GATCCCCAAG ACCCTAAAGT TCGTCGTCGT CATCGTCGCG GTCCTGCTGC CAGTCCTAGC TTACTCTGCC ACCACTGCCC GGCAGGAGGA AGTTCCCCAG
     CTAGGGGTTC TGGGATTTCA AGCAGCAGCA GTAGCAGCGC CAGGACGACG GTCAGGATCG AATGAGACGG TGGTGACGGG CCGTCCTCCT TCAAGGGGTC
   4  IleProLys ThrLeuLysP heValValVa lIleValAla ValLeuLeuP roValLeuAl aTyrSerAla ThrThrAlaA rgGlnGluGl uValProGln

 301 CAGACAGTGG CCCCACAGCA ACAGAGGCAC AGCTTCAAGG GGGAGGAGTG TCCAGCAGGA TCTCATAGAT CAGAACATAC TGGAGCCTGT AACCCGTGCA
     GTCTGTCACC GGGGTGTCGT TGTCTCCGTG TCGAAGTTCC CCCTCCTCAC AGGTCGTCCT AGAGTATCTA GTCTTGTATG ACCTCGGACA TTGGGCACGT
  37 GlnThrValA laProGlnGl nGlnArgHis SerPheLysG lyGluGluCy sProAlaGly SerHisArgS erGluHisTh rGlyAlaCys AsnProCysThr
```

FIG. 3D-2

```
 401 CAGAGGGTGT GGATTACACC AACGCTTCCA ACAATGAACC TTCTTGCTTC CCATGTACAG TTTGTAAATC AGATCAAAAA CATAAAAGTT CCTGCACCAT
     GTCTCCCACA CCTAATGTGG TTGCGAAGGT TGTTACTTGG AAGAACGAAG GGTACATGTC AAACATTTAG TCTAGTTTTT GTATTTTCAA GGACGTGGTA
  71   GluGlyVa lAspTyrThr AsnAlaSerA snAsnGluPr oSerCysPhe ProCysThrV alCysLysSe rAspGlnLys HisLysSerS erCysThrMet

 501 GACCAGAGAC ACAGTGTGTC AGTGTAAAGA AGGCACCTTC CGGAATGAAA ACTCCCCAGA GATGTGCCGG AAGTGTAGCA GGTGCCCTAG TGGGGAAGTC
     CTGGTCTCTG TGTCACACAG TCACATTTCT TCCGTGGAAG GCCTTACTTT TGAGGGGTCT CTACACGGCC TTCACATCGT CCACGGGATC ACCCCTTCAG
 104  ThrArgAsp ThrValCysG lnCysLysGl uGlyThrPhe ArgAsnGluA snSerProGl uMetCysArg LysCysSerA rgCysProSe rGlyGluVal

 601 CAAGTCAGTA ATTGTACGTC CTGGGATGAT ATCCAGTGTG TTGAAGAATT TGGTGCCAAT GCCACTGTGG AAACCCCAGC TGCTGAAGAG ACAATGAACA
     GTTCAGTCAT TAACATGCAG GACCCTACTA TAGGTCACAC AACTTCTTAA ACCACGGTTA CGGTGACACC TTTGGGGTCG ACGACTTCTC TGTTACTTGT
 137 GlnValSerA snCysThrSe rTrpAspAsp IleGlnCysV alGluGluPh eGlyAlaAsn AlaThrValG luThrProAl aAlaGluGlu ThrMetAsnThr

 701 CCAGCCCGGG GACTCCTGCC CCAGCTGCTG AAGAGACAAT GAACACCAGC CCAGGGACTC CTGCCCCAGC TGCTGAAGAG ACAATGACCA CCAGCCCGGG
     GGTCGGGCCC CTGAGGACGG GGTCGACGAC TTCTCTGTTA CTTGTGGTCG GGTCCCTGAG GACGGGGTCG ACGACTTCTC TGTTACTGGT GGTCGGGCCC
 171   SerProGl yThrProAla ProAlaAlaG luGluThrMe tAsnThrSer ProGlyThrP roAlaProAl aAlaGluGlu ThrMetThrT hrSerProGly

 801 GACTCCTGCC CCAGCTGCTG AAGAGACAAT GACCACCAGC CCGGGGACTC CTGCCCCAGC TGCTGAAGAG ACAATGACCA CCAGCCCGGG GACTCCTGCC
     CTGAGGACGG GGTCGACGAC TTCTCTGTTA CTGGTGGTCG GGCCCCTGAG GACGGGGTCG ACGACTTCTC TGTTACTGGT GGTCGGGCCC CTGAGGACGG
 204  ThrProAla ProAlaAlaG luGluThrMe tThrThrSer ProGlyThrP roAlaProAl aAlaGluGlu ThrMetThrT hrSerProGl yThrProAla

 901 TCTTCTCATT ACCTCTCATG CACCATCGTA GGGATCATAG TTCTAATTGT GCTTCTGATT GTGTTTGTTT GAAAGACTTC ACTGTGGAAG AAATTCCTTC
     AGAAGAGTAA TGGAGAGTAC GTGGTAGCAT CCCTAGTATC AAGATTAACA CGAAGACTAA CACAAACAAA CTTTCTGAAG TGACACCTTC TTTAAGGAAG
 237 SerSerHisT yrLeuSerCy sThrIleVal GlyIleIleV alLeuIleVa lLeuLeuIle ValPheVal

1001 CTTACCTGAA AGGTTCAGGT AGGCGCTGGC TGAGGGCGGG GGCGCTGGA CACTCTCTGC CCTGCCTCCC TCTGCTGTGT TCCCACAGAC AGAAACGCCT
     GAATGGACTT TCCAAGTCCA TCCGCGACCG ACTCCCGCCC CCCGCGACCT GTGAGAGACG GGACGGAGGG AGACGACACA AGGGTGTCTG TCTTTGCGGA

1101 GCCCCTGCCC CAAAAAAAAA AAAAAAAAAA AAAAAAAAAA AAAAAAAAAA AAAAAAAAAA AAAAAAAAAA AAAAAAAAAA
     CGGGGACGGG GTTTTTTTTT TTTTTTTTTT TTTTTTTTTT TTTTTTTTTT TTTTTTTTTT TTTTTTTTTT TTTTTTTTTT
```

FIG. 3D-3

```
  1 CCAACTGCAC CTCGGTTCTA TCGATTGAAT TCCCCGGGGA TCCTCTAGAG ATCCCTCGAC

 61 CTCGACCCAC GCGTCCGGAA CCTTTGCACG CGCACAAACT ACGGGGACGA TTTCTGATTG

121 ATTTTTGGCG CTTTCGATCC ACCCTCCTCC CTTCTCATGG GACTTTGGGG ACAAAGCGTC
  1                                       M G   L  W  G   Q  S  V

181 CCGACCGCCT CGAGCGCTCG AGCAGGGCGC TATCCAGGAG CCAGGACAGC GTCGGGAACC
  9 P  T  A  S   S  A  R   A  G  R   Y  P  G  A   R  T  A   S  G  T

241 AGACCATGGC TCCTGGACCC CAAGATCCTT AAGTTCGTCG TCTTCATCGT CGCGGTTCTG
 29 R  P  W  L   L  D  P   K  I  L   K  F  V  V   F  I  V   A  V  L

301 CTGCCGGTCC GGGTTGACTC TGCCACCATC CCCCGGCAGG ACGAAGTTCC CCAGCAGACA
 49 L  P  V  R   V  D  S   A  T  I   P  R  Q  D   E  V  P   Q  Q  T

361 GTGGCCCCAC AGCAACAGAG GCGCAGCCTC AAGGAGGAGG AGTGTCCAGC AGGATCTCAT
 69 V  A  P  Q   Q  Q  R   R  S  L   K  E  E  E   C  P  A   G  S  H

421 AGATCAGAAT ATACTGGAGC CTGTAACCCG TGCACAGAGG GTGTGGATTA CACCATTGCT
 89 R  S  E  Y   T  G  A   C  N  P   C  T  E  G   V  D  Y   T  I  A

481 TCCAACAATT TGCCTTCTTG CCTGCTATGT ACAGTTTGTA AATCAGGTCA AACAAATAAA
109 S  N  N  L   P  S  C   L  L  C   T  V  C  K   S  G  Q   T  N  K

541 AGTTCCTGTA CCACGACCAG AGACACCGTG TGTCAGTGTG AAAAAGGAAG CTTCCAGGAT
129 S  S  C  T   T  T  R   D  T  V   C  Q  C  E   K  G  S   F  Q  D

601 AAAAACTCCC CTGAGATGTG CCGGACGTGT AGAACAGGGT GTCCCAGAGG GATGGTCAAG
149 K  N  S  P   E  M  C   R  T  C   R  T  G  C   P  R  G   M  V  K

661 GTCAGTAATT GTACGCCCCG GAGTGACATC AAGTGCAAAA ATGAATCAGC TGCCAGTTCC
169 V  S  N  C   T  P  R   S  D  I   K  C  K  N   E  S  A   A  S  S

721 ACTGGGAAAA CCCCAGCAGC GGAGGAGACA GTGACCACCA TCCTGGGGAT GCTTGCCTCT
189 T  G  K  T   P  A  A   E  E  T   V  T  T  I   L  G  M   L  A  S

781 CCCTATCACT ACCTTATCAT CATAGTGGTT TTAGTCATCA TTTTAGCTGT GGTTGTGGTT
209 P  Y  H  Y   L  I  I   I  V  V   L  V  I  I   L  A  V   V  V  V

841 GGCTTTTCAT GTCGGAAGAA ATTCATTTCT TACCTCAAAG GCATCTGCTC AGGTGGTGGA
229 G  F  S  C   R  K  K   F  I  S   Y  L  K  G   I  C  S   G  G  G
```

FIG. 3E-1

```
 901 GGAGGTCCCG AACGTGTGCA CAGAGTCCTT TTCCGGCGGC GTTCATGTCC TTCACGAGTT
 249 G  G  P  E   R  V  H   R  V  L   F  R  R  R   S  C  P   S  R  V

 961 CCTGGGGCGG AGGACAATGC CCGCAACGAG ACCCTGAGTA ACAGATACTT GCAGCCCACC
 269 P  G  A  E   D  N  A   R  N  E   T  L  S  N   R  Y  L   Q  P  T

1021 CAGGTCTCTG AGCAGGAAAT CCAAGGTCAG GAGCTGGCAG AGCTAACAGG TGTGACTGTA
 289 Q  V  S  E   Q  E  I   Q  G  Q   E  L  A  E   L  T  G   V  T  V

1081 GAGTYGCCAG AGGAGCCACA GCGTCTGCTG GAACAGGCAG AAGCTGAAGG GTGTCAGAGG
 309 E Xaa P  E   E  P  Q   R  L  L   E  Q  A  E   A  E  G   C  Q  R

1141 AGGAGGCTGC TGGTTCCAGT GAATGACGCT GACTCCGCTG ACATCAGCAC CTTGCTGGAT
 329 R  R  L  L   V  P  V   N  D  A   D  S  A  D   I  S  T   L  L  D

1201 GCCTCGGCAA CACTGGAAGA AGGACATGCA AAGGAAACAA TTCAGGACCA ACTGGTGGGC
 349 A  S  A  T   L  E  E   G  H  A   K  E  T  I   Q  D  Q   L  V  G

1261 TCCGAAAAGC TCTTTTATGA AGAAGATGAG GCAGGCTCTG CTACGTCCTG CCTGTGAAAG
 369 S  E  K  L   F  Y  E   E  D  E   A  G  S  A   T  S  C   L

1321 AATCTCTTCA GGAAACCAGA GCTTCCCTCA TTTACCTTTT CTCCTACAAA GGGAAGCAGC
1381 CTGGAAGAAA CAGTCCAGTA CTTGACCCAT GCCCCAACAA ACTCTACTAT CCAATATGGG
1441 GCAGCTTACC AATGGTCCTA GAACTTTGTT AACGCACTTG GAGTAATTTT TATGAAATAC
1501 TGCGTGTGAT AAGCAAACGG GAGAAATTTA TATCAGATTC TTGGCTGCAT AGTTATACGA
1561 TTGTGTATTA AGGGTCGTTT TAGGCCACAT GCGGTGGCTC ATGCCTGTAA TCCCAGCACT
1621 TTGATAGGCT GAGGCAGGTG GATTGCTTGA GCTCGGGAGT TTGAGACCAG CCTCATCAAC
1681 ACAGTGAAAC TCCATCTCAA TTTAAAAAGA AAAAAAGTGG TTTTAGGATG TCATTCTTTG
1741 CAGTTCTTCA TCATGAGACA AGTCTTTTTT TCTGCTTCTT ATATTGCAAG CTCCATCTCT
1801 ACTGGTGTGT GCATTTAATG ACATCTAACT ACAGATGCCG CACAGCCACA ATGCTTTGCC
1861 TTATAGTTTT TTAACTTTAG AACGGGATTA TCTTGTTATT ACCTGTATTT TCAGTTTCGG
1921 ATATTTTTGA CTTAATGATG AGATTATCAA GACGTACCCC TATGCTAAGT CATGAGCATA
1981 TGGACTTACG AGGGTTCGAC TTAGAGTTTT GAGCTTTAAG ATAGGATTAT TGGGGGCTTA
2041 CCCCCACCTT AATTAGAAGA AACATTTTAT ATTGCTTTAC TA
```

FIG. 3E-2

FIG. 4A-1 | FIG. 4A-2
FIG. 4A-3 | FIG. 4A-4

# FIG. 4A

# FIG. 4A-1

```
-640
-600 CCCAACCCACGATGGTCTGGGAGCTGCGCCCAGGGCTTGGCGCTGGCGGCCCCGCAACAGCACCGAGCGTTTCGGTCGGC
-450 GTCGGGCCCGCCCTCCCCGCTCACTCCCTCCGCCCTCGTGCTCCTCCCGGGGTGCTTGGCACAGCCTCGGATTCCTCCCT
-300 GTGGCGGCGTGGGGAACATCTCGGCAGCCACCGCGCTTCTCCCGCTGGAGCGGGCGTCCAGCTTGGCTGCCCTCGGTCCT
-150 CTTGCAGGATCCTGCCGCCCCTCCAACCGGATCCTGGGTCTAGAGCTCCCCAGAGCGAGGCGCTCGCCAGGACTCCTGCC

  1 ATGCGGCGCCTGACTCGTCGGCTGGTTCTGCCAGTCTTCGGGGTGCTCTGGATCACGGTGCTGCTGTTCTTCTGGGTAAC
     M  R  R  L  T  R  R [L  V  L  P  V  F  G  V  L  W  I  T  V  L  L  F  F  W  V] T

151 GACCTGTGGGACCAGTTTGATGAGCGGCGGTATCTGAATGCCAAAAAGTGGCGCGTTGGTGACGACCCCTATAAGCTGTA
     D  L  W  D  Q  F  D  E  R  R  Y  L  N  A  K  K  W  R  V  G  D  D  P  Y  K  L  Y

301 TGCACACTGCTGGTGTATTGCACGGACCTTCCACCCACTAGCATCATCATCACCTTCCACAACGAGGCCCGCTCCACGCT
     C  T  L  L  V  Y  C  T  D  L  P  P  T  S  I  I  I  T  F  H  N  E  A  R  S  T  L
                Exon6<--|-->Exon7                                               Exon7<--
451 GACTTCAGCAATGATCCTGATGACTGTAAACAGCTCATCAAGTTGCCCAAGGTGAAATGCTTGCGCAATAATGAACGGCA
     D  F  S  N  D  P  D  D  C  K  Q  L  I  K  L  P  K  V  K  C  L  R  N  N  E  R  Q
                                                      Exon8<--|-->Exon9
601 CACTGTGAGGTGAACAGGGACTGGCTCCAGCCTCTGTTGCACAGGGTCAAAGAGGACTACACGCGGGTGGTGTGCCCTGT
     H  C  E  V  N  R  D  W  L  Q  P  L  L  H  R  V  K  E  D  Y  T  R  V  V  C  P  V
                                                                        Exon10<--|--
751 TGGAGCCTCCACTTCCAGTGGGAGCAGCTCTCCCCAGAGCAGAAGGCTCGGCGCCTGGACCCCACGGAGCCCATCAGGAC
     W  S  L  H  F  Q  W  E  Q  L  S  P  E  Q  K  A  R  R  L  D  P  T  E  P  I  R  T
```

# FIG. 4A-2

```
                                        GGCATCCCGAGTGCACCGCTCCCGCCCCGCCCCGCCTTGC
GGGCGGCGGTAGCGCCCCCTCTCAGAGCCCCGCTCACTCCCACCTCGGCTCGCTCCGAGTCGGCCTGTCT
CTCGCTGCTCGAGTCAGTTTCCCTATCGGCGGCAGCGGGCAAGGCGGCGGCGGCGGCGGCAGCCGCG
TCCCTGCCACGTTTCGGGTCGCCCTGCACCCCCCACCCAGGCTCGCTTCTCTTCGAAGCGGGAAGGGCGC
CCGCCAACCCTGACCGCCGGGGGGTGCCCCCGGGACGTAGCGCCGCGGAGAGGAAGCGGCAAAGGGGACC
                                          Exon1<-------------->Exon4
CAAGAGGAAGTTGGAGGTGCCGACGGGACCTGAAGTGCAGACCCCTAAG|CCTTCGGACGCTGACTGGGAC
 K  R  K  L  E  V  P  T  G  P  E  V  Q  T  P  K  P  S  D  A  D  W  D
                                                      Exon4<------------>Exon5
TGCTTTCAACCAGCGGGAGAGTGAGCGGATCTCCAGCAATCGGGCCATCCCGGACACTCGCCATCTGAG|A
 A  F  N  Q  R  E  S  E  R  I  S  S  N  R  A  I  P  D  T  R  H  L  R
      Exon5<------------>Exon6
GCTCAGGACCATCCGCAG|TGTATTAAACCGCACCCCTACGCATCTGATCCGGGAAATCATATTAGTGGAT
 L  R  T  I  R  S  V  L  N  R  T  P  T  H  L  I  R  E  I  I  L  V  D
----->Exon8
AG|GTCTGGTCCGGTCCCGGATTCGGGGCGCTGACATCGCCCAGGGCACCACTCTGACTTTCCTCGACAGC
 G  L  V  R  S  R  I  R  G  A  D  I  A  Q  G  T  T  L  T  F  L  D  S
                                                   Exon9<------------>Exon10
GATCGATATCATTAACCTGGACACCTTCACCTACATCGAGTCTGCCTCGGAGCTCAGAGGGG|GGTTTGAC
 I  D  I  I  N  L  D  T  F  T  Y  I  E  S  A  S  E  L  R  G  G  F  D
--->Exon11
TCCTATCATAGCTGGAGGGCTCTTCGTGATCGACAAAGCTTGGTTTGATTACCTGGGGAAATATGATATG
 P  I  I  A  G  G  L  F  V  I  D  K  A  W  F  D  Y  L  G  K  Y  D  M
```

```
                    Exon11<--------|-------->Exon12
901  GACATGGACATCTGGGGTGGGGAGAACTTTG|AAATCTCCTTCCGAGTGTGGATGTGCGGGGGCAGCCTAGAGATCGTCCC
      D  M  D  I  W  G  G  E  N  F  E  I  S  F  R  V  W  M  C  G  G  S  L  E  I  V  P
Exon12<--------|-------->Exon13
1051 TATATAAA|GAACACCAAGCGGACAGCTGAAGTGTGGATGGATGAATACAAGCAATACTATTACGCTGCCCGGCCATTCGC
      Y  I  K  N  T  K  R  T  A  E  V  W  M  D  E  Y  K  Q  Y  Y  Y  A  A  R  P  F  A
                         Exon14<--------|-------->Exon15
1201 AAGTGGTACCTGGAGAATATCTACCCTGAACTCAG|CATCCCCAAGGAGTCCTCCATCCAGAAGGGCAATATCCGACAGAG
      K  W  Y  L  E  N  I  Y  P  E  L  S  I  P  K  E  S  S  I  Q  K  G  N  I  R  Q  R
                    Exon15<--------|-------->Exon16
1351 AAGGTCAAAGGCGAAGATGCAAAGTCCCAG|GTATGGGCCTTCACATACACCCAGCAGATCCTCCAGGAGGAGCTGTGCCT
      K  V  K  G  E  D  A  K  S  Q  V  W  A  F  T  Y  T  Q  Q  I  L  Q  E  E  L  C  L
    |-------->Exon17
1501 |CAATGGACCAAAACTGGTTCCCACATCGAGCACATAGCATCCCACCTCTGCCTCGATACAGATATGTTCGGTGATGGCAC
      Q  W  T  K  T  G  S  H  I  E  H  I  A  S  H  L  C  L  D  T  D  M  F  G  D  G  T

1651 AGCTCTTGAGGACCCCTGCCAGAAGCAGCAAGGGCCATGGGGTGGTGCTTCCCTGGACCAGAACAGACTGGAAACTGGGC
      S  S  *
1801 CAACTGTCTCAGGGAGGACAGAGGAAAACATCACAAGCCAATGGGGCTCAAAGACAAATCCCACATGTTCTCAAGGCCGT
1951 TGTTCCTTTTCCTACAAAGGAAGCAGTCTCTGGAGGCCAGAAACAAAAGCCTTCTTTTTCACTAGGCCAGGACTACATTG
2101 A............
```

*FIG. 4A-3*

```
CTGCAGCCGAGTGGGGCACGTCTTCCGGAAGAAGCACCCCTACGTTTTCCCTGATGGAAATGCCAACACG
 C  S  R  V  G  H  V  F  R  K  K  H  P  Y  V  F  P  D  G  N  A  N  T
          Exon13<----|---->Exon14
CCTGGAGAGGCCCTTCGGGAATGTTGAGAGCAGATTGGACCTGAGGAAGAATCTGCGCTGCCAGAGCTTC
 L  E  R  P  F  G  N  V  E  S  R  L  D  L  R  K  N  L  R  C  Q  S  F

ACAGAAGTGCCTGGAATCTCAAAGGCAGAACAACCAAGAAACCCCAAACCTAAAGTTGAGCCCCTGTGCC
 Q  K  C  L  E  S  Q  R  Q  N  N  Q  E  T  P  N  L  K  L  S  P  C  A
                                                          Exon16<----|
GTCAGTCATCACCTTGTTCCCTGGCGCCCCAGTGGTTCTTGTCCTTTGCAAGAATGGAGATGACCGACAG
 S  V  I  T  L  F  P  G  A  P  V  V  L  V  L  C  K  N  G  D  D  R  Q

CGAGAACGGCAAGGAAATCGTCGTCAACCCATGTGAGTCCTCACTCATGAGCCAGCACTGGGACATGGTG
 E  N  G  K  E  I  V  V  N  P  C  E  S  S  L  M  S  Q  H  W  D  M  V

AGCAAGCAGCCTGCAACCACCTCAGACATCCTGGACTGGGAGGTCCAGGCAGAGCCCCCAGGACAGGAG

TAAGTTCCAGTCCTGGCCAGTCATTCCCTGATTGGTATCTGGAGACAGAAACCTAATGGGAAGTGTTTAT
AGAGATGAAGAATGGAGGTTGTTTCCAAAAGAAATAAAGAGAAACTTAGAAGTTGAAAAAAAAAAAAAAA
```

Nucleotide sequence of the pp-GalNAc-T14 cDNA and the predicted amino acid sequence of pp-GalNAc-T14. Exon-intron junctions are indicated. The putative N-terminal transmembrane domain was *boxed*. A putative N-glycosylation site was indicated by *double underline*. The starting position of the truncated form was indicated by *triangle*.

## *FIG. 4A-4*

| FIG. 4B-1 | FIG. 4B-2 |
|---|---|
| FIG. 4B-3 | FIG. 4B-4 |

# FIG. 4B

# FIG. 4B-1

GalNAc-T3

-49 ..............................................................................

1 M A H L K R L V K L H I K R H Y H K K **F W K L G A V I**
1 ATGGCTCACCTAAAGCGACTAGTAAAATTACACATTAAAAGACATTACCATAAAAAGTTCTGGAAGCTTGGTGCAGTAAT

51 R M E R N M K N K N K M L D L M L E A V N N I K D A M
151 AGGATGGAAAGGAACATGAAAAACAAAAACAAGATGTTGGATTTAATGCTAGAAGCTGTAAACAATATTAAGGATGCCAT

101 Y Y T A A E L K P V L D R P P Q D S N A P G A S G K A
301 TATTATACAGCAGCAGAATTGAAGCCTGTCCTTGACCGTCCACCTCAGGATTCAAATGCACCTGGTGCTTCTGGTAAAGC

151 N A F A S D R I S L H R D L G P D T R P P E C I E Q K
451 AATGCTTTCGCAAGTGACAGGATTTCTTTGCACCGAGATCTTGGACCAGACACTCGACCTCCTGAATGTATTGAACAAAA

201 L L R T V H S V L Y S S P A I L L K E I I L V D D A S
601 TTGCTTAGAACTGTCCACAGTGTGCTCTATTCTTCACCTGCAATACTGCTGAAGGAAATCATTTTGGTGGATGATGCTAG

251 Q R E R K G L I T A R L L G A T V A T A E T L T F L D
751 CAAAGAGAAAGAAAAGGTCTGATCACTGCTCGGTTGCTAGGAGCAACAGTCGCAACAGCTGAAACGCTCACATTTTTAGA

301 V V S P D I A S I D L N T F E F N K P S P Y G S N H N
901 GTCGTAAGTCCAGATATTGCATCCATAGATCTGAACACGTTTGAATTCAACAAACCTTCTCCTTATGGAAGTAACCATAA

351 K D E T Y P I K T P T F A G G L F S I S K E Y F E Y I
1051 AAAGATGAAACCTACCCAATTAAAACACCCACTTTTGCAGGAGGACTTTTTTCCATATCAAAAGAATATTTTGAGTATAT

401 **C G G Q L E I M P C S V V G N V F R S K S P N S F P K**
1201 TGTGGTGGGCAGTTGGAGATTATGCCTTGCTCTGTTGTTGGACATGTTTTCGCAGCAAAAGCCCTCATAGCTTTCCAAA

451 F Y R R N T D A A K I V K Q K A F G D L S K R F E I K
1351 TTTTATAGGAGAAATACAGATGCAGCAAAAATTGTTAAACAAAAAGCATTTGGTGATCTTTCAAAAAGATTTGAAATAAA

# *FIG. 4B-2*

......................GAAGAAGAAATAACTGTTATTTGTCAAGTGACAAGCTTTTAATGTCAGA

**F F F I I V L V L M** Q R E V S V Q Y S K E E S

TTTTTTCTTTATAATAGTTTTGGTTTTAATGCAAAGAGAAGTAAGTGTTCAATATTCCAAAGAGGAATCA

P K M Q I G A P V R Q N I D A G E R P C L Q G

GCCAAAAATGCAAATAGGAGCACCTGTCAGGCAAAACATTGATGCTGGTGAGAGACCTTGTTTGCAAGGA

F K T T N* L S V E E Q K E K E R G E A K H C F

ATTCAAGACAACCAATTTAAGTGTTGAAGAGCAAAAGGAAAAGGAACGTGGGGAAGCTAAACACTGCTTT

F K R C P P L P T T S V I I V F H N E A W S T

ATTTAAGCGCTGCCCTCCCCTGCCCACCACCAGTGTCATAATAGTTTTCATAATGAAGCGTGGTCCACG

V D E Y L H D K L D E Y V K Q F S I V K I V R

TGTAGATGAGTACTTACATGATAAACTAGATGAATATGTAAAACAATTTTCTATAGTAAAAATAGTCAGA

A H C E C F Y G W L E P L L A R I A E N* Y T A

TGCTCACTGTGAGTGTTTCTATGGTTGGCTAGAACCTCTGTTGGCCAGAATAGCTGAGAACTACACGGCT

R G N F D W S L S F G W E S L P D H E K Q R R

CCGTGGAAATTTTGACTGGAGTCTTTCATTTGGCTGGGAGTCGCTTCCTGATCATGAGAAGCAAAGAAGG

G S Y D E E M E I **W G G E N I E M S P R V W Q**

TGGAAGCTATGATGAAGAAATGGAAATC**TGGGGAGGTGAAAATATAGA**AATGTCTTTCAGAGTATGGCAA

**G T Q V I A R N Q V R L A E V W M D E** Y K E I

AGGCACTCAGGTGATTGCTAGAAACCAAGTTCGCCTTGC**AGAAGTCTGGATGGATGAAT**ACAAGGAAATA

H R L R C K N* F T W Y L N N I Y P E V Y V P D

ACACCGTCTTCGGTGTAAAAATTTTACATGGTATCTGAACAACATTTATCCAGAGGTGTATGTGCCAGAC

```
501  L  N  P  V  I  S  G  Y  I  K  S  V  G  Q  P  L  C  L  D  V  G  E  N  N  Q  G  G
1501 CTTAATCCTGTTATATCTGGATACATTAAAAGCGTTGGTCAGCCTCTATGTCTGGATGTTGGAGAAAACAATCAAGGAGG

551  E  I  R  H  N  I  Q  K  E  L  C  L  H  A  A  Q  G  L  V  Q  L  K  A  C  T  Y  K
1651 GAAATTCGGCACAACATCCAGAAGGAATTATGTCTTCATGCTGCTCAAGGTCTCGTTCAGCTGAAGGCATGTACCTACAA

601  F  L  K  M  C  L  S  A  N  G  E  H  P  S  L  V  S  C  N* P  S  D  P  L  Q  K  W
1801 TTCTTAAAAATGTGCCTTTCAGCAAATGGAGAGCATCCAAGTTTAGTGTCATGCAACCCATCAGATCCACTCCAAAAATG

1951 CTGTGACTAGGCATACACTGTAGTTTTTGAAAATTATGCAAAAGCAGCTAAATGTAACTTATTCCAAGTGCATTTTTCTT
2101 ATACCAAAGACTATTTCAAAATGTCCAGATGTAGGGGAAGAGATGTTTACAGTATGATGAAAATAATTTTCCAAGTAAAG
2251 TATTTCCCCTAGTTTTTGGGGGATAGGAAGAAAGATTTGTTACTGTATTTTTTTAACTACATAAAAATAGATCAATAA
2401 GGTGAACTTTTTTTTGCGTTTGGTTTACTTGTCTGTCAAATGTTTCCTTAAACATGAAACTGAATAAGGAGAAGAGTATT
2551 TAAGTCTTCCTTAAATGACTTTTCTTAAGTAATGATACTGTGTGTTTTCCCAAAGCACTTTTAAAAAAATTTTTATAAAT
2701 TGAATGTTTGTGATATTAAATTTCAAATGCAGAATACTTGACTCATTTAAAGCTAAATTTTGTTACTGATTCAATTATAA
2851 AACACAATAAAAAATCCTCAACACTAAAAAAAAAAAAAA
```

*FIG. 4B-3*

```
 K  P  L  I  M  Y  T  C  H  G  L  G  G  N  Q  Y  F  E  Y  S  A  Q  H
CAAACCATTAATTATGTATACATGTCATGGACTTGGGGAAACCAGTACTTTGAATACTCTGCTCAACAT

 G  H  K  T  V  V  T  G  E  Q  I  W  E  I  Q  K  D  Q  L  L  Y  N  P
AGGTCACAAGACAGTTGTCACTGGAGAGCAGATATGGGAGATCCAGAAGGATCAACTTCTATACAATCCA

 I  L  S  Q  N  D  *
GATACTTAGCCAAAATGATTAAGTGTTCCTTAAAATTAAGTTGAAAAAGGAAATATTCTTTCTCATAAAA

ATTTATATCTTTATGTAGCACTATCTACAGAAATTCTGCAAGTTTCTGTTTCAAAGCACAATAACTAGTA
TGAAGTTTGTGTGTTTTGTACACTTAGGGATATATATATATAGCTACATTCACACACTCACAATTTAAAA
ATGTCAGCATTGGCCTCTGTGTACAAACCAAGAGCTTTTACAGATCCAGAATTTATTAGTTTAAAATGCA
TTTAACACTTAAATTTCTTGGCAAATTTTAAAACATTTTTTAGTCTGTAATACACTCCACTTGAAGCACT
TACTATCTGTTGAAAAGGTGTCCTTTTCCTTTCTTCTAGTATTTTTTTTCTTACCAAAATTCACTAATCT
TTGTAATGGATTTTTGACTTTGTAATGGATTCTTTTCATCAAAAAGCCTTATTTTTTTATCTATGTGGAA
```

The amino acid sequence is shown in single-letter codes. The hydrophobic segment representing the putative transmembrane domain is *underlined* with a *double line* (Kyte & Doolittle, window 8) (32), and adjacent charged amino acid *underlined* with a *dotted line*. Four potential *N*-linked glycosylation sites are indicated by *asterisks*.

## *FIG. 4B-4*

| NSCLC IC50 Summary | | | Apo2L 0.5% FBS MTT | | 10% FBS | | DR5 Ab+XL 0.5% FBS MTT | | 10% FBS | |
|---|---|---|---|---|---|---|---|---|---|---|
| | | | Mean | STD | Mean | STD | Mean | STD | Mean | STD |
| 1 | H2122 | adenocarcin | 10.7 | 0.7 | 3.1 | 1.5 | 148.9 | 64.8 | 205.3 | 92.1 |
| 2 | A427 | | 12.4 | 2.8 | 9.6 | 1.4 | 5.8 | 5.6 | 46.3 | 58.1 |
| 3 | H647 | adenosquam | 14.9 | 1.1 | 31.7 | 5.3 | 326.9 | 57.8 | 741.6 | 263.8 |
| 4 | SK-MES-1 | squamous | 73.4 | 14.8 | 16.6 | 1.2 | 450.3 | 112.9 | 288.3 | 12.2 |
| 5 | H838 | adenocarcin | 63.2 | 0.7 | 90.0 | 2.5 | 1000.0 | 0.0 | 1000.0 | 0.0 |
| 6 | H358 | bronchioalvo | 126.7 | 62.3 | 224.2 | 209.8 | 1000.0 | 0.0 | 1000.0 | 0.0 |
| 7 | H2126 | | 364.4 | 21.1 | 171.6 | 89.3 | 1000.0 | 0.0 | 1000.0 | 0.0 |
| 8 | H460 | large cell | 47.8 | 16.7 | 623.7 | 532.2 | 543.9 | 185.0 | 1000.0 | 0.0 |
| 9 | H1703 | adenocarcin | 509.9 | 693.2 | 1000.0 | 0.0 | 345.5 | 420.0 | 658.3 | 483.2 |
| 10 | H2405 | adenocarcin | 567.1 | 510.1 | 1000.0 | 0.0 | 433.0 | 261.5 | 1000.0 | 0.0 |
| 11 | H650 | bronchioalvo | 287.6 | 30.8 | 1000.0 | 0.0 | 231.8 | 69.5 | 1000.0 | 0.0 |
| 12 | H1568 | adenocarcin | 69.8 | 14.0 | 1000.0 | 0.0 | 1000.0 | 0.0 | 1000.0 | 0.0 |
| 13 | H1666 | bronchioalvo | 1000.0 | 0.0 | 249.8 | 84.5 | 1000.0 | 0.0 | 897.5 | 145.0 |
| 14 | H322T | bronchioalvo | 1000.0 | 0.0 | 509.1 | 694.2 | 1000.0 | 0.0 | 1000.0 | 0.0 |
| 15 | SW1573 | alveolar cell | 1000.0 | 0.0 | 1000.0 | 0.0 | 466.6 | 157.3 | 1000.0 | 0.0 |
| 16 | H292 | mucoepiderm | 1000.0 | 0.0 | 1000.0 | 0.0 | 293.3 | 69.3 | 1000.0 | 0.0 |
| 17 | H1650 | bronchioalvo | 1000.0 | 0.0 | 1000.0 | 0.0 | 1000.0 | 0.0 | 760.1 | 339.3 |
| 18 | H522 | adenocarcin | 1000.0 | 0.0 | 1000.0 | 0.0 | 1000.0 | 0.0 | 1000.0 | 0.0 |
| 19 | EKVX | adenocarcin | 1000.0 | 0.0 | 1000.0 | 0.0 | 1000.0 | 0.0 | 1000.0 | 0.0 |
| 20 | H661 | large cell | 1000.0 | 0.0 | 1000.0 | 0.0 | 1000.0 | 0.0 | 1000.0 | 0.0 |
| 21 | H23 | adenocarcin | 1000.0 | 0.0 | 1000.0 | 0.0 | 1000.0 | 0.0 | 1000.0 | 0.0 |
| 22 | LXFL 529 | large cell | 1000.0 | 0.0 | 1000.0 | 0.0 | 1000.0 | 0.0 | 1000.0 | 0.0 |
| 23 | H226 | squamous | 1000.0 | 0.0 | 1000.0 | 0.0 | 1000.0 | 0.0 | 1000.0 | 0.0 |
| 24 | A549 | | 1000.0 | 0.0 | 1000.0 | 0.0 | 1000.0 | 0.0 | 1000.0 | 0.0 |
| 25 | H1781 | bronchioalvo | 1000.0 | 0.0 | 1000.0 | 0.0 | 1000.0 | 0.0 | 1000.0 | 0.0 |
| 26 | H1299 | | 1000.0 | 0.0 | 1000.0 | 0.0 | 1000.0 | 0.0 | 1000.0 | 0.0 |
| 27 | HOP 62 | adenocarcin | 1000.0 | 0.0 | 1000.0 | 0.0 | 1000.0 | 0.0 | 1000.0 | 0.0 |
| 28 | H2009 | adenocarcin | 1000.0 | 0.0 | 1000.0 | 0.0 | 1000.0 | 0.0 | 1000.0 | 0.0 |
| 29 | HOP 92 | large cell | 1000.0 | 0.0 | 1000.0 | 0.0 | 1000.0 | 0.0 | 1000.0 | 0.0 |
| 30 | H1793 | adenocarcin | 1000.0 | 0.0 | 1000.0 | 0.0 | 1000.0 | 0.0 | 1000.0 | 0.0 |
| 31 | H1975 | adenocarcin | 1000.0 | 0.0 | 1000.0 | 0.0 | 1000.0 | 0.0 | 1000.0 | 0.0 |
| 32 | H1651 | adenocarcin | 1000.0 | 0.0 | 1000.0 | 0.0 | 1000.0 | 0.0 | 1000.0 | 0.0 |
| 33 | calu-1 | epidermoid c | 1000.0 | 0.0 | 1000.0 | 0.0 | 1000.0 | 0.0 | 1000.0 | 0.0 |
| 34 | H1435 | adenocarcin | 1000.0 | 0.0 | 1000.0 | 0.0 | 1000.0 | 0.0 | 1000.0 | 0.0 |
| 35 | HOP 18 | large cell | 1000.0 | 0.0 | 1000.0 | 0.0 | 1000.0 | 0.0 | 1000.0 | 0.0 |
| 36 | H520 | squamous | 1000.0 | 0.0 | 1000.0 | 0.0 | 1000.0 | 0.0 | 1000.0 | 0.0 |
| 37 | H441 | adenocarcin | 1000.0 | 0.0 | 1000.0 | 0.0 | 1000.0 | 0.0 | 1000.0 | 0.0 |
| 38 | H2030 | adenocarcin | 1000.0 | 0.0 | 1000.0 | 0.0 | 1000.0 | 0.0 | 1000.0 | 0.0 |
| 39 | H1155 | | 1000.0 | 0.0 | 1000.0 | 0.0 | 1000.0 | 0.0 | 1000.0 | 0.0 |
| 40 | H1838 | adenocarcin | 1000.0 | 0.0 | 1000.0 | 0.0 | 1000.0 | 0.0 | 1000.0 | 0.0 |
| 41 | H596 | adenosquare | 1000.0 | 0.0 | 1000.0 | 0.0 | 1000.0 | 0.0 | 1000.0 | 0.0 |
| 42 | HLFα | epidermoid | 1000.0 | 0.0 | 1000.0 | 0.0 | 1000.0 | 0.0 | 1000.0 | 0.0 |

*FIG. 5*

| Pancreatic Cancer IC50 Summary | | Apo2L 0.5% FBS MTT | | 10% FBS | | DR5 Ab+XL 0.5% FBS MTT | | 10% FBS | |
|---|---|---|---|---|---|---|---|---|---|
| | | Mean | STD | Mean | STD | Mean | STD | Mean | STD |
| 1 | Panc 05.04 | 40.1 | 5.3 | 26.8 | 20.1 | 24.9 | 4.3 | 23.3 | 12.8 |
| 2 | BxPC3 | 247.0 | 117.6 | 34.3 | 11.6 | 364.6 | 113.5 | 411.8 | 12.7 |
| 3 | HPAC | 1000.0 | 0.0 | 10.8 | 1.3 | 1000.0 | 0.0 | 580.8 | 323.7 |
| 4 | SU.86.86 | 68.5 | 69.3 | 27.8 | 9.9 | 247.6 | 162.3 | 390.6 | 10.9 |
| 5 | HuP-T3 | 629.3 | 524.3 | 57.5 | 62.3 | 421.0 | 271.5 | 367.7 | 95.7 |
| 6 | PSN1 | 715.7 | 492.4 | 357.5 | 556.9 | 305.0 | 163.7 | 423.4 | 223.4 |
| 7 | MiaPaCa-2 | 563.5 | 617.3 | 1000.0 | 0.0 | 66.0 | 16.1 | 137.4 | 50.8 |
| 8 | Panc 08.13 | 1000.0 | 0.0 | 1000.0 | 0.0 | 44.8 | 28.7 | 17.0 | 3.1 |
| 9 | PA-TU-8988T | 1000.0 | 0.0 | 1000.0 | 0.0 | 368.0 | 133.8 | 375.4 | 119.4 |
| 10 | Panc 03.27 | 1000.0 | 0.0 | 288.7 | 189.1 | 1000.0 | 0.0 | 1000.0 | 0.0 |
| 11 | Capan-1 | 371.2 | 545.1 | 681.2 | 552.2 | 613.4 | 437.1 | 793.6 | 357.4 |
| 12 | SW 1990 | 158.3 | 20.5 | 844.7 | 269.0 | 467.8 | 416.0 | 913.6 | 149.7 |
| 13 | CFPAC-1 | 883.4 | 164.9 | 535.4 | 657.1 | 381.9 | 22.6 | 925.5 | 105.4 |
| 14 | PL45 | 1000.0 | 0.0 | 1000.0 | 0.0 | 538.7 | 652.4 | 1000.0 | 0.0 |
| 15 | PA-TU-8902 | 871.5 | 181.8 | 1000.0 | 0.0 | 1000.0 | 0.0 | 1000.0 | 0.0 |
| 16 | Aspc-1 | 1000.0 | 0.0 | 1000.0 | 0.0 | 1000.0 | 0.0 | 1000.0 | 0.0 |
| 17 | Hs766T | 1000.0 | 0.0 | 1000.0 | 0.0 | 1000.0 | 0.0 | 1000.0 | 0.0 |
| 18 | Panc 10.05 | 1000.0 | 0.0 | 1000.0 | 0.0 | 1000.0 | 0.0 | 1000.0 | 0.0 |
| 19 | Panc1 | 1000.0 | 0.0 | 1000.0 | 0.0 | 1000.0 | 0.0 | 1000.0 | 0.0 |
| 20 | Capan-2 | 1000.0 | 0.0 | 1000.0 | 0.0 | 1000.0 | 0.0 | 1000.0 | 0.0 |
| 21 | HPAF-II | 1000.0 | 0.0 | 1000.0 | 0.0 | 1000.0 | 0.0 | 1000.0 | 0.0 |
| 22 | Panc 02.03 | 1000.0 | 0.0 | 1000.0 | 0.0 | 1000.0 | 0.0 | 1000.0 | 0.0 |
| 23 | Panc 04.03 | 1000.0 | 0.0 | 1000.0 | 0.0 | 1000.0 | 0.0 | 1000.0 | 0.0 |

*FIG. 6*

| NHL IC50 Summary | | | Apo2L 10% FBS MTT | | DR5 Ab+XL 10% FBS MTT | | |
|---|---|---|---|---|---|---|---|
| | | | Mean | STD | Mean | STD | |
| 1 | JEKO-1 | *MCL* | 11.5 | 6.4 | 61.8 | 10.7 | **7% Apo2L** |
| 2 | SU-DHL-4 | *DLBCL* | 1504.1 | 1727.4 | 13.6 | 5.6 | |
| 3 | OCI-LY-19 | *DLBCL* | 3000 | 0 | 26.2 | 15.8 | **29% DR5 Ab+** |
| 4 | SR | *DLBCL* | 3000 | 0 | 187.8 | 43.7 | |
| 5 | Farage | *DLBCL* | 3000 | 0 | 1269.1 | 1504.1 | |
| 6 | DOHH-2 | *DLBCL, FL* | 3000 | 0 | 84% death | anti-hu IgG | |
| 7 | Toledo | *DLBCL* | 3000 | 0 | 3000 | 0 | |
| 8 | WSU-NHL | *FL* | 3000 | 0 | 3000 | 0 | |
| 9 | KARPAS-422 | *DLBCL, FL* | 3000 | 0 | 3000 | 0 | |
| 10 | GRANTA-519 | *NHL, MCL* | 3000 | 0 | 3000 | 0 | |
| 11 | Pfeiffer | *DLBCL* | 3000 | 0 | 3000 | 0 | |
| 12 | HT | *DLBCL* | 3000 | 0 | 3000 | 0 | |
| 13 | SC-1 | *FL* | 3000 | 0 | 3000 | 0 | |
| 14 | DB | *DLBCL* | 3000 | 0 | 3000 | 0 | |

***FIG. 7***

FIG. 8

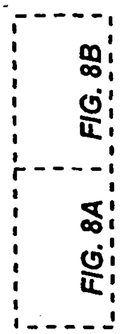
FIG. 8A
FIG. 8B

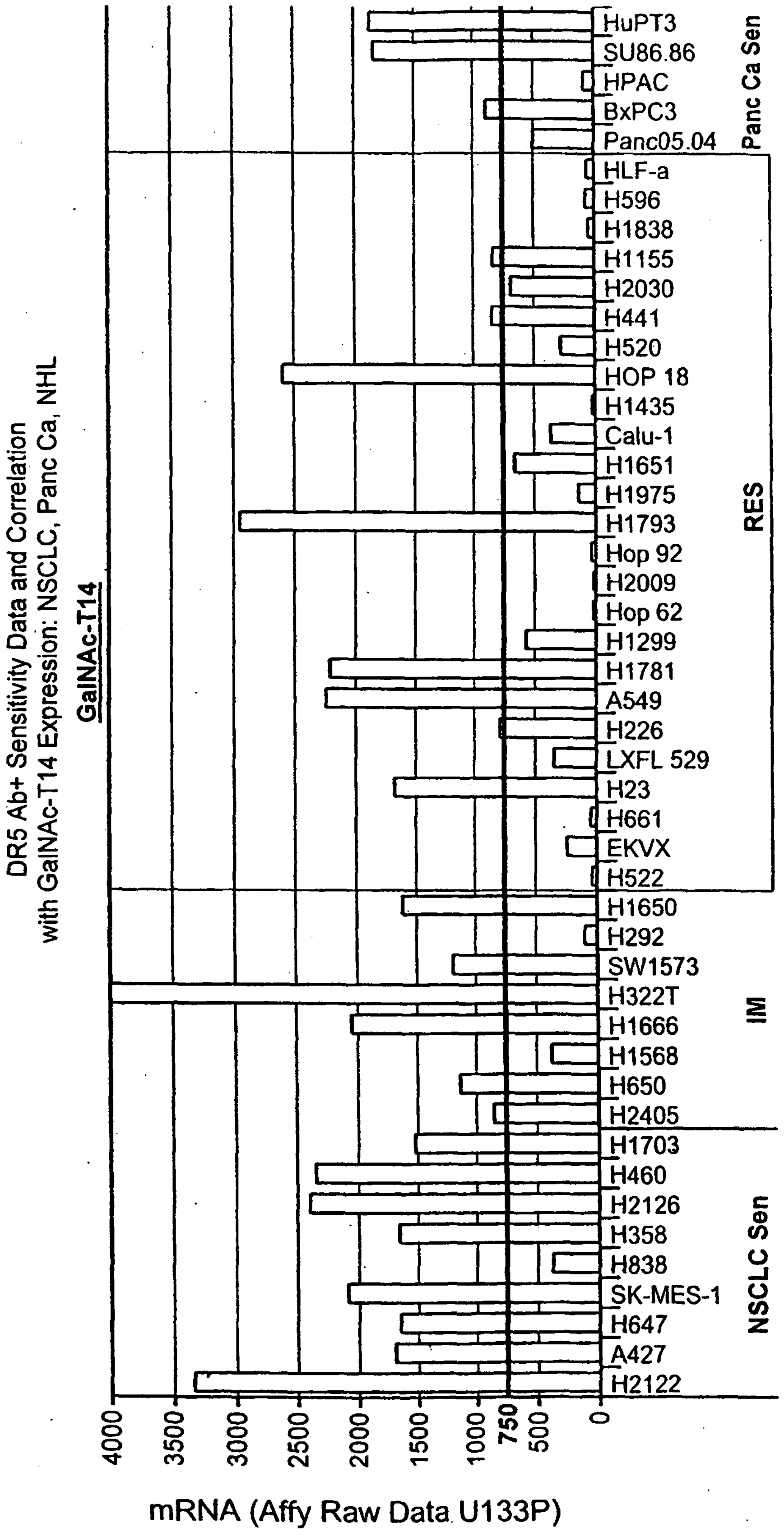
DR5 Ab+ Sensitivity Data and Correlation
with GalNAc-T14 Expression: NSCLC, Panc Ca, NHL
GalNAc-T14
HuPT3
SU86.86
HPAC
BxPC3
Panc05.04
HLF-a
H596
H1838
H1155
H2030
H441
H520
HOP 18
H1435
Calu-1
H1651
H1975
H1793
Hop 92
H2009
Hop 62
H1299
H1781
A549
H226
LXFL 529
H23
H661
EKVX
H522
H1650
H292
SW1573
H322T
H1666
H1568
H650
H2405
H1703
H460
H2126
H358
H838
SK-MES-1
H647
A427
H2122
Panc Ca Sen
RES
IM
NSCLC Sen
4000
3500
3000
2500
2000
1500
1000
750
500
0
mRNA (Affy Raw Data U133P)

FIG. 8A

| | Sen/IM | Res | |
|---|---|---|---|
| >750 | 27 | 8 | 35 |
| <750 | 7 | 35 | 42 |
| | 34 | 43 | 77 |

Sensitivity 27/34=79%
Specificity 35/43=81%
Positive Predictive Value 27/35=77%
***Negative Predictive Value 35/42=83%***

Fisher's Exact Test
p-value = 8.251603801901834e-8 (for Apo2L: 0.000054)

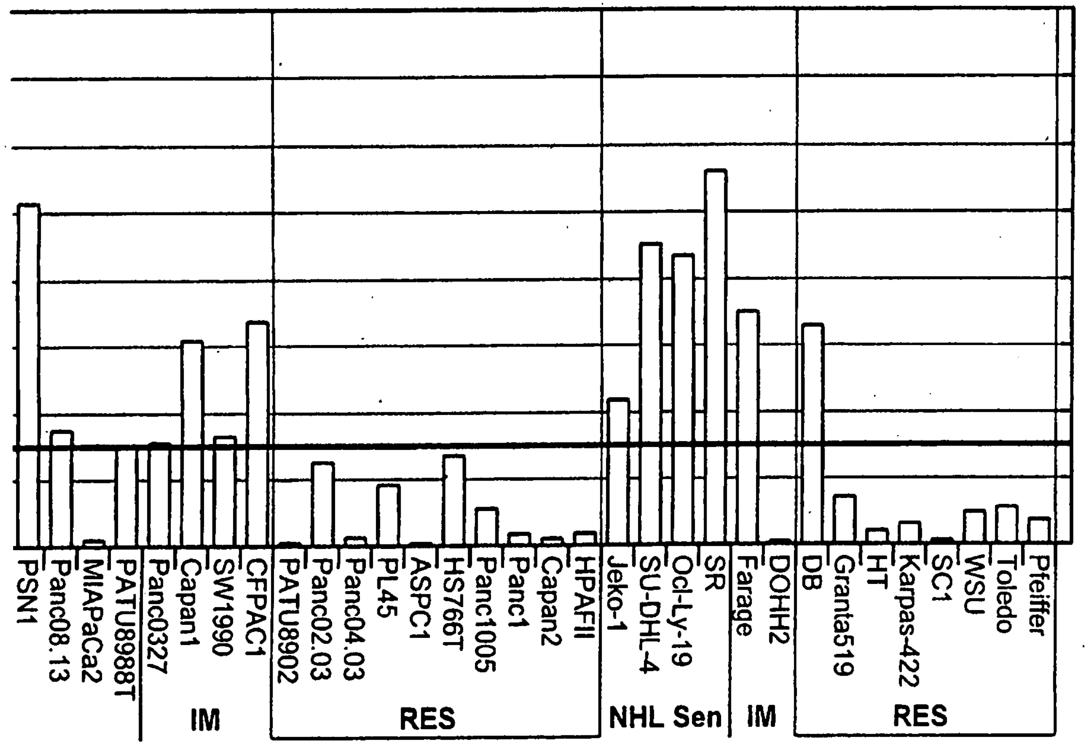


*FIG. 8B*

FIG. 9

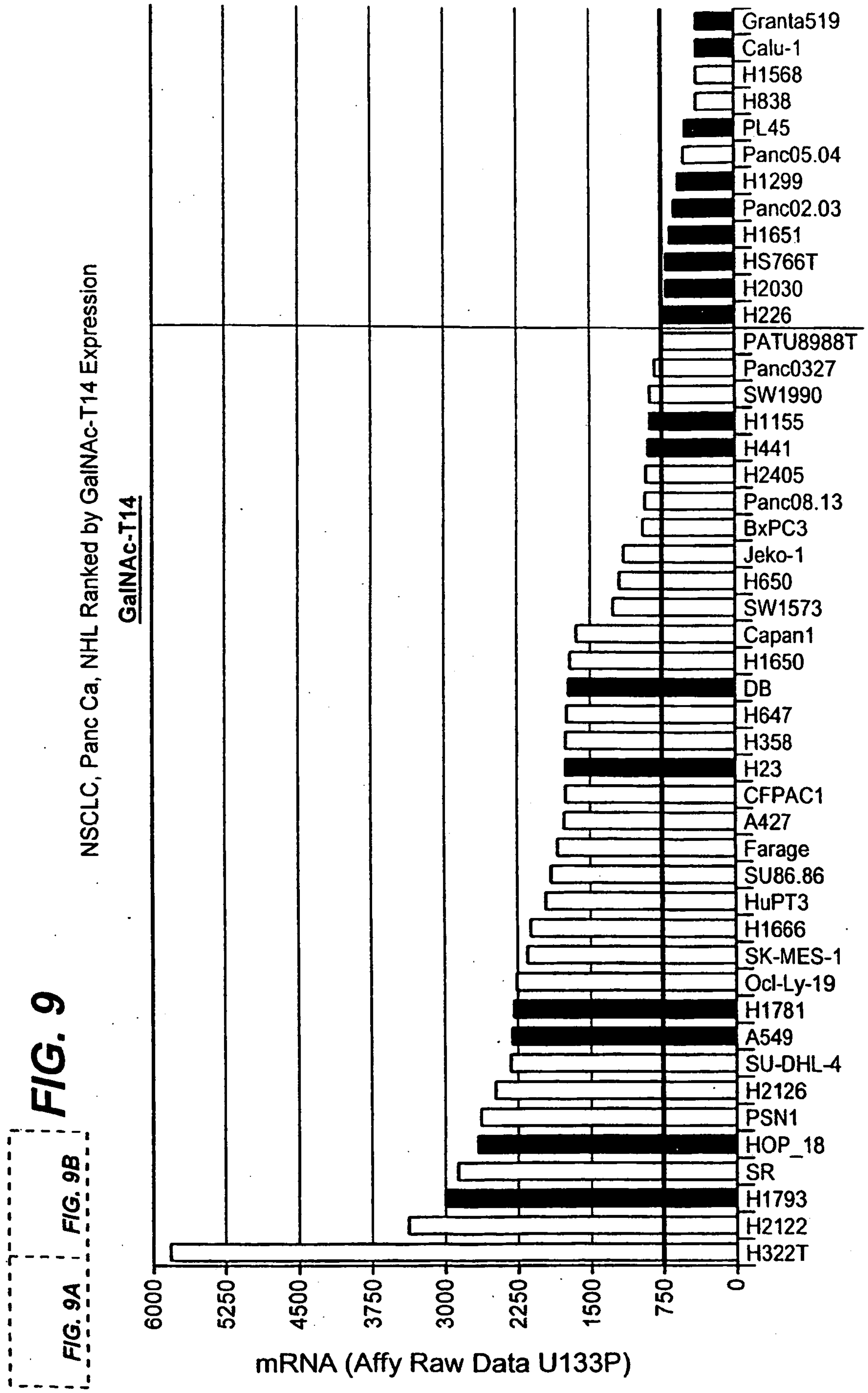
FIG. 9A
FIG. 9B
NSCLC, Panc Ca, NHL Ranked by GalNAc-T14 Expression
GalNAc-T14
Granta519
Calu-1
H1568
H838
PL45
Panc05.04
H1299
Panc02.03
H1651
HS766T
H2030
H226
PATU8988T
Panc0327
SW1990
H1155
H441
H2405
Panc08.13
BxPC3
Jeko-1
H650
SW1573
Capan1
H1650
DB
H647
H358
H23
CFPAC1
A427
Farage
SU86.86
HuPT3
H1666
SK-MES-1
Ocl-Ly-19
H1781
A549
SU-DHL-4
H2126
PSN1
HOP_18
SR
H1793
H2122
H322T
6000
5250
4500
3750
3000
2250
1500
750
0
mRNA (Affy Raw Data U133P)

FIG. 9A

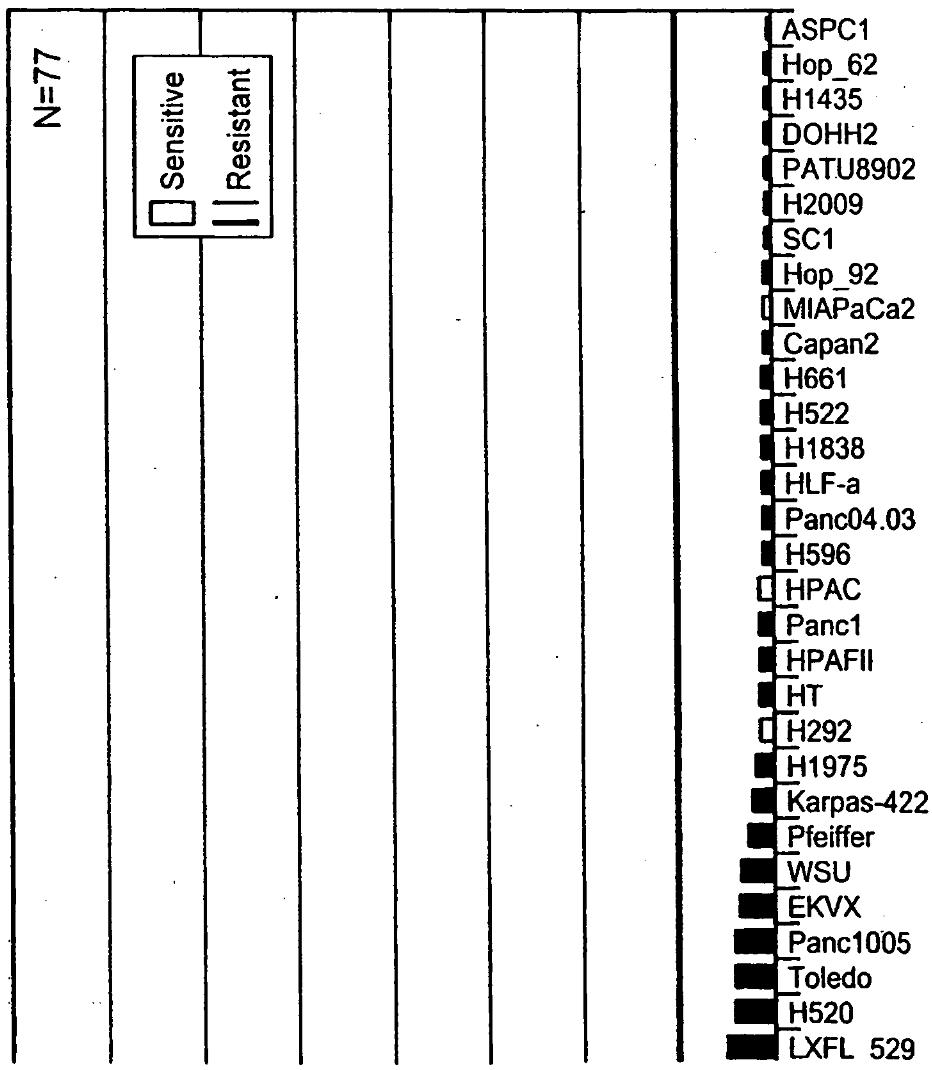
N=77
Sensitive
Resistant
ASPC1
Hop_62
H1435
DOHH2
PATU8902
H2009
SC1
Hop_92
MIAPaCa2
Capan2
H661
H522
H1838
HLF-a
Panc04.03
H596
HPAC
Panc1
HPAFII
HT
H292
H1975
Karpas-422
Pfeiffer
WSU
EKVX
Panc1005
Toledo
H520
LXFL 529

*FIG. 9B*

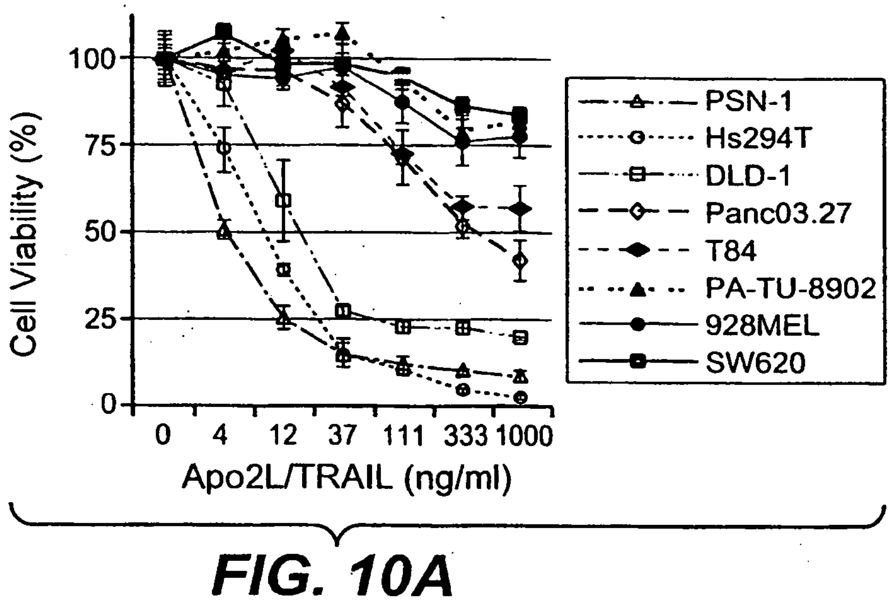


FIG. 10A

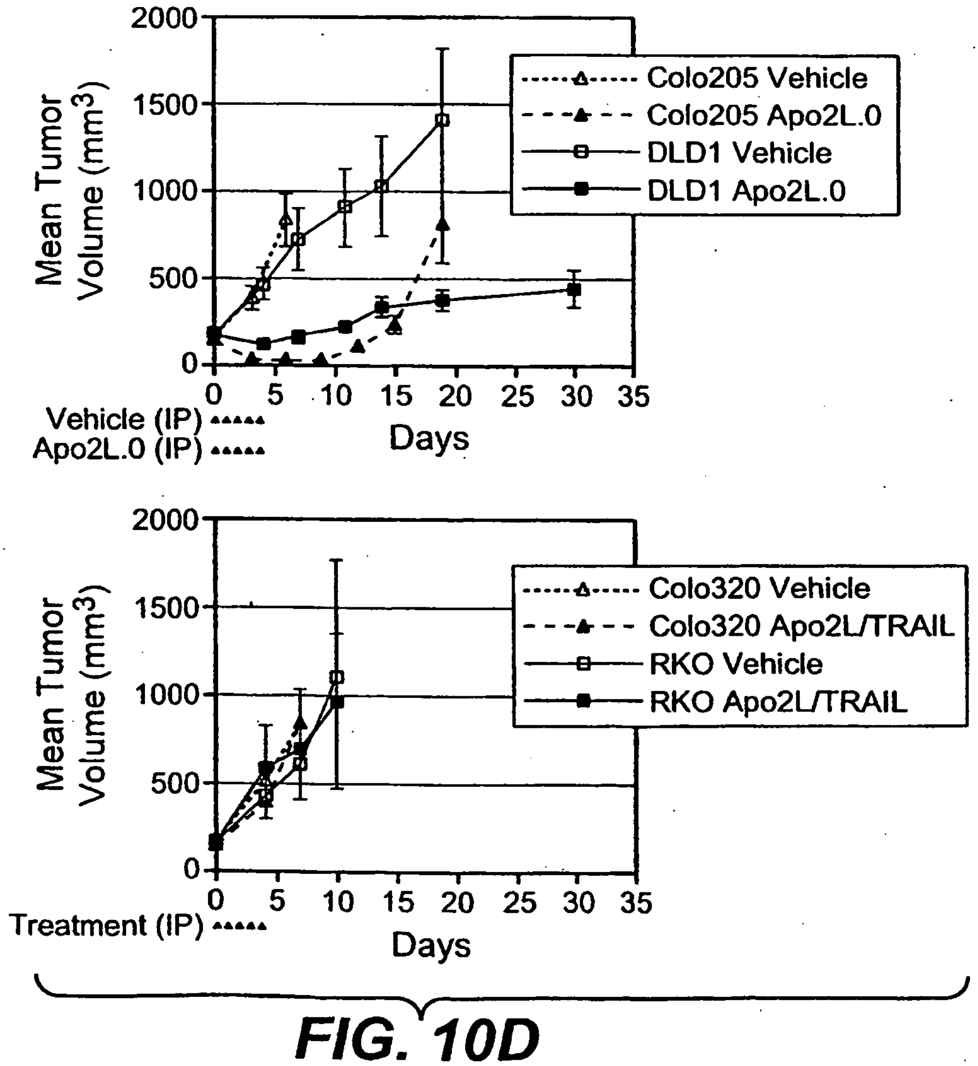


FIG. 10D

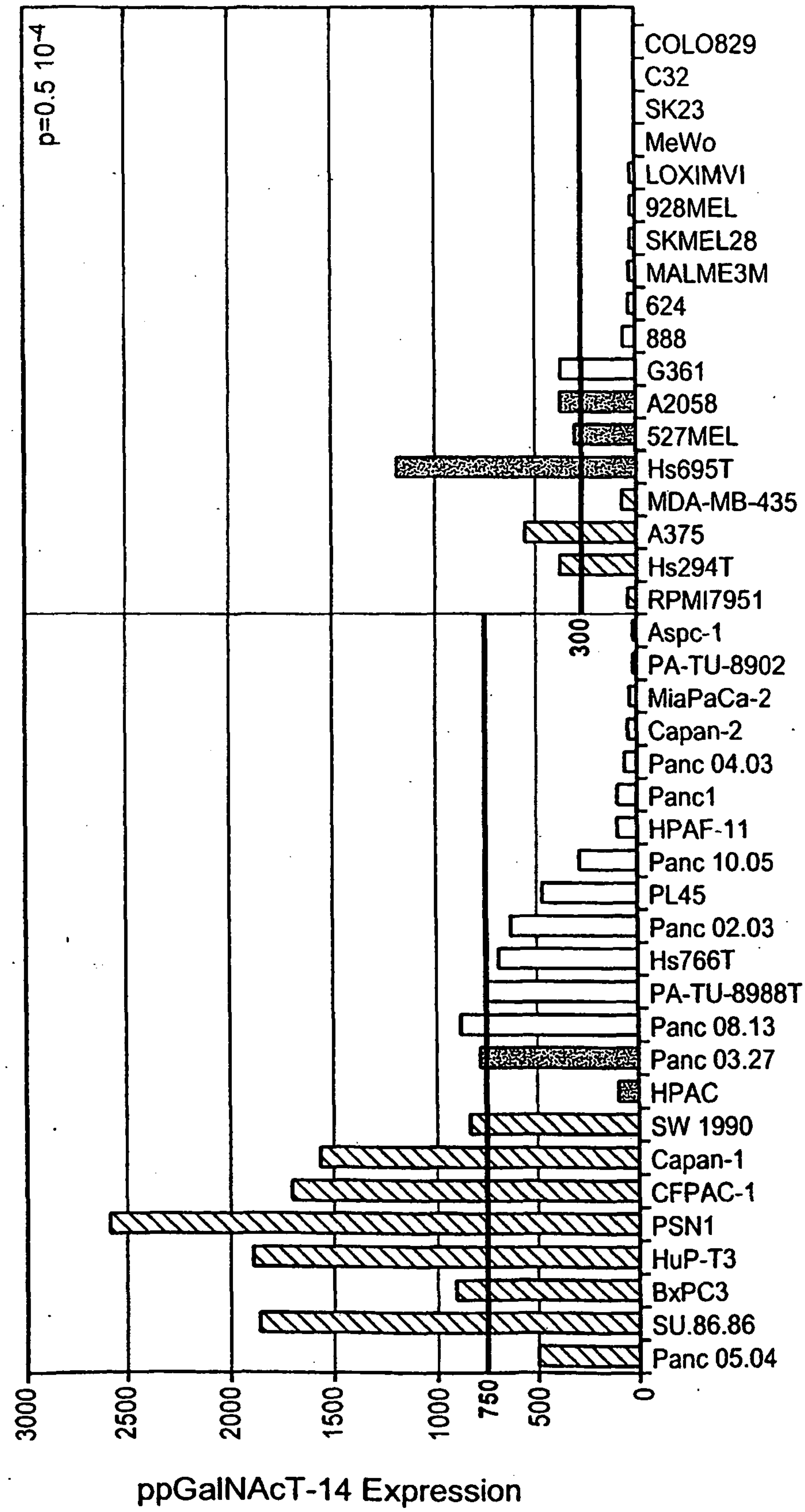
p=0.5 10-4
COLO829
C32
SK23
MeWo
LOXIMVI
928MEL
SKMEL28
MALME3M
624
888
G361
A2058
527MEL
Hs695T
MDA-MB-435
A375
Hs294T
RPMI7951
Aspc-1
PA-TU-8902
MiaPaCa-2
Capan-2
Panc 04.03
Panc1
HPAF-11
Panc 10.05
PL45
Panc 02.03
Hs766T
PA-TU-8988T
Panc 08.13
Panc 03.27
HPAC
SW 1990
Capan-1
CFPAC-1
PSN1
HuP-T3
BxPC3
SU.86.86
Panc 05.04
300
3000
2500
2000
1500
1000
750
500
0
ppGalNAcT-14 Expression

FIG. 10B

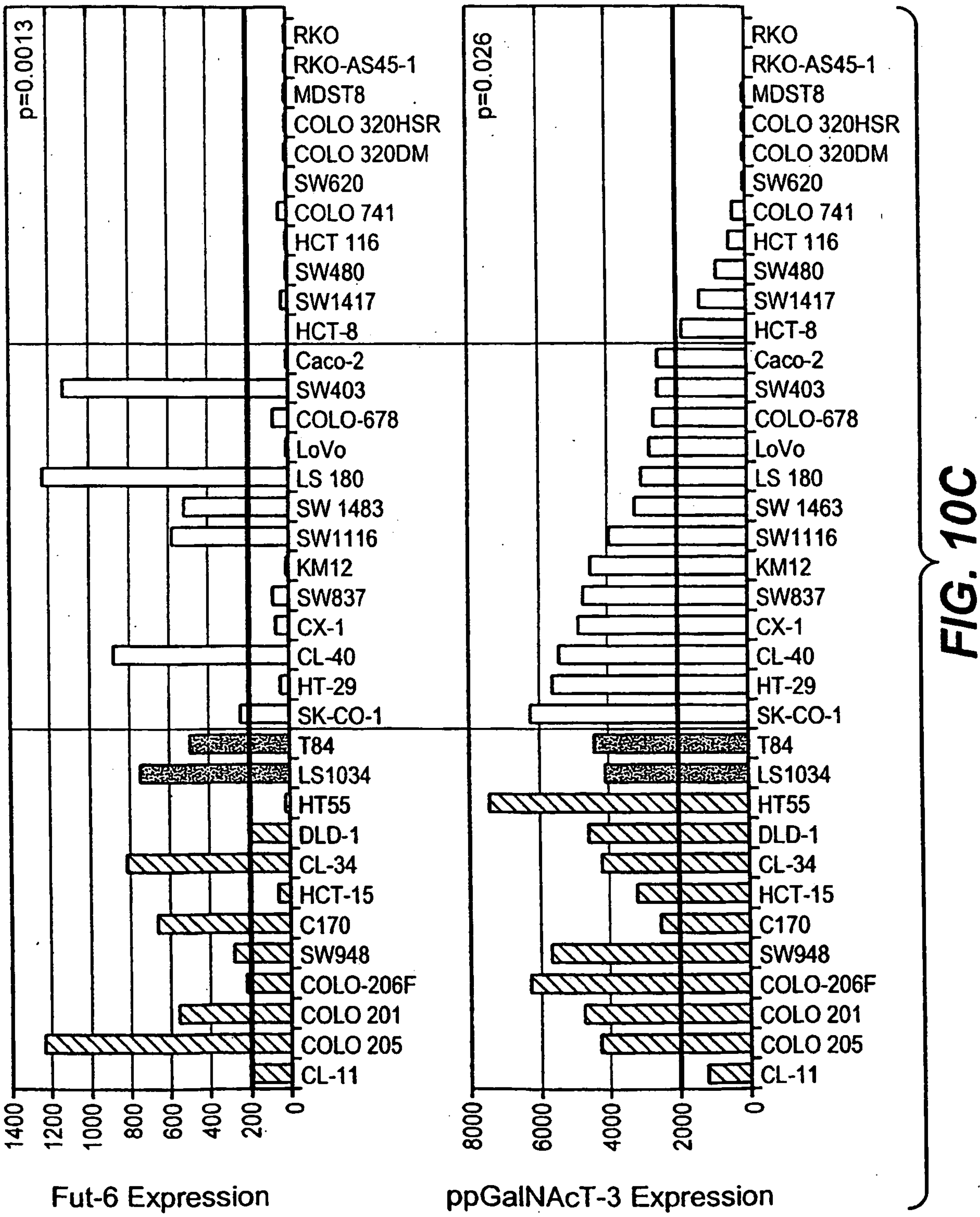
p=0.0013
p=0.026
RKO
RKO-AS45-1
MDST8
COLO 320HSR
COLO 320DM
SW620
COLO 741
HCT 116
SW480
SW1417
HCT-8
Caco-2
SW403
COLO-678
LoVo
LS 180
SW 1483
SW 1463
SW1116
KM12
SW837
CX-1
CL-40
HT-29
SK-CO-1
T84
LS1034
HT55
DLD-1
CL-34
HCT-15
C170
SW948
COLO-206F
COLO 201
COLO 205
CL-11
1400 1200 1000 800 600 400 200 0
8000 6000 4000 2000 0
Fut-6 Expression
ppGalNAcT-3 Expression

FIG. 10C

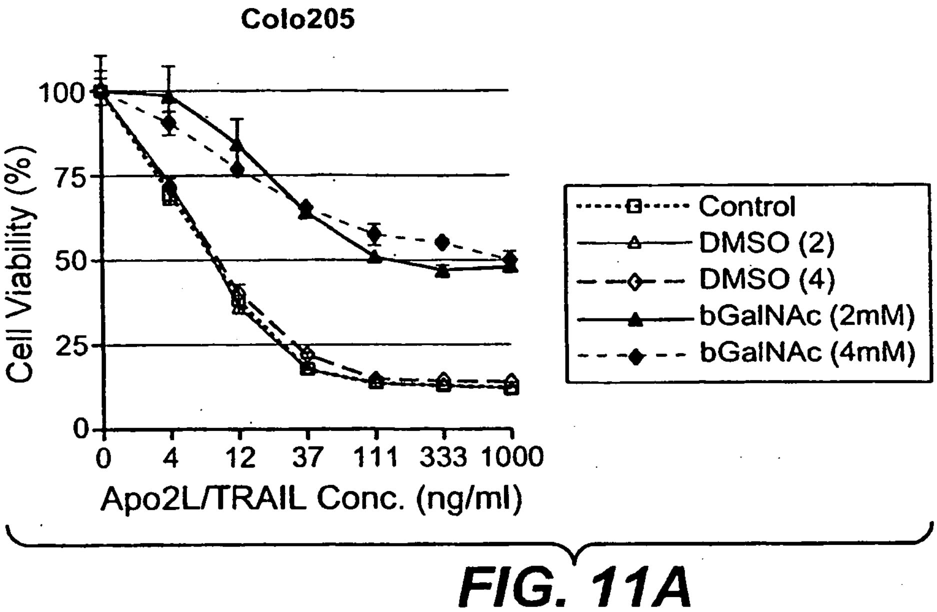


FIG. 11A

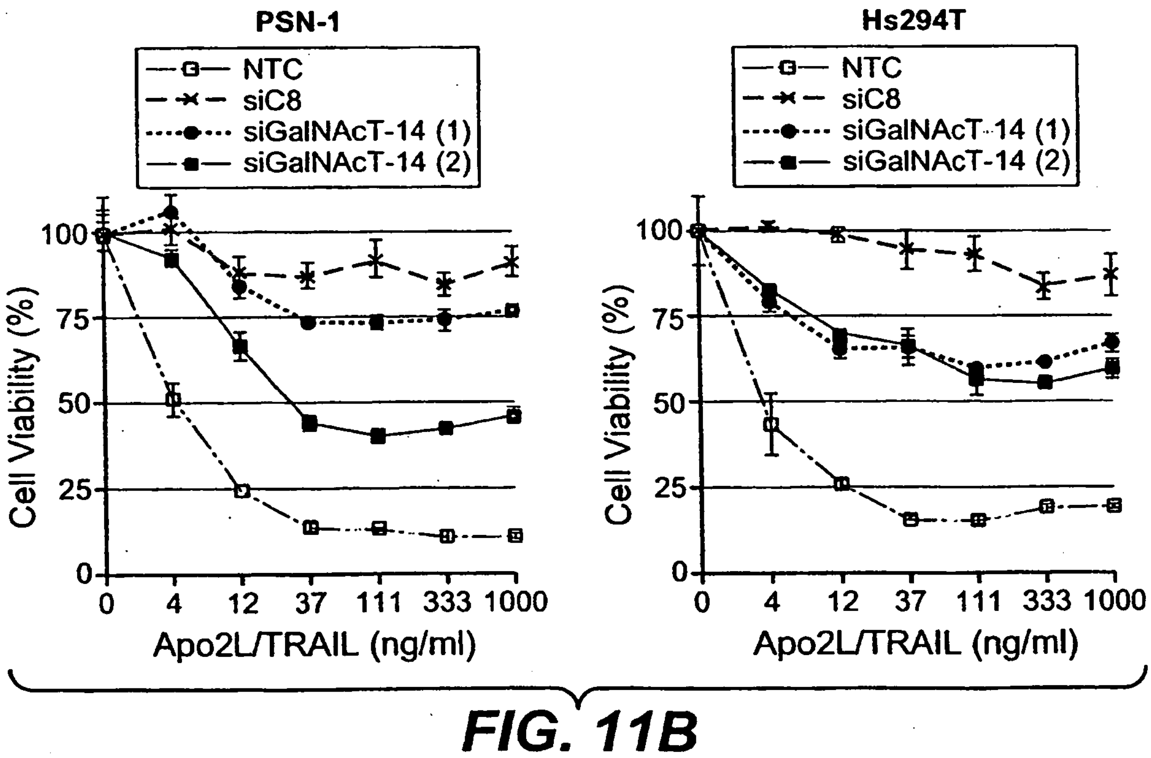


FIG. 11B

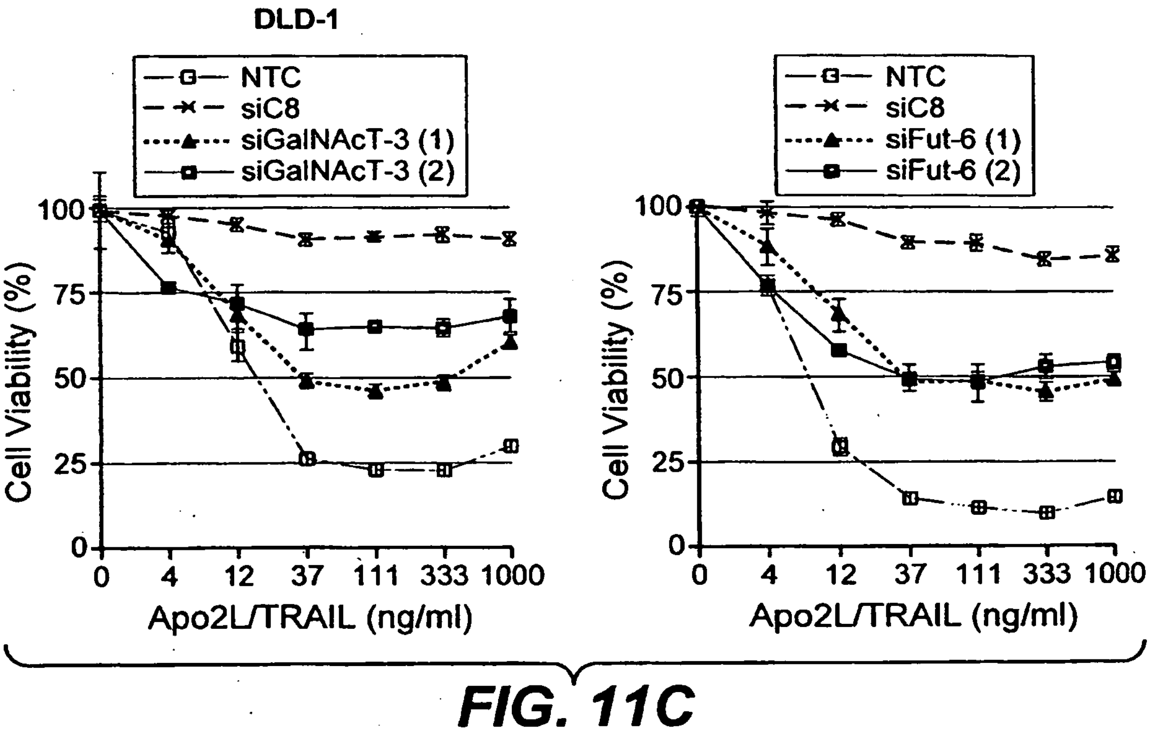


FIG. 11C

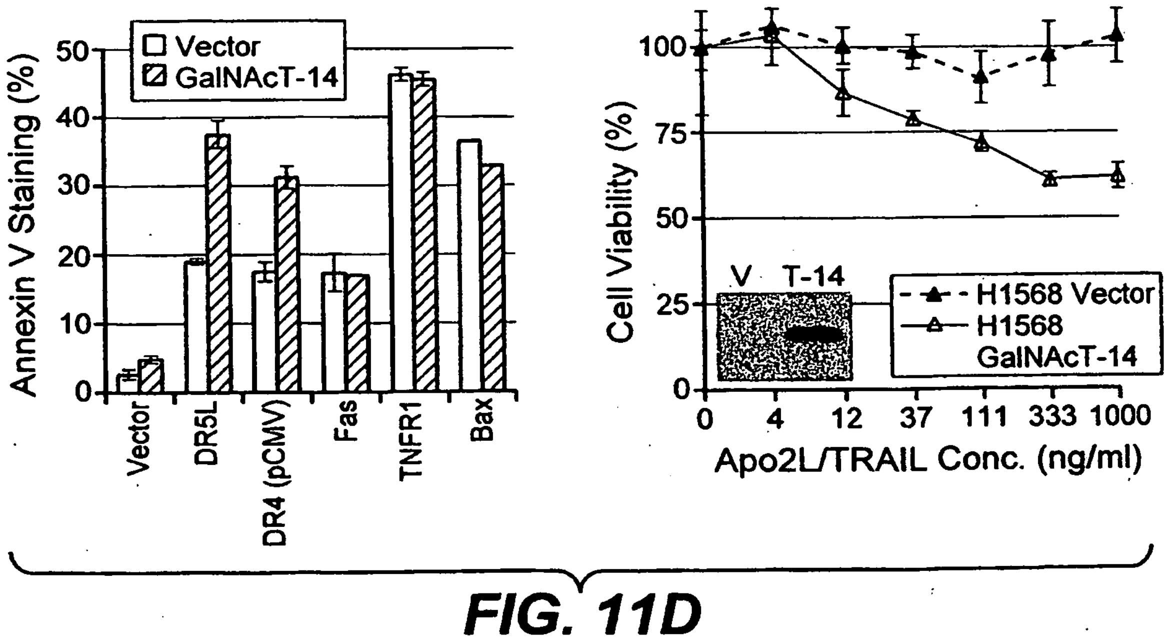


FIG. 11D

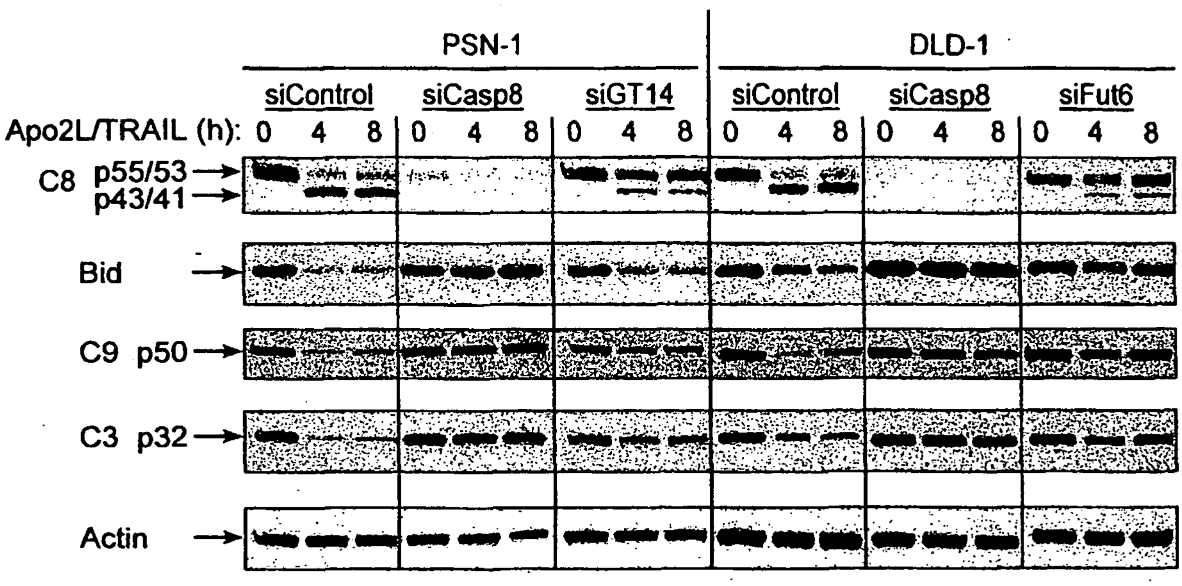


*FIG. 12A*

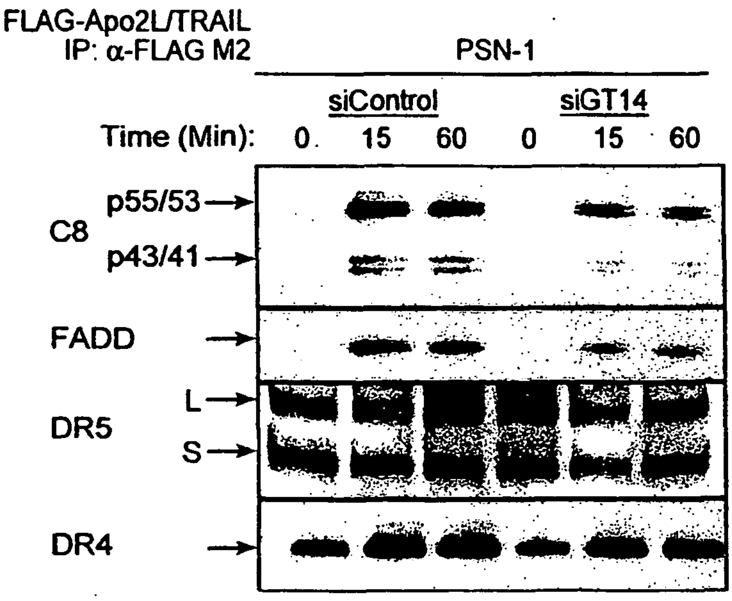


*FIG. 12C*

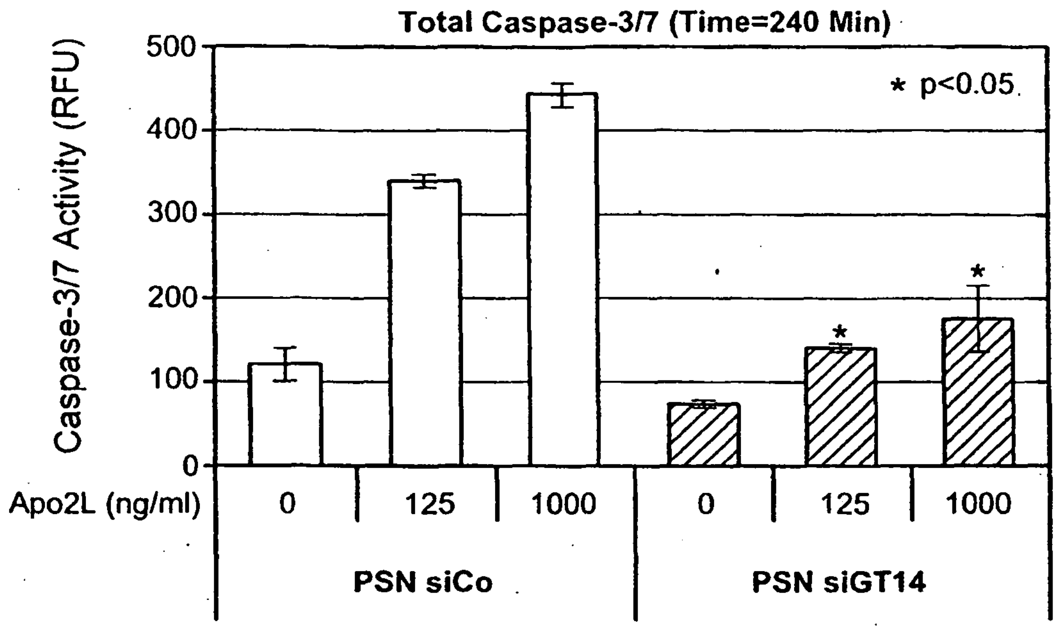


*FIG. 12B*

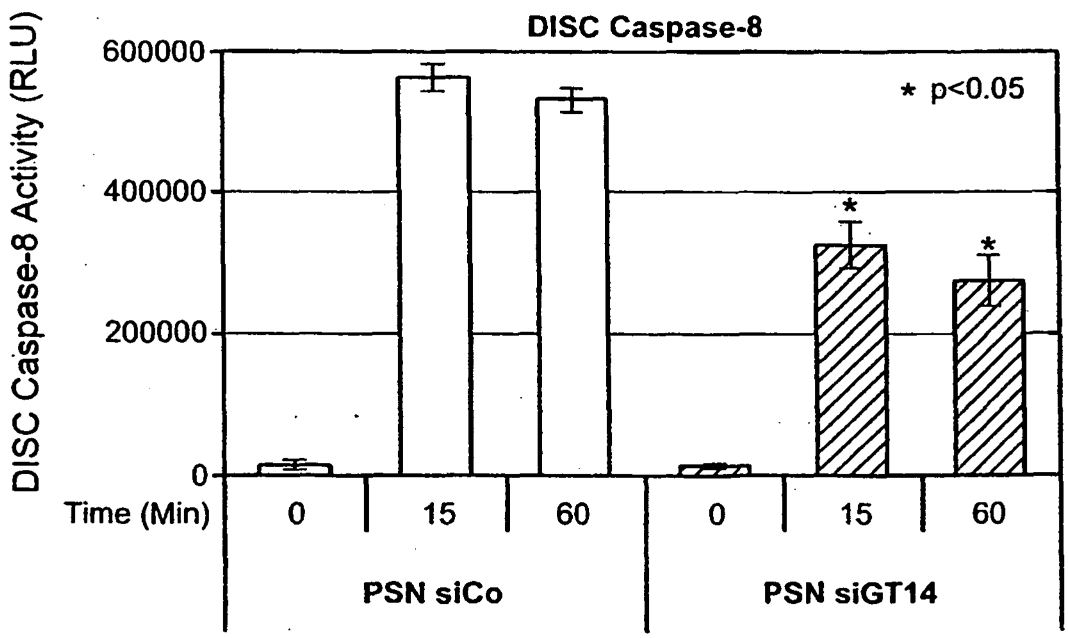


*FIG. 12D*

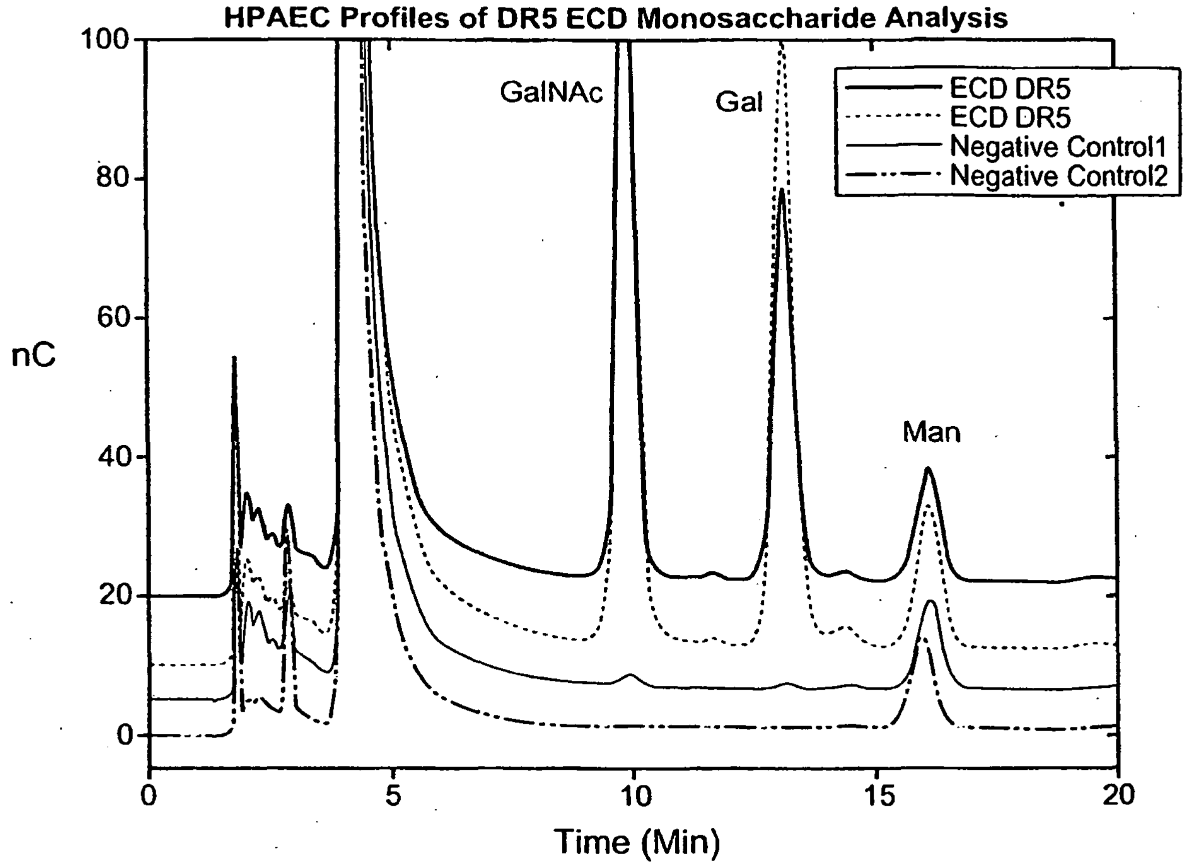


*FIG. 13A*

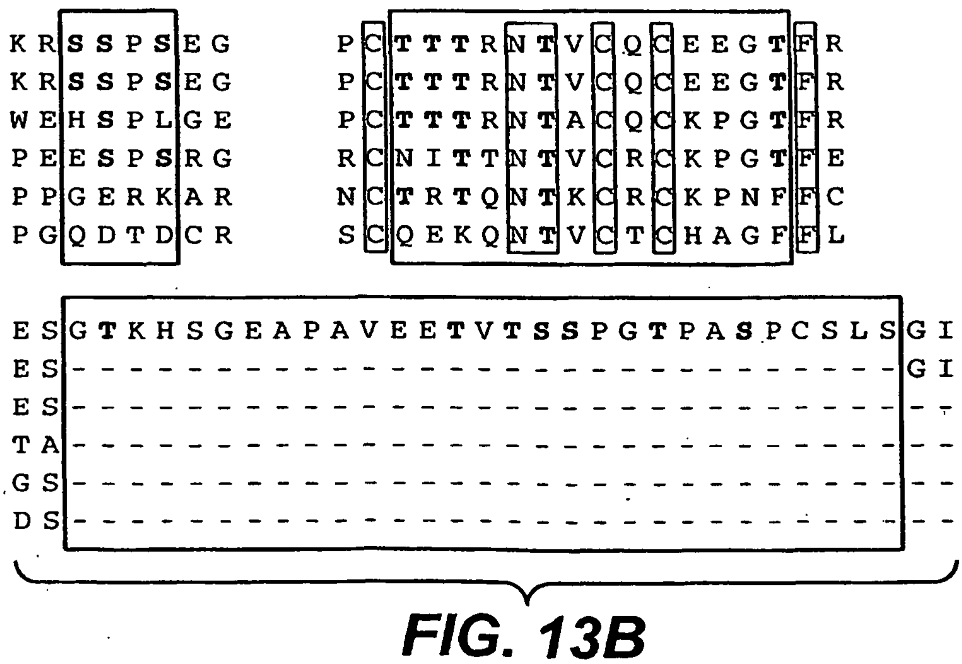


*FIG. 13B*

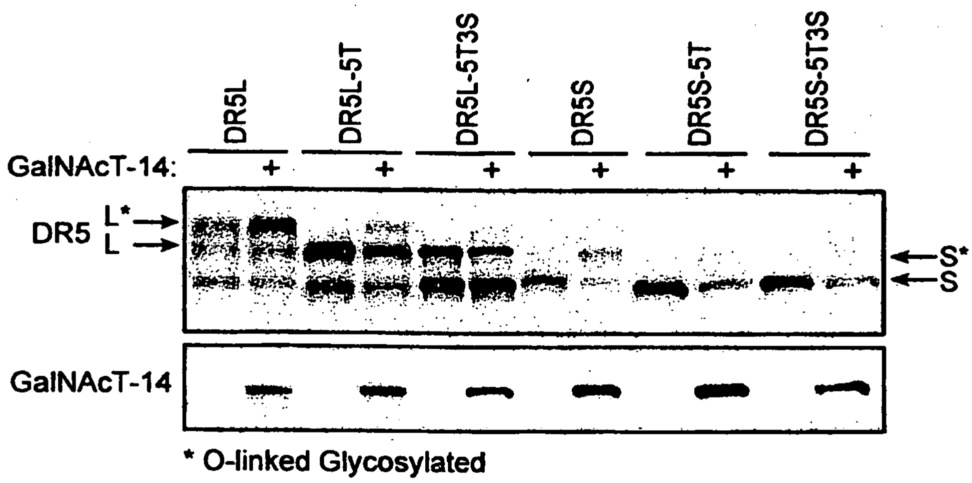


*FIG. 13C*

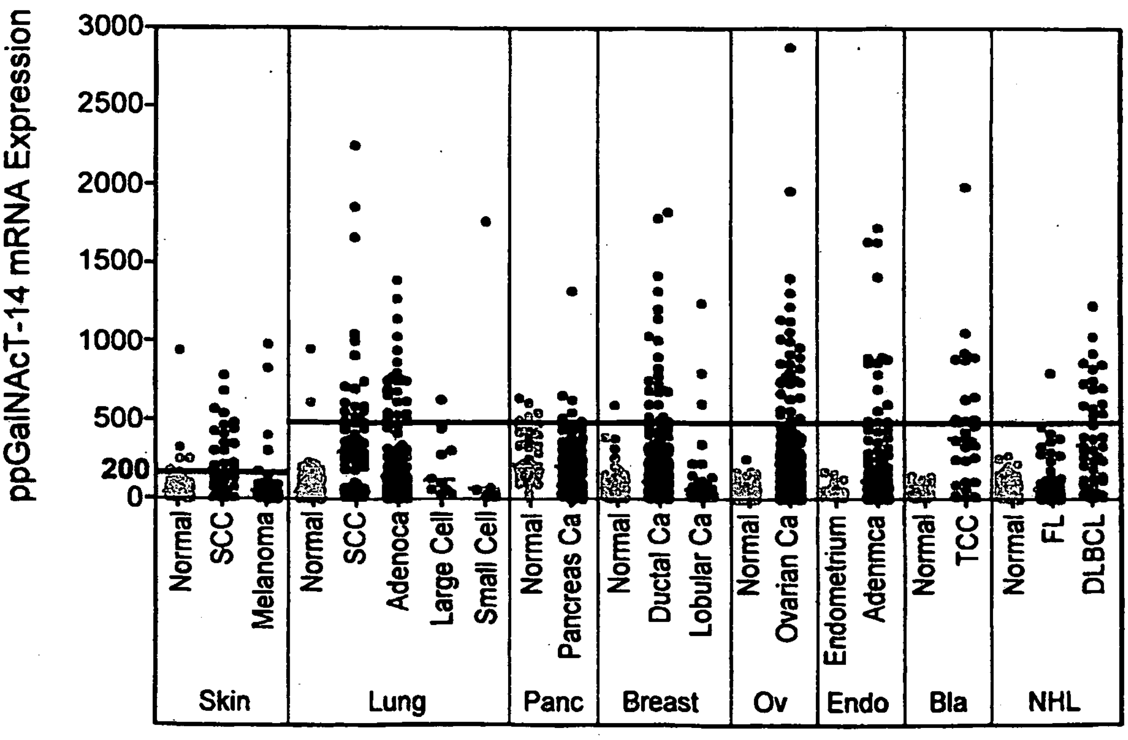


*FIG. 13E*

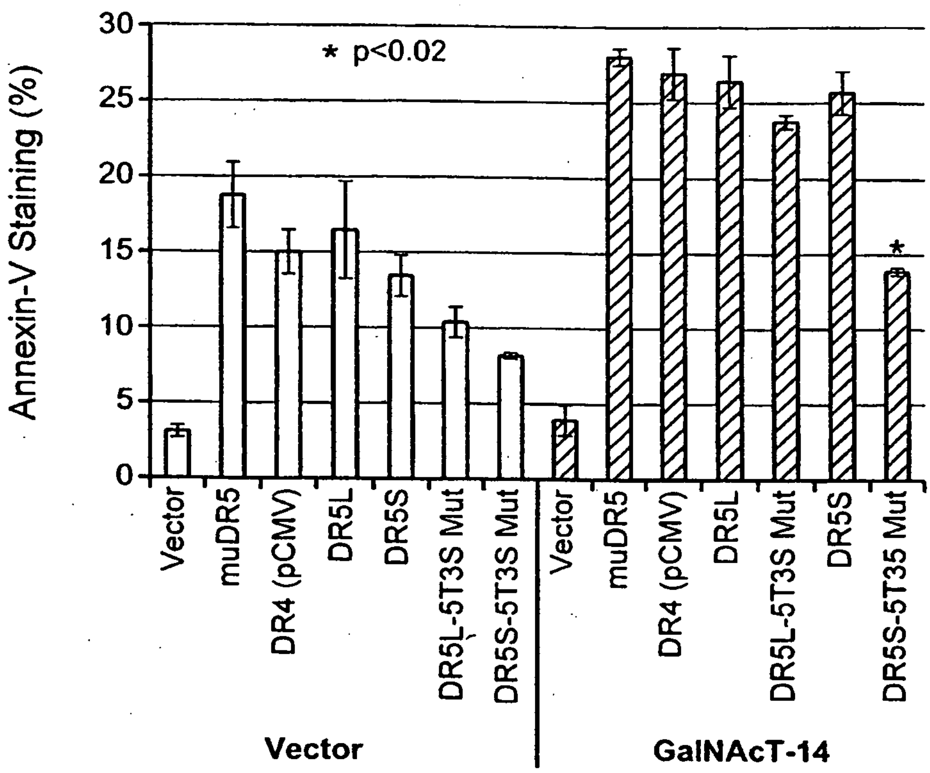
Annexin-V Staining (%)
30
25
20
15
10
5
0
* p<0.02
*
Vector
muDR5
DR4 (pCMV)
DR5L
DR5S
DR5L-5T3S Mut
DR5S-5T3S Mut
Vector
muDR5
DR4 (pCMV)
DR5L
DR5L-5T3S Mut
DR5S
DR5S-5T35 Mut
Vector
GalNAcT-14

*FIG. 13D*

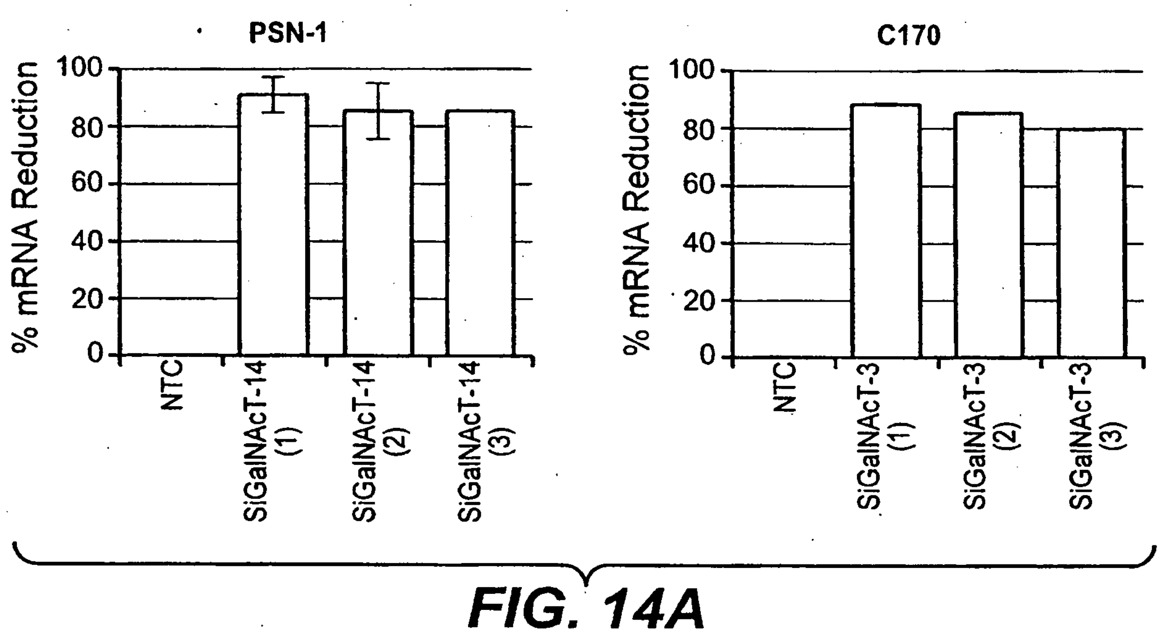


*FIG. 14A*

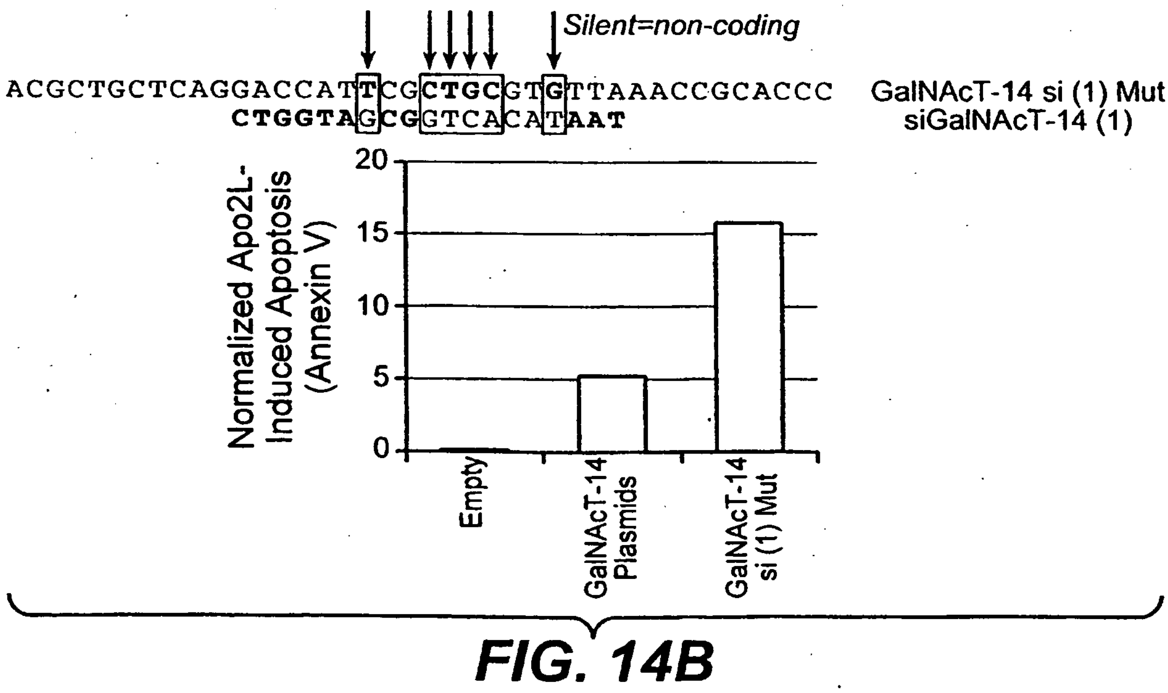


*FIG. 14B*

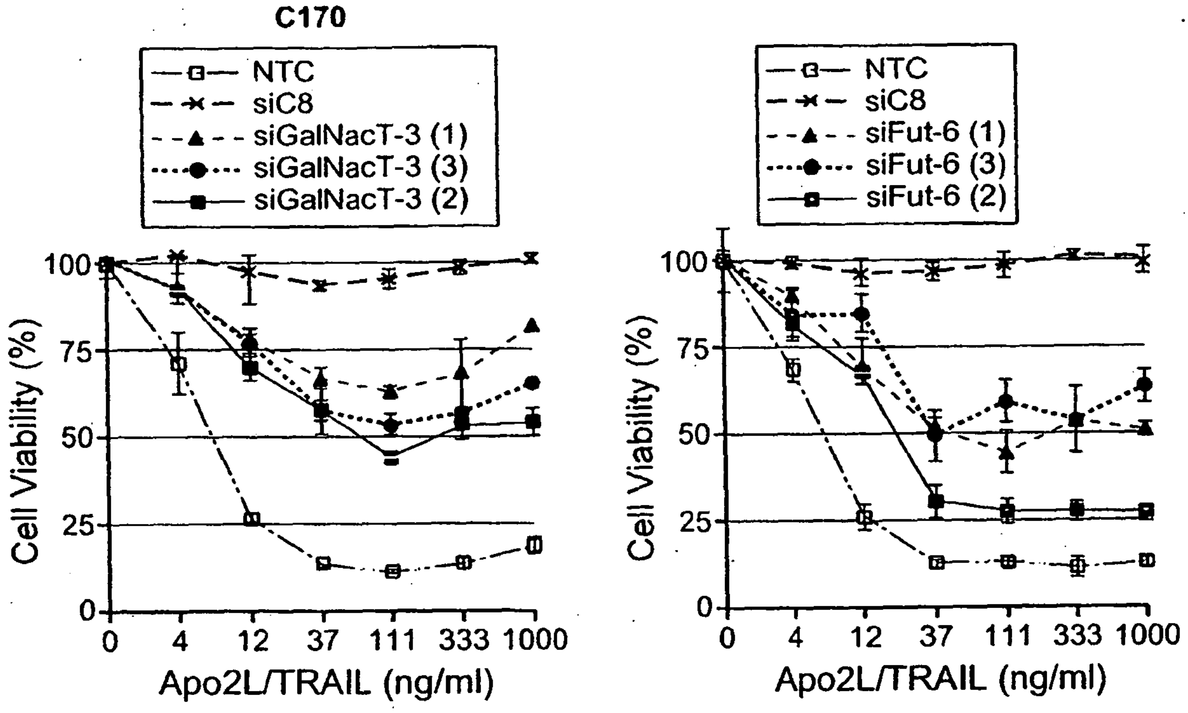


**T1**

| Tumor Type | Cell Line | Transient RNAi | Retroviral shRNA | Apo2L/TRAIL Protection by Knock-down: ppGalNAcT-14 | ppGalNAcT-13 | Fut-6 |
|---|---|---|---|---|---|---|
| Pancreatic Cancer | PSN-1 | Yes | | + + | | |
| | Panc 05.04 | Yes | | + + | | |
| | Panc 03.27 | Yes | | + | | |
| | Su.86.8 | No | Yes | + | | |
| | Hup-13 | Yes | | 0 | | |
| | | | | 4/5 | | |
| Melanoma | A375 | Yes | | + + | | |
| | Hs294T | Yes | | + + | | |
| | | | | 2/2 | | |
| Colorectal Cancer | C170 | Yes | | | + + | + + |
| | DLD-1 | Yes | | | + + | + + |
| | SW948 | Yes | | | 0 | 0 |
| | | | | | 2/3 | 2/3 |

*FIG. 14C*

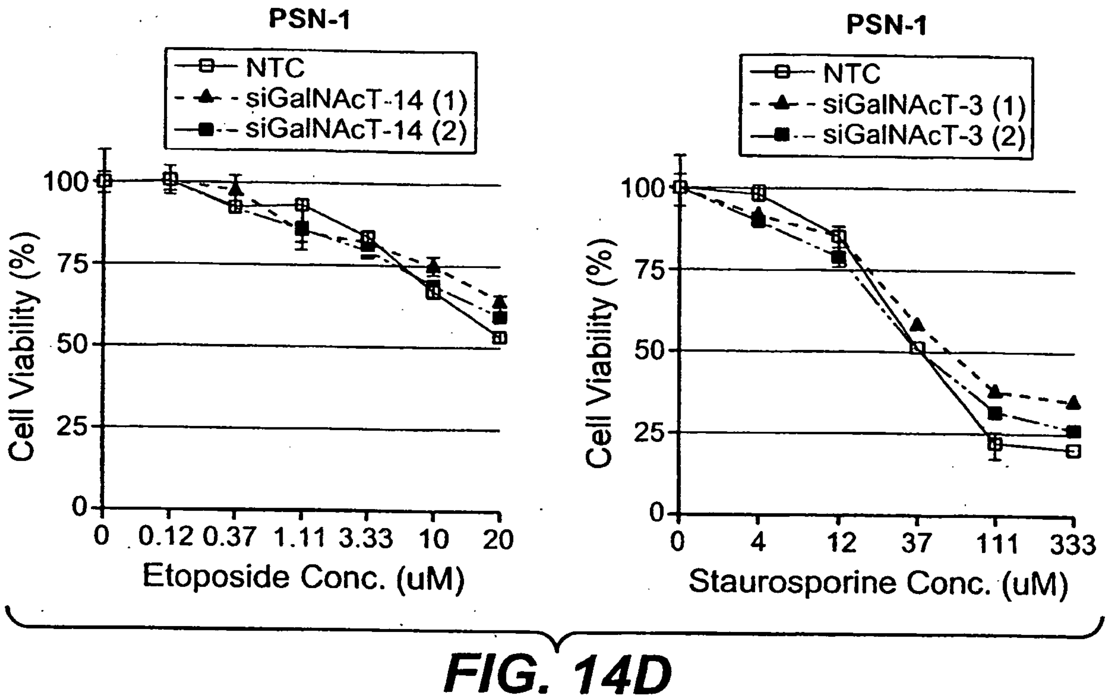


*FIG. 14D*

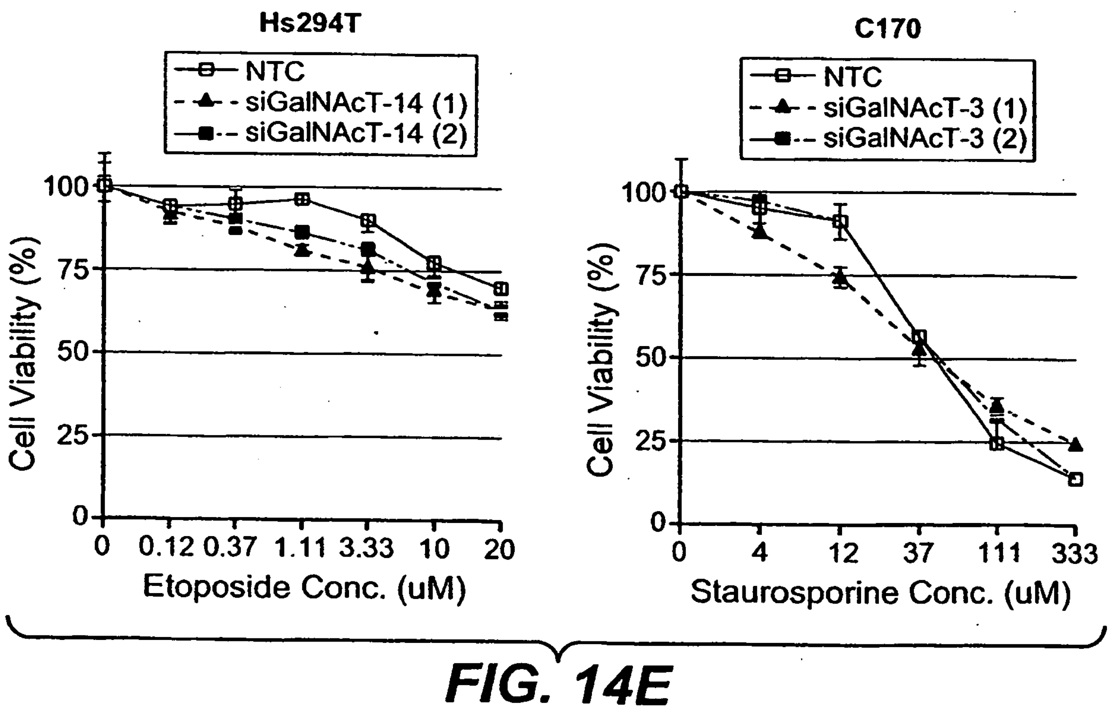


*FIG. 14E*

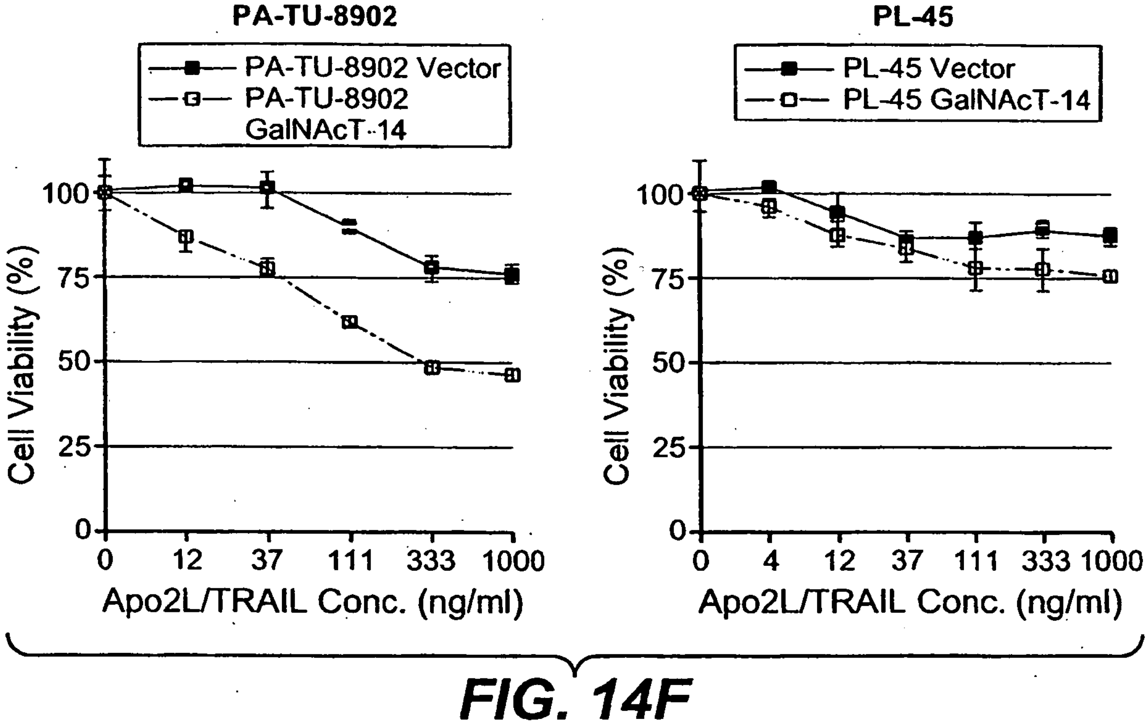
PA-TU-8902
PA-TU-8902 Vector
PA-TU-8902 GalNAcT-14
Cell Viability (%)
100
75
50
25
0
0
12
37
111
333
1000
Apo2L/TRAIL Conc. (ng/ml)
PL-45
PL-45 Vector
PL-45 GalNAcT-14
Cell Viability (%)
100
75
50
25
0
0
4
12
37
111
333
1000
Apo2L/TRAIL Conc. (ng/ml)

*FIG. 14F*

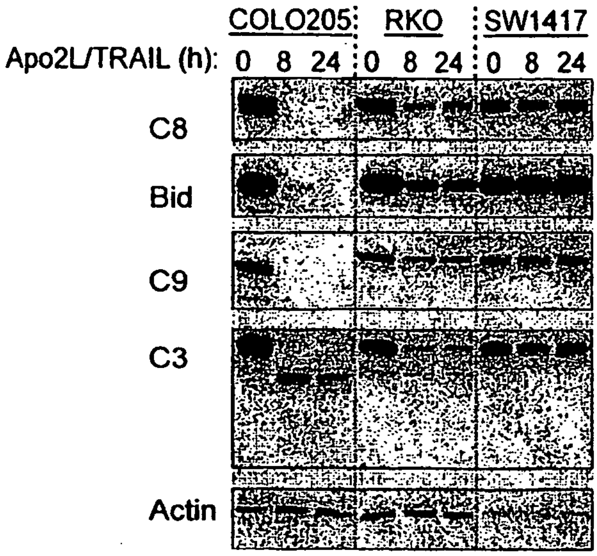


*FIG. 15A*

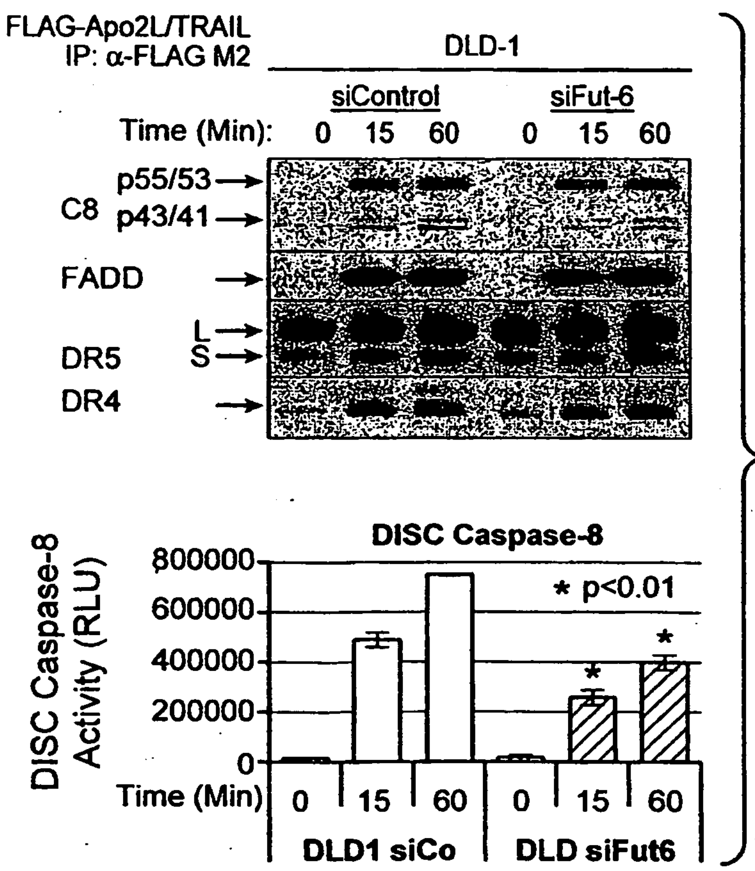


*FIG. 15B*

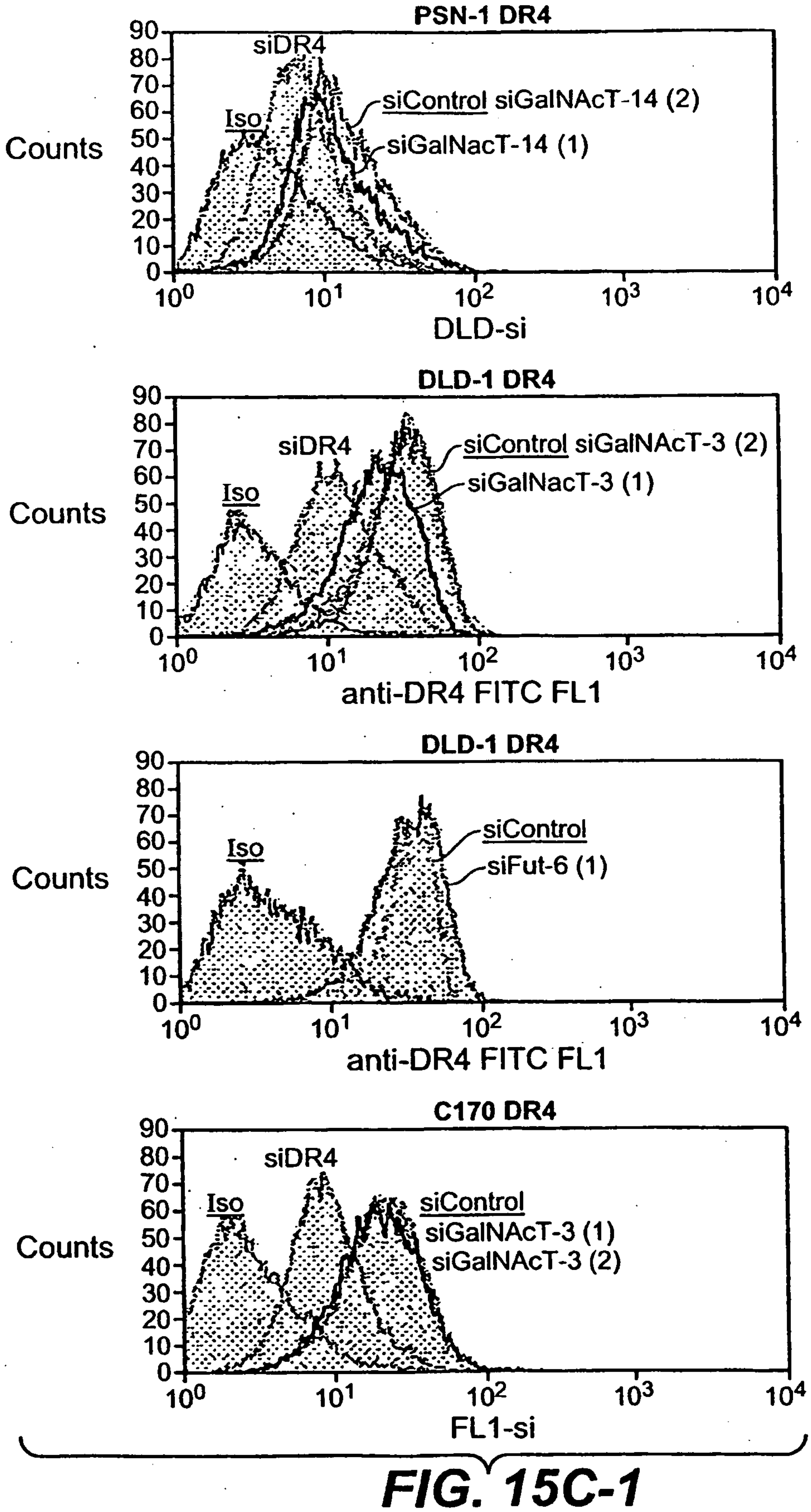
PSN-1 DR4
Counts
siDR4
Iso
siControl siGalNAcT-14 (2)
siGalNacT-14 (1)
DLD-si
DLD-1 DR4
Counts
siDR4
Iso
siControl siGalNAcT-3 (2)
siGalNacT-3 (1)
anti-DR4 FITC FL1
DLD-1 DR4
Counts
Iso
siControl
siFut-6 (1)
anti-DR4 FITC FL1
C170 DR4
Counts
siDR4
Iso
siControl
siGalNAcT-3 (1)
siGalNAcT-3 (2)
FL1-si

*FIG. 15C-1*

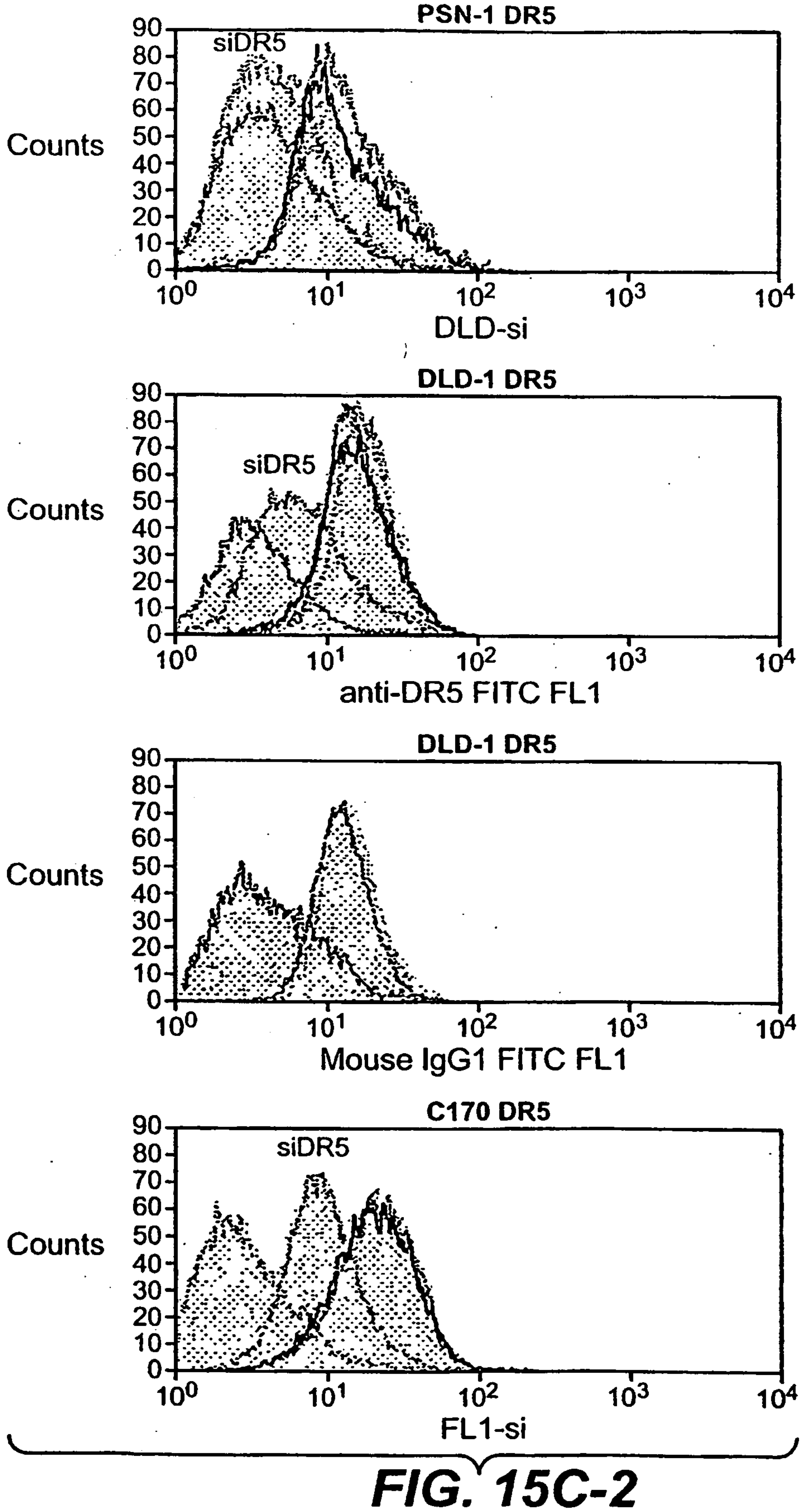
PSN-1 DR5
siDR5
Counts
90
80
70
60
50
40
30
20
10
0
$10^0$
$10^1$
$10^2$
$10^3$
$10^4$
DLD-si
DLD-1 DR5
siDR5
Counts
anti-DR5 FITC FL1
DLD-1 DR5
Counts
Mouse IgG1 FITC FL1
C170 DR5
siDR5
Counts
FL1-si

*FIG. 15C-2*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9701633 A
- WO 9725428 A
- WO 9828426 A
- WO 9846751 A
- WO 9818921 A
- EP 417563 A
- US 5763223 A
- US 6284236 B
- US 6030945 A
- US 6746668 B
- US 0015512 W
- US 0123691 W
- WO 9832856 A
- WO 9937684 A
- WO 0073349 A
- US 20030036168 A
- US 6433147 B
- US 6461823 B
- US 6342383 B
- WO 9851793 A
- WO 9841629 A
- WO 9835986 A
- EP 870827 A
- WO 9846643 A
- WO 9902653 A
- WO 9909165 A
- WO 991179 A
- WO 03042367 A
- WO 02097033 A
- WO 03038043 A
- US 20020072091 A
- US 20020098550 A
- US 6313269 B
- US 20010010924 A
- US 200301255540 A
- US 20020160446 A
- US 20020048785 A
- US 20040141952 A
- US 20050129699 A
- US 20050129616 A
- US 6342369 B
- US 6569642 B
- US 6072047 A
- US 6642358 B
- US 6743625 B
- WO 03066661 A
- WO 03037913 A
- US 20030073187 A
- US 20030108516 A
- US 20030180296 A
- US 6252050 B
- WO 9858062 A
- WO 9910484 A
- WO 0100832 A
- EP 404097 A
- WO 9311161 A
- US 4816567 A
- US 5693780 A
- US 5500362 A
- US 5821337 A
- US 4275149 A
- US 4737456 A
- US 4318980 A
- US 4016043 A
- US 4424279 A
- US 4018653 A
- WO 0175166 A
- US 5700637 A
- US 5445934 A
- US 5807522 A
- US 6358682 B, Kallioniemi
- US 4657760 A
- US 5206344 A
- US 5225212 A


### Non-patent literature cited in the description


- **ASHKENAZI.** *Nature Review,* 2002, vol. 2, 420-430
- **ASHKENAZI ; DIXIT.** *Science,* 1998, vol. 281, 1305-1308
- **ASHKENAZI ; DIXIT.** *Curr. Opin. Cell Biol.,* 2000, vol. 11, 255-260
- **GOLSTEIN.** *Curr. Biol.,* 1997, vol. 7, 750-753
- **WALLACH.** Cytokine Reference. Academic Press, 2000, 377-411
- **LOCKSLEY et al.** *Cell,* 2001, vol. 104, 487-501
- **GRUSS ; DOWER.** *Blood,* 1995, vol. 85, 3378-3404
- **SCHMID et al.** *Proc. Natl. Acad. Sci.,* 1986, vol. 83, 1881

- **DEALTRY et al.** *Eur. J. Immunol.,* 1987, vol. 17, 689
- **PITTI et al.** *J. Biol. Chem.,* 1996, vol. 271, 12687-12690
- **WILEY et al.** *Immunity,* 1995, vol. 3, 673-682
- **BROWNING et al.** *Cell,* 1993, vol. 72, 847-856
- **ARMITAGE et al.** *Nature,* 1992, vol. 357, 80-82
- **MARSTERS et al.** *Curr. Biol.,* 1998, vol. 8, 525-528
- **CHICHEPORTICHE et al.** *Biol. Chem.,* 1997, vol. 272, 32401-32410
- **HAHNE et al.** *J. Exp. Med.,* 1998, vol. 188, 1185-1190
- **MOORE et al.** *Science,* 1999, vol. 285, 260-263
- **SHU et al.** *J. Leukocyte Biol.,* 1999, vol. 65, 680
- **SCHNEIDER et al.** *J. Exp. Med.,* 1999, vol. 189, 1747-1756
- **MUKHOPADHYAY et al.** *J. Biol. Chem.,* 1999, vol. 274, 15978-15981
- **SALVESEN et al.** *Cell,* 1997, vol. 91, 443-446
- **ASHKENAZI.** *Nature Reviews,* 2002, vol. 2, 420-430
- **HOHMAN et al.** *J. Biol. Chem.,* 1989, vol. 264, 14927-14934
- **BROCKHAUS et al.** *Proc. Natl. Acad. Sci.,* 1990, vol. 87, 3127-3131
- **LOETSCHER et al.** *Cell,* 1990, vol. 61, 351
- **SCHALL et al.** *Cell,* 1990, vol. 61, 361
- **SMITH et al.** *Science,* 1990, vol. 248, 1019-1023
- **LEWIS et al.** *Proc. Natl. Acad. Sci.,* 1991, vol. 88, 2830-2834
- **GOODWIN et al.** *Mol. Cell. Biol.,* 1991, vol. 11, 3020-3026
- **STAMENKOVIC et al.** *EMBO J.,* 1989, vol. 8, 1403-1410
- **MALLETT et al.** *EMBO J.,* 1990, vol. 9, 1063-1068
- **ANDERSON et al.** *Nature,* 1997, vol. 390, 175-179
- **CHICHEPORTICHE et al.** *J. Biol. Chem.,* 1997, vol. 272, 32401-32410
- **PAN et al.** *Science,* 1997, vol. 276, 111-113
- **PAN et al.** *Science,* 1997, vol. 277, 815-818
- **SHERIDAN et al.** *Science,* 1997, vol. 277, 818-821
- **DEGLI-ESPOSTI et al.** *J. Exp. Med.,* 1997, vol. 186, 1165-1170
- **MARSTERS et al.** *Curr. Biol.,* 1997, vol. 7, 1003-1006
- **TSUDA et al.** *BBRC,* 1997, vol. 234, 137-142
- **NOCENTINI et al.** *Proc. Natl. Acad. Sci.,* 1997, vol. 94, 6216-6221
- **VONBULOW et al.** *Science,* 1997, vol. 278, 138-141
- **PITTI et al.** *J. Biol. Chem.,* 1996, vol. 271, 12697-12690
- **MARIANI et al.** *J. Cell. Biol.,* 1997, vol. 137, 221-229
- **HYMOWITZ et al.** *Molec. Cell,* 1999, vol. 4, 563-571
- **CHA et al.** *Immunity,* 1999, vol. 11, 253-261
- **MONGKOLSAPAYA et al.** *Nature Structural Biology,* 1999, vol. 6, 1048
- **HYMOWITZ et al.** *Biochemistry,* 1999, vol. 39, 633-644
- **BODMER et al.** *J. Biol. Chem.,* 2000, vol. 275, 20632-20637
- **THOMAS et al.** *J. Immunol.,* 1998, vol. 161, 2195-2200
- **JOHNSEN et al.** *Cytokine,* 1999, vol. 11, 664-672
- **GRIFFITH et al.** *J. Exp. Med.,* 1999, vol. 189, 1343-1353
- **SONG et al.** *J. Exp. Med.,* 2000, vol. 191, 1095-1103
- **RIEGER et al.** *FEBS Letters,* 1998, vol. 427, 124-128
- **ASHKENAZI et al.** *J. Clin. Invest.,* 1999, vol. 104, 155-162
- **WALCZAK et al.** *Nature Med.,* 1999, vol. 5, 157-163
- **KEANE et al.** *Cancer Research,* 1999, vol. 59, 734-741
- **MIZUTANI et al.** *Clin. Cancer Res.,* 1999, vol. 5, 2605-2612
- **GAZITT.** *Leukemia,* 1999, vol. 13, 1817-1824
- **YU et al.** *Cancer Res.,* 2000, vol. 60, 2384-2389
- **CHINNAIYAN et al.** *Proc. Natl. Acad. Sci.,* 2000, vol. 97, 1754-1759
- **GLINIAK et al.** *Cancer Res.,* 1999, vol. 59, 6153-6158
- **ROTH et al.** *Biochem. Biophys. Res. Comm.,* 1999, vol. 265, 1999
- **JO et al.** *Nature Med.,* 2000, vol. 6, 564-567
- **NAGATA.** *Nature Med.,* 2000, vol. 6, 502-503
- **LAWRENCE et al.** Nature Med. 2001, vol. 7, 383-385
- **QIN et al.** Nature Med. 2001, vol. 7, 385-386
- **SCREATON et al.** *Curr. Biol.,* 1997, vol. 7, 693-696
- **WALCZAK et al.** *EMBO J.,* 1997, vol. 16, 5386-5387
- **WU et al.** *Nature Genetics,* 1997, vol. 17, 141-143
- **HYMOWITZ et al.** *Molecular Cell,* 1999, vol. 4, 563-571

- **KISCHKEL et al.** *Immunity,* 2000, vol. 12, 611-620
- **SPRICK et al.** *Immunity,* 2000, vol. 12, 599-609
- **BODMER et al.** *Nature Cell Biol.,* 2000, vol. 2, 241-243
- **MCFARLANE et al.** *J. Biol. Chem.,* 1997, vol. 272, 25417-25420
- **SCHNEIDER et al.** *FEBS Letters,* 1997, vol. 416, 329-334
- **MONGKOLSAPAYA et al.** *J. Immunol.,* 1998, vol. 160, 3-6
- **PAN et al.** *FEBS Letters,* 1998, vol. 424, 41-45
- **DEGLI-ESPOSTI et al.** *Immunity,* 1997, vol. 7, 813-820
- **GRIFFITH et al.** *J. Immunol.,* 1999, vol. 162, 2597-2605
- **CHUNTHARAPAI et al.** *J. Immunol.,* 2001, vol. 166, 4891-4898
- **ICHIKAWA et al.** *Nature Med.,* 2001, vol. 7, 954-960
- **HYLANDER et al.** An Antibody to DR5 (TRAIL-Receptor 2) Suppresses the Growth of Patient Derived Gastrointestinal Tumors Grown in SCID mice. *2d International Congress on Monoclonal Antibodies in Cancers,* 29 August 2002
- **WANG et al.** *cloning of the GalNAC polypeptide,* vol. 300, 738-744
- **ASHKENAZI et al.** *J. Clin. Invest.,* 1999
- **WALCZAK et al.** *Nature Med.,* 1999
- **PAN et al.** *Science,* 1997, vol. 276, 111
- **WANG et al.** *BBRC,* 2003, vol. 300, 738-744
- **BENNETT et al.** *J. Biol. Chem.,* 1996, vol. 271, 17006-17012
- **SHARP et al.** *J. Biol. Chem.,* 2005, vol. 280, 19401
- **SAMBROOK et al.** Molecular Cloning: A Laboratory Manual. Cold Spring Harbor Laboratory Press, 1989
- **HANG et al.** The chemistry and biology of mucin-type O-linked glycosylation initiated by the polypeptide N-acetyl- -galactosaminyltransferases. *Bioorganic & Medicinal Chemistry,* May 2005, www.sciencedirect.com
- **BENNETT et al.** *J. Biol. Chemistry,* 1996, vol. 271, 17006-17012
- **KABAT et al.** Sequences of Proteins of Immunological Interest. National Institutes of Health, 1991
- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **AGNEW.** *Chem Intl. Ed. Engl.,* 1984, vol. 33, 183-186
- Cell cycle regulation, oncogens, and antineoplastic drugs. **MURAKAMI et al.** The Molecular Basis of Cancer. WB Saunders, 1995, 13
- **NICOLETTI et al.** *J. Immunol. Methods,* 1991, vol. 139, 271-279
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **RAVETCH ; KINET.** *Annu. Rev. Immunol,* 1991, vol. 9, 457-92
- **CLYNES et al.** *PNAS (USA),* 1998, vol. 95, 652-656
- **DAËRON.** *Annu. Rev. Immunol.,* 1997, vol. 15, 203-234
- **CAPEL et al.** *Immunomethods,* 1994, vol. 4, 25-34
- **HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GAZZANO-SANTORO et al.** *J. Immunol. Methods,* 1996, vol. 202, 163
- Current Protocols In Molecular Biology. 1995
- Manual of Histological Staining Method of the Armed Forces Institute of Patholog. The Blakston Division McGraw-Hill Book Company, 1960
- The Armed Forces Institute of Pathology Advanced Laboratory Methods in Histology and Pathology. Armed Forces Institute of Pathology, 1994
- Current Protocols in Immunology. Wiley-Interscience, 1991, vol. 1-2
- Methods for the Preparation of Enzyme-Antibody Conjugates for use in Enzyme Immunoassay. **O'SULLIVAN et al.** Methods in Enzym. Academic press, 1981, vol. 73, 147-166
- **LEONG et al.** *Appl. Immunohistochem.,* 1996, vol. 4 (3), 201

- **LOCKART.** *Nature Biotechnology,* 1996, vol. 14, 1675-1680
- **CHEUNG, V.G. et al.** *Nature Genetics,* 1999, vol. 21, 15-19
- **THOMAS.** *Proc. Natl. Acad. Sci. USA,* 1980, vol. 77, 5201-5205
- **DE MARZO et al.** *Am. J. Pathol.,* 1999, vol. 155 (6), 1985-1992
- **BROOKS et al.** *Cancer Epidemiol. Biomarkers Prev.,* 1998, vol. 7, 531-536
- **LETHE et al.** *Int. J. Cancer,* 1998, vol. 76 (6), 903-908
- **MORDENTI et al.** *Pharmaceut. Res.,* 1991, vol. 8, 1351
- Chemotherapy Service. Williams & Wilkins, 1992
- **DANIAL et al.** *Cell,* 2004, vol. 116, 205
- **CORY et al.** *Nat. Rev. Cancer,* 2002, vol. 2, 647
- **ASHKENAZI et al.** *Science,* 1998, vol. 281, 1305
- **LEBLANC et al.** *Cell Death Differ.,* 2003, vol. 10, 66
- **KISCHKEL et al.** *EMBO J.,* 1995, vol. 14, 5579
- **KISCHKEL et al.** *Immunity,* 2000, vol. 12, 611
- **SCAFFIDI et al.** *J. Biol. Chem.,* 1999, vol. 274, 1541
- **ASHKENAZI.** *Nat. Rev. Cancer,* 2002, vol. 2, 420
- **KELLEY et al.** *Curr. Opin. Pharmacol.,* 2004, vol. 4, 333
- **IGNEY et al.** *Nat. Rev. Cancer,* 2002, vol. 2, 277
- **DELANNOY et al.** *Glycoconj.,* 1996, vol. 13, 717
- **EDITORIAL.** *Nat. Cell. Biol.,* 2003, vol. 5, 489
- **WEI et al.** *Science,* 2001, vol. 292, 727
- **JULENIUS et al.** *Glycobiology,* 2005, vol. 15, 153, http://www.cbs.dtu.dk/services/NetOGlyc
- **HANG et al.** *Bioorg. Med. Chem.,* 2005, vol. 13, 5021
- **HANISCH.** *Biol. Chem.,* 2001, vol. 382, 143
- **BROCKHAUSEN.** *Biochim. Biophys. Acta,* 1999, vol. 1473, 67
- **DUBE et al.** *Nat. Rev. Drug Discovery,* 2005, vol. 4, 477
- **FUSTER et al.** *Nat. Rev. Cancer,* 2005, vol. 5, 526
- **FUSTER et al.** *Cancer Res.,* 2003, vol. 63, 2775
- **OHYAMA et al.** *EMBO J.,* vol. 18, 1516
- **TAKADA et al.** *Cancer Res.,* 1993, vol. 53, 354
- **LAWRENCE et al.** *Nat. Med.,* 2001, vol. 7, 383
- **HOFFMAN et al.** *Nat. Rev. Genetics,* 2004, vol. 5, 229
- **YAUCH et al.** *Clin. Cancer Res.,* 2005, vol. 11, 8686
- **KISCHKEL.** *Immunity,* 2000, vol. 12, 611